# EUROPÄISCHE PATENTSCHRIFT

(11) **EP 1 372 850 B1**
(45) Veröffentlichungstag und Bekanntmachung des Hinweises auf die Patenterteilung: **27.09.2006**
(21) Anmeldenummer: 02707992.0
(22) Anmeldetag: 28.03.2002
(51) Int. Cl.: B01L 3/14, G01N 33/49

(54) **AUFNAHMEEINRICHTUNG, INSBESONDERE FÜR KÖRPERFLÜSSIGKEITEN, MIT EINER TRENNVORRICHTUNG SOWIE TRENNVORRICHTUNG HIERZU**
HOLDING DEVICE, PARTICULARLY FOR BODY FLUIDS, COMPRISING A SEPARATING DEVICE, AND A SEPARATING DEVICE THEREFOR
DISPOSITIF DE RECEPTION NOTAMMENT POUR LIQUIDES ORGANIQUES, MUNI D'UN DISPOSITIF DE SEPARATION ET DISPOSITIF DE SEPARATION CORRESPONDANT

(30) Priorität: 30.03.2001 AT 5122001; 03.08.2001 AT 12102001; 25.03.2002 AT 4552002
(43) Veröffentlichungstag der Anmeldung: 02.01.2004
(73) Patentinhaber: Greiner Bio-One GmbH, 4550 Kremsmünster (AT)
(72) Erfinder: KONRAD, Franz, A-4690 Schwanenstadt (AT)
(74) Vertreter: Secklehner, Günter
(86) Internationale Anmeldenummer: PCT/AT2002/000095
(87) Internationale Veröffentlichungsnummer: WO 2002/078848

(56) Entgegenhaltungen:
- EP-A- 0 753 741
- EP-A- 1 005 910
- EP-A- 1 006 360
- US-A- 3 849 072

## Beschreibung

Die Erfindung betrifft eine Trennvorrichtung zum Einsetzen in den Innenraum eines Aufnahmebehälters sowie eine Aufnahmeeinrichtung mit einer derartigen Trennvorrichtung, wie dies in den Ansprüchen 1, 36, 49 und 69 beschrieben wird.

Eine Trennvorrichtung zum Einsetzen in einen Aufnahmeraum eines Aufnahmebehälters einer Aufnahmeeinrichtung ist der US 5,266,199 A bekannt geworden, welche einen elastischen Tragkörper, einen diesen umgebenden elastischen Ring, der in seinem Umfangsbereich eine Trennung aufweist, sowie ein in einen Strömungskanal innerhalb des elastischen Tragkörpers zur Abdichtung einsetzbare Kugel aufweist. Der den elastischen Tragkörper umgebende elastische Ring dient dabei als Dichtungsvorrichtung zwischen der Innenwand des Aufnahmebehälters und dem elastischen Tragkörper in der Einsatzstellung der Trennvorrichtung. Der im elastischen Tragkörper angeordnete Strömungskanal, der sich zwischen den in Richtung einer Längsachse voneinander distanzierten Endbereichen erstreckt, wird in der Einsatzstellung durch die auf den dichteren Bestandteilen aufschwimmende Kugel verschlossen.

Aus der EP 0 753 741 A1 ist eine Aufnahmeeinrichtung mit einem Aufnahmebehälter bekannt geworden, der zwei in einer Längsachse voneinander distanzierte Enden aufweist, von denen zumindest eines mit einer Öffnung ausgebildet ist. Die Innenabmessung des Aufnahmebehälters im Bereich des ersten offenen Endes in der senkrecht zur Längsachse ausgerichteten Ebene ist größer der inneren Abmessung im Bereich des weiteren Endes in der dazu parallel ausgerichteten Ebene in der gleichen Raumrichtung. Weiters ist in das offene Ende ein ringförmiger Bauteil eingesetzt, welcher die offene Stirnseite des Aufnahmebehälters mit einem Bund abdeckt und ein zylindrischer Wandteil in den Innenraum des Aufnahmebehälters zumindest bereichsweise hineinragt. Der ringförmige Bauteil weist im Anschluß an den zylinderförmigen Wandteil einen Absatz und damit verbunden eine Querschnittserweiterung auf, an welcher sich das elastische Dichtelement der Trennvorrichtung in der Ausgangsstellung abstützt. Im Zentrum weist die Trennvorrichtung eine Ausnehmung auf, welche mit einer dünnen Deckplatte im Bereich des oberen Endes des Aufnahmebehälters verschlossen ist. Das Zusammenfügen der einzelnen Bauteile, insbesondere das Einsetzen der Trennvorrichtung, erfolgt in einer Vakuumkammer, da nach dem Einsetzen der Trennvorrichtung ohne Beschädigung dieser ein Zugang in den Innenraum nicht mehr möglich ist. Zusätzlich wird noch am bundförmigen Ansatz des ringförmigen Bauteils noch eine Folie aufgeklebt sowie eine Kappe angebracht. Die Befüllung des Innenraumes erfolgt mittels eines Durchstechens der dünnen Deckplatte der Trennvorrichtung, der dünnen Folie sowie gegebenenfalls der Kappe. Durch diesen Befüllvorgang wird im Innenraum das Vakuum abgebaut, wodurch auch Luft mit in den Innenraum eingesaugt wird. Anschließend daran erfolgt der Zentrifugiervorgang, bei welchem die Trennvorrichtung aus dem ringförmigen Bauteil in Richtung des verschlossenen Endes heraustritt und mit seinem Dichtelement weiters an der inneren Oberfläche des Aufnahmebehälters zur Anlage kommt. Die Sinkgeschwindigkeit im Gemisch bzw. den bereits getrennten Bestandteilen wird durch die Anpreßkraft des elastischen Dichtelementes an der inneren Oberfläche bestimmt. Durch die Wahl der Dichte der gesamten Trennvorrichtung in bezug auf die zu trennenden Bestandteile des Gemisches erfolgt ein Aufschwimmen derselben an der Trennfläche zwischen den beiden eine unterschiedliche Dichte aufweisenden Medien. Ein Durchtritt des leichteren Mediums während des Zentrifugiervorganges ist zwischen der inneren Oberfläche des Aufnahmebehälters und dem elastischen Dichtelement möglich.

Eine weitere Aufnahmeeinrichtung mit einer Trennvorrichtung ist aus der EP 1 005 910 A2 bekannt geworden, welche einen zylinderförmig ausgebildeten Aufnahmebehälter mit nahezu konstantem inneren Durchmesser aufweist. Am offenen Ende des Aufnahmebehälters ist eine durchstechbare Verschlußvorrichtung angeordnet, an welcher auch in der Ausgangsstellung die Trennvorrichtung nahezu anliegend angeordnet ist. Diese Trennvorrichtung ist aus einem flexiblen rückstellbaren Werkstoff gebildet, wobei am äußeren Umfang der Trennvorrichtung eine Dichtvorrichtung zur Abdichtung mit der inneren Oberfläche des Aufnahmebehälters vorgesehen ist. Zusätzlich ist im Innenraum noch ein deformierbares Element eingesetzt, welches während der Beaufschlagung mit der Zentrifugalkraft durch den vom Medium ausgeübten Druck an die Innenwand des äußeren Behälters gedrückt wird und so zwischen der Trennvorrichtung und dem eingesetzten deformierten Einsatzteil ein Durchströmkanal gebildet wird, welcher nach Wegnahme der Zentrifugalkraft mit den an der Trennvorrichtung angeordneten Dichtelementen wieder eine dichtende Lage einnimmt, wodurch die voneinander separierten Medien voneinander getrennt bleiben.

Es ist bereits eine Aufnahmeeinrichtung für ein Gemisch von zumindest zwei Medien, gemäß DE 195 13 453 A1, bekannt, welche einen eprouvettenartigen Aufnahmebehälter aufweist, der in einem offenen Stimendbereich mit einer Verschlußvorrichtung verschlossen und in dem eine Trennvorrichtung zum Abtrennen der unterschiedlichen Medien des Gemisches nach dem Trennen eingesetzt ist. Um zu verhindern, daß die nachfolgend nur mehr mit einem Medium in Berührung kommende Stirnfläche der Trennvorrichtung beim Einfüllen des Gemisches in den Innenraum des Behälters kontaminiert wird, ist die Trennvorrichtung im Mittelbereich mit einer Durchgangsöffnung versehen, durch die das Gemisch in den verbleibenden Innenraum des Aufnahmebehälters eingebracht werden kann. Während des nachfolgenden Trennvorgangs durch Zentrifugieren in herkömmlicher Weise mit einer radialen Zentrifugalkraft (rcf) von 1.000 g bis 5.000 g - wobei g die Schwerkraft und 1 g ein Wert von 9,81 m/s² ist - wird das eine aus dem Gemisch abgetrennte Medium durch den Durchbruch in der Trennvorrichtung in den zwischen der Dichtungsvorrichtung und der Trennvorrichtung befindlichen Bereich überführt und sinkt in Folge dessen in Richtung des geschlossenen Endes des Aufnahmebehälters ab. Um zu verhindern, daß nach der Trennung durch den Durchbruch das zwischen dem geschlossenen Ende und der Trennvorrichtung befindliche andere Medium sich mit dem davon abgetrennten Medium wieder vermischen kann, ist in einer der üblichen verbleibenden Menge des anderen Mediums entsprechenden Höhe ein sich in Richtung des geschlossenen Endes konusförmig erweiternder Endanschlag vorgesehen, mit dem die Trennvorrichtung auf dem Endanschlag, der durch den Durchbruch hindurchdringt, aufläuft. Sobald der Außendurchmesser des Endanschlages dem Innendurchmesser des Durchbruchs entspricht, verbleibt die Trennvorrichtung in dieser Position und es ist dadurch der Durchbruch mit dem Anschlag verschlossen und es kann kein Austausch oder keine nochmalige Vermischung der beiden Medien stattfinden. Nachteilig ist bei dieser Ausführungsvariante, daß ein Röhrchen mit einem innenliegenden Anschlag hergestellt werden muß und keine sichere Funktion der Mediumtrennung, bedingt durch den in der Trennvorrichtung angeordneten Durchbruch, sichergestellt werden kann. Weiters ist ein nachträgliches Einsetzen der Trennvorrichtung in den Innenraum des Aufnahmebehälters nur schwierig zu realisieren.

Andere Aufnahmeeinrichtungen für das Zentrifugieren zu trennender Gemische aus zumindest zwei unterschiedlichen Medien, bei welchen der Aufnahmebehälter in beiden Stirnendbereichen mit einer Verschlußvorrichtung verschlossen ist, sind aus der WO 96/05770 A1 bekannt. Im Inneren ist eine durch eine Dichtscheibe gebildete Trennvorrichtung angeordnet, die durch ein Gel gebildet ist. Während des Zentrifugiervorgangs wandert dieser Gelkolben auf Grund seines spezifischen Gewichtes, welches höher ist als das spezifische Gewicht des Mediums mit dem geringeren spezifischen Gewicht und niederer ist als das spezifische Gewicht des Mediums mit höherem spezifischen Gewicht, auf Grund der auf ihn einwirkenden Fliehkräfte zwischen die zwei unterschiedlichen, voneinander getrennten Medien. In dieser positionierten Stellung kann damit eine Trennung des einen Mediums vom anderen Medium des Gemisches erfolgen. Nachteilig ist hierbei, daß die Lagerdauer, bedingt durch die Trennvorrichtung aus Gel, in vielen Fällen für die normale Einsatzdauer nicht ausreicht.

Weitere Aufnahmeeinrichtungen mit darin angeordneten Trennvorrichtungen, welche mit unterschiedlichsten Ventilanordnungen sowie Filterelementen gebildet sind, sind aus der EP 0 311 011 A2, der US 3,897,343 A, der US 3,897,340 A, US 4,202,769 A sowie der US 3,897, 337 A bekannt geworden.

Andere Aufnahmeeinrichtungen mit darin befindlichen Trennvorrichtungen sind aus der EP 1 106 250 A2, EP 1 106 251 A2, EP 1 106 252 A2, EP 1 106 253 A2 und der EP 1 107 002 A2 bekannt geworden, wobei die Trennvorrichtungen in den unterschiedlichsten Ausführungsformen ausgebildet sind und auf dem Prinzip der Verformbarkeit eines Bauteils der Trennvorrichtung während dem Zentrifugiervorgang sowie der Dichteabstimmung zwischen den zu trennenden Medien beruhen.

Der vorliegenden Erfindung liegt die Aufgabe zugrunde, eine Trennvorrichtung sowie eine Aufnahmeeinrichtung mit einer darin angeordneten Trennvorrichtung zu schaffen, mit welcher eine sichere und dauerhafte Trennung der zu separierenden Bestandteile des Gemisches während des Zentrifugiervorganges sowie während der nachfolgenden Aufbewahrung sichergestellt ist.

Die Aufgabe der Erfindung wird durch die im Anspruch 1 angegebenen Merkmale gelöst. Die sich aus der Merkmalskombination des Kennzeichenteils dieses Anspruches ergebende Vorteile liegen darin, daß so eine Trennvorrichtung aus zumindest zwei Grundkörper geschaffen wird, welcher in den Innenraum des Aufnahmebehälters einsetzbar ist und bei welchem bis hin zum Erreichen einer Arbeitsstellung stets ein Durchtritt eines der zu trennenden Bestandteile des Gemisches ermöglicht wird. Anschließend ist eine selbsttätig mechanische Anlage bzw. ein mechanische Festsitzen an der inneren Oberfläche des Aufnahmebehälters in der vorbestimmbaren Arbeitsstellung sichergestellt. Dieses Festsitzen wird dann erreicht, wenn die Grundkörper den Strömungskanal verschließen und die innere Abmessung des Innenraums des Aufnahmebehälters im Bereich der Arbeitsstellung kleiner dem äußeren Umfang der Trennvorrichtung in der gleichen Stellung ist. Durch diese Trennvorrichtung und das mechanische Festsitzen ist weiters eine Überkopfentnahme des zwischen der Trennvorrichtung und der bedarfsweise öffenbaren Verschlußvorrichtung enthaltenen und getrennten Bestandteil des Gemisches möglich, ohne daß dabei ein Verrutschen der Trennvorrichtung in Richtung der Verschlußvorrichtung erfolgt und so eine ungewollte Vermischung der bereits von einander getrennten Bestandteile möglich ist.

Vorteilhaft ist auch eine weitere Ausführungsform nach Anspruch 2 oder 3, da dadurch stets eine gesicherte Bewegung der Trennvorrichtung auch durch das leichtere Medium hindurch sichergestellt wird. Bei höher gewählten Dichtewerte kann die im Bereich zwischen der Innenwandung des Behälters und der Trennvorrichtung auftretende Reibungskraft auch bei geringeren Zentrifugalkräften sicher überwunden werden, um so eine sichere Verstellbewegung bis hin zur Arbeitsstellung zu gewährleisten.

Vorteilhaft ist weiters eine Ausbildung nach Anspruch 4, da dadurch auch bei mehreren Grundkörpern stets ein sicherer Durchtritt des zu trennenden Mediums durch den Strömungskanal sichergestellt ist, wobei auch bei Erreichen der Arbeitsstellung stets auch eine sichere Abdichtung zwischen den zu trennenden Aufnahmeräumen innerhalb der Aufnahmeeinrichtung sichergestellt ist.

Vorteilhaft ist auch eine Weiterbildung nach Anspruch 5, da dadurch die Grundkörper relativ zueinander nur eine senkrecht zur Verstellbewegung ausgerichtete Bewegung zueinander durchführen können und eine gegenseitige Verlagerung derselben in Richtung der Längsachse verhindert ist.

Durch die Ausbildung nach Anspruch 6 wird erreicht, daß die Grundkörper an die jeweils gegenüberliegenden Innenwandungen des Aufnahmebehälters angedrückt werden und so zwischen diesen der Strömungskanal solange ausgebildet ist, bis daß die beiden Grundkörper auch an den einander zugewandten Bereichen dichtend aneinander anliegen.

Bei der Ausgestaltung nach Anspruch 7 oder 8 ist von Vorteil, daß die Grundkörper während deren gesamten Einsatzdauer innerhalb des Aufnahmebehälters zueinander stets in einer senkrecht zur Längsachse ausgerichteten Verstellebene relativ verstellbar sind, jedoch in Richtung der Längsachse zueinander stets die gleiche Lage aufweisen. Weiters wird auch noch dadurch der Montageaufwand erleichtert, da nur ein einziger Teil in den Aufnahmeraum einzusetzen ist.

Durch die Weiterbildung nach Anspruch 9 wird eine gleichmäßig gerichtete Druckkraft auf die Grundkörper ausgeübt und so ein Verkanten bzw. eine Verklemmung während des Verstellvorganges verhindert.

Durch die Weiterbildungen nach den Ansprüchen 10 bis 13 werden die für die Öffnungsbewegung zur Bildung des Strömungskanals aufzubringenden Kräfte gleichmäßig auf die Grundkörper übertragen, wodurch einerseits eine gesicherte Anlage der Grundkörper an den vorgesehenen Stellen der Innenwandungen sichergestellt ist und andererseits ein ungehindertes Durchströmen durch den Strömungskanal erzielt wird.

Durch die Ausbildung nach Anspruch 14 kann die Trennvorrichtung auf einfache Art und Weise kostengünstig gefertigt werden.

Vorteilhaft ist auch eine Ausbildung nach Anspruch 15, da dadurch auch im gegenseitigen Anlagebereich der Grundkörper aneinander eine gesicherte Abdichtung des Strömungskanals erzielt wird.

Vorteilhaft ist aber auch eine Ausbildung nach Anspruch 16, da dadurch eine noch höhere Sicherheit für die Abdichtung der zu trennenden Aufnahmeräume zwischen den zu trennenden Medien erzielbar ist.

Gemäß einer Ausbildung, wie im Anspruch 17 beschrieben, werden Toträume im Bereich der Trennvorrichtung vermieden und dadurch eine vollständige Trennung, ohne der Gefahr einer nachträglichen Verunreinigung eines der Medien, erzielt.

Vorteilhaft ist auch ein Ausbildung nach Anspruch 18 oder 19, da dadurch die Trennvorrichtung nur im Bereich der Dichtlippen an der Innenwandung des Aufnahmebehälters zur Anlage kommt und so auftretende Fertigungstoleranzen einfach ausgeglichen werden können.

Gemäß einer Ausbildung, wie im Anspruch 20 beschrieben, wird auch in dem, dem Aufnahmebehälter zugewandten Bereich des Strömungskanals eine sichere Abdichtung zwischen den voneinander zu trennenden Aufnahmeräumen geschaffen.

Durch die Weiterbildung nach Anspruch 21 wird auf alle Fälle in dem zur Abdichtung vorgesehenen Bereich eine einwandfreie Dichtung geschaffen.

Nach einer vorteilhaften Weiterbildung gemäß Anspruch 22 kann ein hoher Grad an Materialeinsparung erzielt werden.

Bei einer Ausbildung gemäß den Ansprüchen 23 oder 24 wird eine großflächige Anlage der Grundkörper an den Innenwandungen des Aufnahmebehälters verhindert. Gleichzeitig wird damit auch eine ausreichende Führung während der gesamten Verstellbewegung bis zum Festsitzen erzielt. Gleichfalls wird auch noch die zum Festsitzen notwendige Reibungskraft erhöht, da für die Anlage eine wesentlich kleinere Fläche zur Verfügung steht und so Fertigungstoleranzen leichter ausgeglichen werden können.

Möglich sind aber auch Ausbildungen nach den Ansprüchen 25 oder 26, da dadurch das Durchströmen des Gemische bzw. auch nur eines Bestandteiles desselben durch den Strömungskanal begünstigt wird.

Die Ausgestaltung nach Anspruch 27 oder 28 ermöglicht eine gegenseitige Ausrichtung der Grundkörper in einer senkrecht zur Längsachse ausgerichteten Ebene zueinander, wobei zusätzlich noch durch den Rückhaltefortsatz eine Begrenzung des Verstellweges bei Verwendung einer Druckvorrichtung zwischen diesen erzielt und der Montageaufwand zum Einsetzen in den Innenraum der Aufnahmevorrichtung verringert werden kann.

Weitere vorteilhafte Ausbildungen sind in den Ansprüchen 29 bis 31 gekennzeichnet, wodurch einerseits eine Verbindung der Grundkörper zueinander geschaffen und andererseits die Funktionen des Druckelementes bzw. Stützelements realisiert werden können. Dadurch kann mit einer geringen Anzahl von Grundkörpern das Auslangen gefunden werden.

Vorteilhaft ist auch eine Ausbildung nach Anspruch 32 oder 33, da dadurch einerseits die für den Verstellvorgang notwendigen Dichtewerte für den Tragkörper erzielt und andererseits im kritischen Abdichtungsbereich ein sicherer Verschluß der voneinander zu trennenden Aufnahmeräume geschaffen wird.

Bei der Ausgestaltung gemäß der Ansprüche 34 oder 35 kann ein Anhaften von Bestandteilen des zu trennenden Gemisches an der Trennvorrichtung bzw. dessen Bauteilen gesichert verhindert werden.

Die Aufgabe der Erfindung wird aber auch durch die im Anspruch 36 angegebenen Merkmale gelöst. Vorteilhaft ist dabei, daß durch zumindest einen verschließbaren Strömungskanal zwischen den voneinander distanzierten Endbereichen der Trennvorrichtung und durch mindestens ein Druckelement im Bereich seiner Ausgangsstellung bis hin zu seinem Erreichen der Arbeitsstellung eine Durchtritt in beiden Richtungen ermöglicht wird, da das Druckelement die durch mindestens zwei Grundkörper gebildete Trennvorrichtung bereichsweise an die Innenwandung des Aufnahmebehälters andrückt. Durch das Zusammenwirken des sich stetig in Richtung des verschlossenen Endes verkleinernden Aufnahmeraums mit der Distanz zwischen den Grundkörpern bzw. zwischen diesen und dem Aufnahmebehälter kommt es nach einem vorbestimmbaren Verstellweg zu einem abmessungsbedingten mechanischen Festsitzen bzw. Festklemmen der Trennvorrichtung im Bereich der inneren Oberfläche des Aufnahmebehälters. Dieses Festsitzen bzw. Festklemmen wird dann erreicht, wenn die Grundkörper den Strömungskanal verschließen und die innere Abmessung des Aufnahmeraums im Bereich der Arbeitsstellung der Trennvorrichtung kleiner dem äußeren Umfang der Trennvorrichtung in der gleichen Stellung ist. Diese stetige Verkleinerung des Strömungskanals erfolgt durch die stete Verjüngung des Innenraums des Aufnahmebehälters, welcher in Art einer Steuerkurve für das vollständige Verschließen des Strömungskanals verantwortlich ist. Die Verstellbewegung wird durch die auf die Trennvorrichtung einwirkende Zentrifugalkraft bewirkt, wobei einerseits der Trennvorgang des zu trennenden Gemisches in seine einzelne Bestandteile und andererseits die Verstellbewegung so lange erfolgt, bis daß der vorbestimmbare mechanische Stop zwischen der Trennvorrichtung und dem Aufnahmebehälter erfolgt. Dabei kann das Ausmaß des Verstellweges in Richtung der Längsachse durch die Größe der Wahl des Strömungskanals im Bereich der Ausgangsstellung und das Ausmaß der Verjüngung des Innenraums festgelegt werden. Durch diese Ausbildung der Trennvorrichtung ist auch bereits eine Montage bzw. ein Einsetzen der Trennvorrichtung in den Innenraum und ein nachfolgendes Evakuieren sowie Verschließen durch die Verschlußvorrichtung möglich, da stets ein Zugang zu dem hier verschlossenen Ende durch die Trennvorrichtung hindurch ermöglicht wird. Damit kann eine ungehinderte Befüllung durchgeführt werden und es ist kein nachträgliches Einsetzen der Trennvorrichtung bzw. auch ein Abnehmen der Verschlußvorrichtung vor Beginn des Zentrifugiervorganges notwendig. Dadurch wird eine hohe Betriebssicherheit gewährleistet.

Von Vorteil sind aber auch Ausbildungen nach den Ansprüchen 37 bis 42, da dadurch bereits in der Ausgangslage eine vordefinierte Rückhaltekraft für die in den Innenraum eingesetzte Trennvorrichtung vor Beginn des Zentrifugiervorganges und somit auch während des Befüllvorganges sichergestellt werden kann.

Eine verbesserte Lagefixierung der Trennvorrichtung im Bereich der Arbeitsstellung wird mit Vorteil gemäß der im Anspruch 43 und 44 angegebenen Merkmale erzielt. Dabei kann die Trennvorrichtung mit ihrem dem weiteren Ende des Aufnahmebehälters zugewandten Endbereich oder aber auch mit der Dichtungsvorrichtung auf dieser vorzugsweise als Anschlagfläche ausgebildeten Positioniervorrichtung bei Erreichen der vorbestimmbaren Arbeitsstellung zur Anlage kommen. Damit ist eine Weiterbewegung und damit verbunden eine ungewünschte Vermischung in jedem Fall gesichert verhindert.

Vorteilhaft ist aber auch eine Weiterbildung nach Anspruch 45, da durch die Wahl der Größe der Verjüngung bzw. der Abnahme der inneren Querschnittsabmessung des Aufnahmebehälters der vorbestimmbare Verstellweg der Trennvorrichtung bis hin zu seiner Arbeitsstellung, in welcher eine allseits dichtende Abtrennung zwischen dem Innenraum, welcher zwischen der Trennvorrichtung und dem verschlossenen Ende bzw. der Trennvorrichtung und dem offenen Ende des Aufnahmebehälters angeordnet ist, einfach festlegbar ist.

Durch die unterschiedliche Wahl der Werkstoffe, wie dies in den Ansprüchen 46 und 47 beschrieben ist, ist es möglich, die Aufnahmeeinrichtung an die unterschiedlichsten Einsatzbedingungen abstimmen zu können.

Vorteilhaft ist weiters eine Ausbildung nach Anspruch 48, da so durch das Ausmaß des Unterdruckes im Innenraum des Aufnahmebehälters die Menge des aufzunehmenden Gemisches festlegbar ist.

Die Aufgabe der Erfindung wird aber auch eigenständig durch die Merkmale des Anspruches 49 gelöst. Die sich aus der Merkmalskombination des Kennzeichenteils dieses Anspruches ergebenden Vorteile liegen darin, daß so eine Trennvorrichtung aus einem Grundkörper geschaffen wird, welcher in einen Innenraum eines Aufnahmebehälters einsetzbar ist, bei welchem bis hin zum Erreichen der Arbeitsstellung stets ein Durchtritt eines der zu trennenden Bestandteile des Gemisches ermöglicht wird. Anschließend ist eine selbsttätig mechanische Anlage bzw. ein mechanisches Festsitzen an der inneren Oberfläche des Aufnahmebehälters in einer vorbestimmbaren Arbeitsstellung, bei der eine innere Abmessung des Innenraums des Aufnahmebehälters kleiner dem äußeren Umfang einer Hüll-Linie der Trennvorrichtung in der gleichen Stellung ist, sichergestellt. Dieses Festsitzen wird dann erreicht, wenn die beiden einander zugewandten Spaltflächen des Spaltes zur Anlage einander gebracht werden.

Vorteilhaft sind auch Weiterbildungen nach den Ansprüchen 51 bis 53, da durch die konische bzw. kegelige Ausbildung des Grundkörpers der Trennvorrichtung eine dichtende Anlage über nahezu die gesamte Anlagefläche desselben an der inneren Oberfläche des Aufnahmebehälters erzielbar ist.

Durch die Weiterbildung nach Anspruch 54 wird erreicht, daß ein Durchtritt eines der Medien zwischen den beiden voneinander distanzierten Endbereichen des Grundkörpers gesichert verhindert wird.

Durch die Ausbildung nach Anspruch 55 wird eine Anlage am Dichtelement der Verschlußvorrichtung ermöglicht, wodurch der Einfüllvorgang des zu trennenden Gemisches in den Innenraum der Aufnahmeeinrichtung erheblich verbessert sowie erleichtert wird, da so eine größere Benetzung mit dem Gemisch im Bereich des oberen Abschnitts der Trennvorrichtung verhindert werden kann.

Vorteilhaft ist auch eine Ausbildung nach Anspruch 56, da so einerseits der Befüllvorgang des zu trennenden Gemisches in den Innenraum als auch ein Durchtritt eines der Medien während des Zentrifugiervorganges durch den Grundkörper hindurch auf einfache Art ermöglicht wird.

Gemäß einer Ausbildung wie im Anspruch 57 und 58 beschrieben, wird in Verbindung mit einem Einsatzteil eine gesicherte Abdichtung bei einer gegenseitigen Anlage erzielt.

Dabei weist sich eine Ausgestaltung nach Anspruch 59 als vorteilhaft, da so das Rückstellverhalten und damit verbunden die Anlagekraft an der inneren Oberfläche des Aufnahmebehälters festgelegt werden kann.

Nach einer vorteilhaften Weiterbildung nach Anspruch 60 kann eine Ventilanordnung geschaffen werden, mit der es möglich ist, einerseits einen Durchtritt eines Bestandteiles des Gemisches durch die Trennvorrichtung hindurch zu ermöglichen und andererseits auch eine abgedichtete Trennung zu erzielen.

Gemäß Anspruch 61 wird ein Austritt des Einsatzteils aus der Verbindungsöffnung in Richtung des offenen Endes des Aufnahmebehälters bei darin eingesetzter Stellung verhindert.

Bei einer Ausbildung gemäß den Ansprüchen 62 oder 63 wird ein Austritt des Einsatzteils aus der Verbindungsöffnung in Richtung des verschlossenen Endes des Aufnahmebehälters verhindert, wobei jedoch stets eine Strömungsverbindung zwischen den beiden Endbereichen des Grundkörpers erhalten bleibt.

Möglich ist dabei aber auch eine Ausbildung nach Anspruch 64, da so nach Beendigung des Zentrifugiervorganges eine dichtende Trennung zwischen den beiden Bestandteilen innerhalb des Aufnahmebehälters erzielbar ist.

Die Ausgestaltung nach Anspruch 65 ermöglicht die Sinkgeschwindigkeit der Trennvorrichtung während des Zentrifugiervorganges innerhalb des Gemisches festzulegen.

Von Vorteil ist aber auch eine Ausbildung nach Anspruch 66, da so eine Abstimmung der Position bzw. Lage des Einsatzteils in bezug zum Grundkörper während des Zentrifugiervorganges festgelegt werden kann, insbesondere dann, wenn die Dichte des Einsatzteils geringfügig größer der Dichte des Grundkörpers gewählt ist, da so während des Zentrifugiervorganges bis kurz vor Beendigung desselben ein Durchfluß durch die Verbindungsöffnung zwischen den beiden voneinander getrennten Endbereichen bestehen bleibt.

Weitere vorteilhafte Ausbildungen des Einsatzteils sind in den Ansprüchen 67 und 68 gekennzeichnet, wobei hier stets eine dichtende Anlage zwischen dem Einsatzteil und der Verbindungsöffnung sichergestellt ist.

Diese Aufgabe der Erfindung wird aber auch durch die im Anspruch 69 angegebenen Merkmale gelöst. Vorteilhaft ist dabei, daß durch die Anordnung zumindest einer selbsttätig wirkenden Ventilanordnung durch den sich keilförmig erweiternden Spalt in der Ausgangsstellung der Trennvorrichtung stets ein Zugang zum Innenraum des Aufnahmebehälters möglich ist, wobei zusätzlich noch durch die gewählten Abmessungen zwischen der Trennvorrichtung und dem Aufnahmebehälter im Bereich der offenen Stirnseite eine vorbestimmbare Anlage- bzw. Haltekraft erzielbar ist. Durch das Zusammenwirken des sich konisch in Richtung des verschlossenen Endes verjüngenden Innenraums mit der gewählten Spaltgröße im Bereich der äußeren Anlagefläche kommt es nach einem vorbestimmbaren Verstellweg zu einem abmessungsbedingten mechanischen Festsitzen bzw. Festklemmen der Trennvorrichtung an der inneren Oberfläche des Aufnahmebehälters. Dieses Festsitzen bzw. Festklemmen wird dann erreicht, wenn die beiden Spaltflächen des Spaltes zur Anlage aneinander gebracht werden. Dies erfolgt durch die stete Verjüngung des Innenraums im Zusammenwirken mit den elastischen Verformungseigenschaften der Trennvorrichtung. Bis zum vollständigen Verschließen des Spaltes und dem damit bedingten Aneinanderliegen der einander zugewandten Spaltflächen ist ein Durchtritt des leichteren Mediums in den Innenraum zwischen der Trennvorrichtung und der Verschlußvorrichtung bzw. dem offenen Ende des Aufnahmebehälters möglich. Der Verstellweg kann durch die gegenseitige maßliche Abstimmung zwischen dem Innenkonus des Aufnahmebehälters sowie der Spaltgröße festgelegt werden, wobei jedoch ein sicheres Festsitzen vor Erreichen des verschlossenen Endes sicher gestellt sein muß. Durch diese vorbestimmbare Position der dichtenden Anlage der Spaltflächen aneinander sowie dem Festsitzen an der inneren Oberfläche des Aufnahmebehälters kann eine Position gewählt werden, bei welcher ein Durchtritt der schwereren Bestandteile des zu trennenden Gemisches auf alle Fälle verhindert ist. Diese Lage befindet sich zumeist im Bereich des Mittels zwischen den beiden Ebenen des Aufnahmebehälters.

Vorteilhaft ist auch eine weitere Ausführungsform nach Anspruch 70, da durch die Wahl der Größe der Verjüngung bzw. der Abnahme der inneren Querschnittsabmessung des Aufnahmebehälters der vorbestimmbare Verstellweg der Trennvorrichtung bis hin zu seiner Arbeitsstellung, in welcher eine allseits dichtende Abtrennung zwischen dem Innenraum, welcher zwischen der Trennvorrichtung und dem verschlossenen Ende bzw. zwischen der Trennvorrichtung und dem offenen Ende des Aufnahmebehälters angeordnet ist, erzielbar ist.

Vorteilhaft ist weiters eine Ausbildung nach Anspruch 71, da dadurch ein lageunabhängiges Einsetzen der Trennvorrichtung in den Innenraum des Aufnahmebehälters sowie eine einfache und kostengünstige Fertigung ermöglicht wird.

Durch die Ausbildung nach Anspruch 72 ist es möglich, jene Verstellkraft, die durch die Zentrifugalbeschleunigung auf die Trennvorrichtung einwirkt, festzulegen, die aufgewendet werden muß, um die Trennvorrichtung von der Ausgangsstellung zu einer Bewegung in Richtung der Arbeitsstellung zu verbringen.

Durch die Ausbildung nach Anspruch 73 ist es möglich, eine exakte Abstimmung zwischen den Abmessungen des Grundkörpers der Trennvorrichtung sowie des Aufnahmebehälters zu erzielen.

Bei der Ausgestaltung nach Anspruch 74 ist von Vorteil, daß durch die exakte Abstimmung zwischen den inneren Abmessungen des Aufnahmebehälters in Verbindung zur Spaltgröße die vorbestimmbare Position bzw. Lage der Arbeitsstellung der Trennvorrichtung innerhalb der Aufnahmeeinrichtung auf einfache Weise vorbestimmbar ist.

Eine verbesserte Lagefixierung der Trennvorrichtung im Bereich der Arbeitsstellung wird mit Vorteil gemäß der im Anspruch 75 und 76 angegebenen Merkmale erzielt. Dabei kann die Trennvorrichtung mit ihrem dem weiteren Ende des Aufnahmebehälters zugewandten Endbereich oder aber auch mit der Dichtungsvorrichtung auf dieser vorzugsweise als Anschlagfläche ausgebildeten Positioniervorrichtung bei Erreichen der vorbestimmbaren Arbeitsstellung zur Anlage kommen. Damit ist eine Weiterbewegung und damit verbunden eine ungewünschte Vermischung in jedem Fall gesichert verhindert.

Durch die unterschiedliche Wahl der Werkstoffe, wie dies in den Ansprüchen 77 und 78 beschrieben ist, ist es möglich, die Aufnahmeeinrichtung an die unterschiedlichsten Einsatzbedingungen abstimmen zu können.

Bei der Ausgestaltung gemäß der Ansprüche 79 oder 80 kann ein Anhaften von Bestandteilen des zu trennenden Gemisches am Grundkörper bzw. dem Einsatzteil gesichert verhindert werden.

Schließlich ist weiters eine Ausbildung nach Anspruch 81 vorteilhaft, da so durch das Ausmaß des Unterdruckes im Innenraum des Aufnahmebehälters die Menge des aufzunehmenden Gemisches festlegbar ist.

Die Erfindung wird im nachfolgenden anhand der in den Zeichnungen gezeigten Ausführungsbeispiele näher erläutert.

Es zeigen:
- Fig. 1: eine erfindungsgemäß ausgebildete Aufnahmeeinrichtung mit einer sich in der Ausgangsstellung befindlichen Trennvorrichtung sowie einer Verschlußvorrichtung, in schematisch vereinfachter Darstellung in Seitenansicht, geschnitten;
- Fig. 2: die Trennvorrichtung nach Fig. 1 in Seitenansicht, geschnitten gemäß den Linien II-II in Fig. 3 und in vergrößerter Darstellung;
- Fig. 3: die Trennvorrichtung nach Fig. 2 in Draufsicht;
- Fig. 4: einen Teilbereich des Grundkörpers im Bereich der Verbindungsöffnung in Draufsicht, geschnitten gemäß den Linien IV-IV in Fig. 2 und vergrößertem Maßstab;
- Fig. 5: eine andere Ausbildung der Rückhaltevorrichtung im Grundkörper in Draufsicht, geschnitten und vergrößertem Maßstab;
- Fig. 6: eine Trennvorrichtung mit einem anderen Einsatzteil und Rückhaltevorrichtung in Seitenansicht, geschnitten und in vergrößerter Darstellung;
- Fig. 7: die Aufnahmeeinrichtung nach erfolgter Trennung der Medien und bei einer in die Arbeitsstellung verstellten Trennvorrichtung;
- Fig. 8: die Trennvorrichtung in der Arbeitsstellung in Draufsicht, geschnitten gemäß den Linien VIII-VIII in Fig. 7;
- Fig. 9: eine weitere erfindungsgemäß ausgebildete Aufnahmeeinrichtung mit einer anderen sich in der Ausgangsstellung befindlichen Trennvorrichtung, sowie einer Verschlußvorrichtung, in schematisch vereinfachter Darstellung, in Seitenansicht geschnitten;
- Fig. 10: den Grundkörper der Trennvorrichtung nach Fig. 9 in Seitenansicht geschnitten gemäß den Linien X-X in Fig. 11 und vereinfachter vergrößerter Darstellung;
- Fig. 11: den Grundkörper nach Fig. 10 in Draufsicht;
- Fig. 12: den Einsatzteil der Trennvorrichtung nach Fig. 9 in Seitenansicht;
- Fig. 13: die Aufnahmeeinrichtung mit der Trennvorrichtung nach den Fig. 9 bis 12 in deren Arbeitsstellung;
- Fig. 14: eine andere erfindungsgemäße Ausbildung einer Trennvorrichtung in einer teilweise gezeigten Aufnahmeeinrichtung, in Seitenansicht geschnitten und in vereinfachter schematischer Darstellung;
- Fig. 15: den Grundkörper der Trennvorrichtung nach Fig. 14 in Draufsicht und bei entferntem Einsatzteil;
- Fig. 16: einen Teilbereich der Trennvorrichtung nach den Fig. 14 und 15 in deren Arbeitsstellung, in Seitenansicht geschnitten und vereinfachter schematischer Darstellung;
- Fig. 17: eine weitere erfindungsgemäß ausgebildete Aufnahmeeinrichtung mit einer anderen sich in der Ausgangsstellung befindlichen Trennvorrichtung sowie einer Verschlußvorrichtung, in schematisch vereinfachter Darstellung, in Seitenansicht geschnitten;
- Fig. 18: die Aufnahmeeinrichtung nach Fig. 17 in deren Arbeitsstellung, in Seitenansicht geschnitten und vereinfachter schematischer Darstellung;
- Fig. 19: eine andere erfindungsgemäße Ausbildung einer Aufnahmeeinrichtung mit einer in der Arbeitsstellung befindlichen Trennvorrichtung, in Seitenansicht geschnitten und vereinfachter schematischer Darstellung;
- Fig. 20: eine weitere erfindungsgemäß ausgebildete Aufnahmeeinrichtung mit einer anderen sich in der Ausgangsstellung befindlichen Trennvorrichtung bei entfernter Verschlußvorrichtung in schematisch vereinfachter Darstellung, in Seitenansicht geschnitten;
- Fig. 21: die Trennvorrichtung nach Fig. 20 in Draufsicht und vergrößerter schematisch vereinfachter Darstellung;
- Fig. 22: die Trennvorrichtung nach den Fig. 20 und 21 in Seitenansicht geschnitten, gemäß den Linien XXII-XXII in Fig. 21;
- Fig. 23: eine weitere erfindungsgemäße Ausbildung einer Trennvorrichtung in geschlossener Stellung des Strömungskanals in Seitenansicht geschnitten und vereinfachter, schematischer Darstellung;
- Fig. 24: eine andere erfindungsgemäße Ausbildung einer Aufnahmeeinrichtung, bei entfernter Verschlußvorrichtung sowie Trennvorrichtung, in Seitenansicht geschnitten und vereinfachter, schematischer Darstellung;
- Fig. 25: eine andere erfindungsgemäße Trennvorrichtung in einer Ansicht von unten und schematisch vereinfachter Darstellung;
- Fig. 26: die Trennvorrichtung nach Fig. 25 in Ansicht geschnitten, gemäß den Linien XXVI-XXVI in Fig. 25;
- Fig. 27: eine weitere erfindungsgemäße Trennvorrichtung in einer Ansicht von unten und vereinfacht schematischer Darstellung;
- Fig. 28: die Trennvorrichtung nach Fig. 27 in Ansicht geschnitten, gemäß den Linien XXVIII-XXVIII in Fig. 27;
- Fig. 29: eine andere erfindungsgemäß ausgebildete Trennvorrichtung in Draufsicht, teilweise geschnitten und vereinfacht schematischer Darstellung;
- Fig. 30: eine weitere erfindungsgemäße Ausbildung einer Aufnahmeeinrichtung bei entfernter Verschlußvorrichtung sowie Trennvorrichtung in Seitenansicht geschnitten und vereinfachter schematischer Darstellung;
- Fig. 31: eine weitere erfindungsgemäße Ausbildung der Trennvorrichtung in Draufsicht, teilweise geschnitten und vereinfachter schematischer Darstellung.

Einführend sei festgehalten, daß in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen.

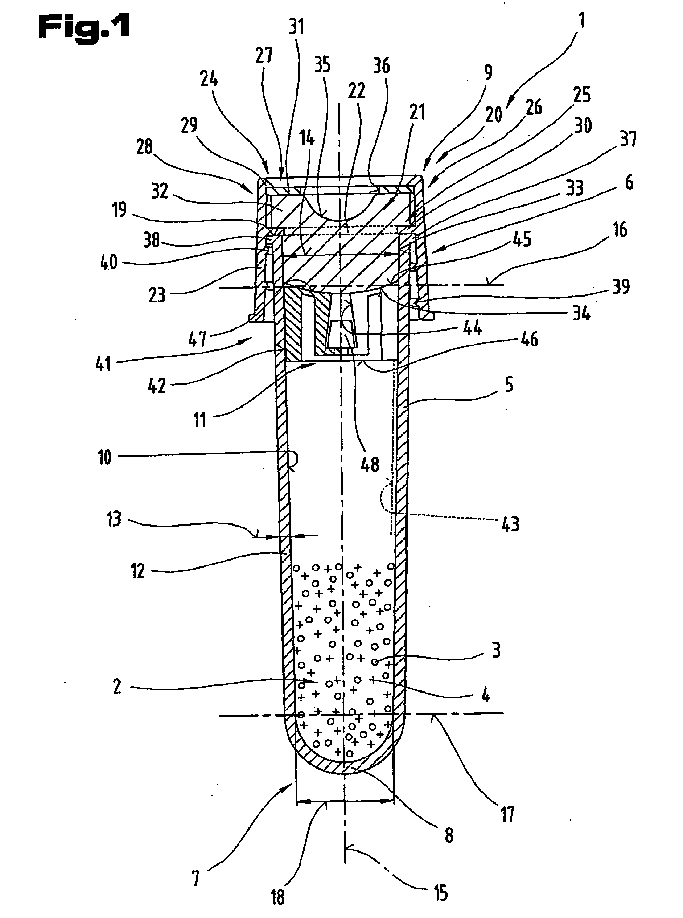

In den Fig. 1 bis 4 ist eine Aufnahmeeinrichtung 1 für ein Gemisch 2 aus zumindest zwei zueinander unterschiedlichen Bestandteilen bzw. Medien 3, 4, wie beispielsweise Körperflüssigkeiten, Gewebeteilen bzw. Gewebekulturen, gezeigt, welche derart ausgebildet ist, daß das sich in der Aufnahmeeinrichtung 1 befindliche Gemisch 2 in zumindest zwei seiner Bestandteile separierbar ist. Dieses Separieren bzw. Trennen des Gemisches 2 in seine Bestandteile bzw. Medien 3, 4 kann beispielsweise physikalisch durch Zentrifugierung auf herkömmliche Art und Weise erfolgen und ausgehend von der Ruheposition bis zum Erreichen einer radialen Zentrifugalbeschleunigung von 1.000 g bis 5.000 g, vorzugsweise 2.200 g, durchgeführt werden, wobei g die Erdbeschleunigung und der Wert von 1 g 9,81 m/s² beträgt. Dadurch ist es beispielsweise möglich, die festere Phase von der flüssigen Phase abzuscheiden bzw. nach den unterschiedlichen Dichtewerten zu trennen, wie dies in den nachfolgenden Figuren für unterschiedliche Ausführungsformen noch detaillierter beschrieben wird.

Die Aufnahmeeinrichtung 1 besteht aus einem in etwa zylinderförmig ausgebildeten Aufnahmebehälter 5 mit zwei voneinander distanzierten Enden 6, 7, wobei bei diesem Ausführungsbeispiel das Ende 6 offen ausgebildet und das Ende 7 durch eine Stirnwand 8 verschlossen ausgebildet ist. Das hier offene Ende 6 ist mit einer vereinfacht dargestellten Verschlußvorrichtung 9 bedarfsweise verschließbar und kann beispielsweise gemäß der EP 0 445 707 B1, der EP 0 419 490 B1, der US 5,275,299 A, der US 5,495,958 A sowie der US 5,522,518 A ausgebildet sein, wobei, um Wiederholungen zu vermeiden, auf die Offenbarung für die Ausbildung der Kappe, der Dichtungsvorrichtung, des Gehäuses bzw. Aufnahmebehälters, der Kupplungsvorrichtung zwischen der Kappe und der Dichtungsvorrichtung sowie der Kappe und dem Aufnahmebehälter 5 und des Halterings Bezug genommen und in die gegenständliche Anmeldung übernommen wird. In einen vom Aufnahmebehälter 5 umschlossenen Innenraum 10 ist eine Trennvorrichtung 11 eingesetzt, welche in der Ausgangsstellung unmittelbar benachbart zur Verschlußvorrichtung 9 angeordnet ist. Das verfahrensmäßige Vorgehen für den Zusammenbau bzw. die Montage wird nachfolgend noch detaillierter beschrieben. Dieser Aufnahmebehälter 5 mit der Verschlußvorrichtung 9 kann beispielsweise auch als evakuiertes Blutprobenentnahmeröhrchen in den verschiedensten Ausführungsformen ausgebildet bzw. eingesetzt sein.

Der Aufnahmebehälter 5 kann beispielsweise flaschen-, phiolen-, kolbenförmig oder dgl. ausgebildet sowie aus den unterschiedlichsten Materialien, wie beispielsweise Kunststoff oder Glas, gebildet sein. Wird für den Aufnahmebehälter 5 als Material Kunststoff gewählt, kann dieses flüssigkeitsdicht, insbesondere wasserdicht sowie gegebenenfalls gasdicht sein und beispielsweise aus Polyethylenterephthalat (PET), Polypropylen (PP), Polyethylen (PE), Polystyrol (PS), High-Density-Polyethylen (PE-HD), Acrylnitril-Butadien-Styrol-Copolymere (ABS) oder dgl. bzw. einer Kombination daraus bestehen. Weiters weist der Aufnahmebehälter 5 eine Behälterwand 12 mit einer Wandstärke 13 auf, wobei sich die Behälterwand 12, ausgehend von dem einen Ende 6 mit einer inneren Abmessung 14 in einer senkrecht zu einer zwischen den beiden Enden 6, 7 verlaufenden Längsachse 15 ausgerichteten Ebene 16 hin zu einer weiteren, im Bereich des Endes 7 angeordneten und parallel zur ersten Ebene 16 verlaufenden weiteren Ebene 17 mit einer dazu geringeren Abmessung 18 erstreckt. Die Behälterwand 12 des Aufnahmebehälters 5 weist eine dem Innenraum 10 zugewandte innere Oberfläche sowie eine davon abgewandte äußere Oberfläche auf, welche somit einen Außenumfang für den Aufnahmebehälter 5 festlegt. Auf Grund der inneren Oberfläche der Behälterwand mit der inneren lichten Abmessung 14, 18 ist somit ein innerer Querschnitt, welcher die unterschiedlichsten Querschnittsformen, wie z.B. kreisförmig, ellipsenformig, oval, mehreckig usw., aufweisen kann, festgelegt. Die Form des äußeren Querschnittes kann auch kreisförmig, ellipsenförmig, oval, mehreckig usw. ausgebildet sein, wobei es jedoch auch möglich ist, die Form des äußeren Querschnittes unterschiedlich zur Form des inneren Querschnittes auszuführen.

Vorteilhaft ist es, wenn die innere Abmessung 14 des Aufnahmebehälters 5, ausgehend vom einen Ende 6 hin zu dem von diesem distanzierten weiteren Ende 7 sich stetig minimal verringernd zur inneren Abmessung 18 ausgebildet ist, um beispielsweise den Aufnahmebehälter 5, wenn dieser aus Kunststoffmaterial in einem Spritzgußvorgang gefertigt ist, aus dem Spritzgußwerkzeug einfach entformen zu können. Weiters ist durch diese kegelige Verjüngung zwischen den beiden Ebenen 16, 17 das Ausmaß der Abnahme der inneren Abmessung ausgehend von der hier größeren Abmessung 14 zur kleineren Abmessung 18 vorbestimmt. Die Verjüngung bzw. der Kegelwinkel beträgt, bezogen auf die inneren gegenüberliegenden Oberflächen des Aufnahmebehälters 5, zwischen 0,1° und 3,0°, bevorzugt zwischen 0,6° und 0,8°. An dieser Stelle sei erwähnt, daß sich die beschriebenen Abmessungen auf den Abstand zwischen den sich einander gegenüberliegenden inneren bzw. äußeren Oberflächen der Grundkörper, den Durchmesser, den Umfang entlang einer Umhüllenden bzw. einer Hüll-Linie sowie den Querschnitt bzw. die Querschnittsfläche jeweils in einer der senkrecht zur Längsachse 15 ausgerichteten Ebenen sowie stets die gleiche Raumrichtung für die Ermittlung der Abmessungen beziehen können.

Wie weiters aus dieser Darstellung zu ersehen ist, weist das Ende 6 eine offene Stirnseite 19 auf, welche von der bedarfsweise öffenbaren Verschlußvorrichtung 9 verschließbar ist. Dazu besteht die Verschlußvorrichtung 9 aus einer die offene Stirnseite 19 umfassenden Kappe 20 und einer darin gehalterten Dichtungsvorrichtung 21, wie beispielsweise einem Dichtstopfen 22 aus einem durchstechbaren, hochelastischen und selbstverschließenden Werkstoff, wie z.B. Pharmagummi, Silikonkautschuk oder Brombutylkautschuk. Diese Kappe 20 ist konzentrisch zu der Längsachse 15 angeordnet und durch einen kreisringförmig ausgebildeten Kappenmantel 23 gebildet. Zwischen der Kappe 20 und der Dichtungsvorrichtung 21 sind Mittel zum Kuppeln, wie beispielsweise Kupplungsteile 24 bis 27 einer Kupplungsvorrichtung 28, bestehend bei der Kappe 20 aus zumindest über den Innenumfang bereichsweise angeordnete Fortsätze 29, 30, gegebenenfalls einem Haltering 31, und bei der Dichtungsvorrichtung 21 aus einem zumindest bereichsweise über dessen Außenumfang vorragenden Ansatz 32.

Die Dichtungsvorrichtung 21 ist im vorliegenden Ausführungsbeispiel durch den Dichtstopfen 22 gebildet und weist eine umlaufende und in etwa konzentrisch zur Längsachse 15 angeordnete zylinderförmige Dichtfläche 33 auf, welche in ihrer dichtenden Lage im Abschnitt des Endes 6 an der inneren Oberfläche des Aufnahmebehälters 5 zur Anlage kommt. Dadurch ist in diesem Abschnitt die innere Oberfläche des Aufnahmebehälters 5 in ihrer Oberflächengüte als Dichtfläche auszubilden. Weiters weist die Dichtungsvorrichtung 21 eine in etwa senkrecht zur Längsachse 15 ausgerichtete, weitere Dichtfläche 34 auf, welche im Zusammenwirken mit der an der inneren Oberfläche anliegenden Dichtfläche 33 den Innenraum 10 des Aufnahmebehälters 5 an dessen offener Stirnseite 19 gegenüber der äußeren Umgebung abschließt bzw. abdichtet. Durch die Anordnung des Fortsatzes 30 zwischen dem die Dichtfläche 33 überragenden Ansatz 32 und der offenen Stirnseite 19 des Aufnahmebehälters 5 kann eine Verklebung bzw. starke Anhaftung des Ansatzes 32 direkt an der Stirnseite 19 vermieden werden.

Des weiteren kann bevorzugt die Dichtungsvorrichtung 21 auf der dem Haltering 31 zugewandten Seite eine Vertiefung 35 aufweisen, die in etwa eine gleiche Querschnittsfläche wie eine Öffnung 36 aufweist, wobei diese Öffnung 36 in ihrer Abmessung derart ausgebildet ist, daß ein ungehindertes Hindurchführen einer hier nicht dargestellten Kanüle und anschließendes Hindurchstechen durch die Dichtungsvorrichtung 21 möglich ist.

Der den Kupplungsteil 26 bildende Ansatz 32, welcher über die Dichtfläche 33 der Dichtungsvorrichtung 21 zumindest in Teilbereichen des Umfanges flanschartig vorragt, ist zwischen den Fortsätzen 29 sowie 30 gehaltert, die in zwei in Richtung der Längsachse 15 voneinander distanzierten und senkrecht zu dieser ausgerichteten Ebenen angeordnet und beispielsweise als zumindest bereichsweise bzw. auch ringförmig umlaufende Vorsprünge bzw. Arretierfortsätze ausgebildet sind. Zur sicheren Halterung der Dichtungsvorrichtung 21 in der Kappe 20 ist es zusätzlich noch möglich, zwischen dem Ansatz 32 und dem Fortsatz 29 den Haltering 31 einzusetzen. Dabei weist der Haltering 31 einen größeren Außendurchmesser auf als eine sich zwischen den Fortsätzen 29 bzw. 30 ausbildende innere Abmessung in senkrechter Richtung zur Längsachse 15. Gleichfalls ist der Durchmesser der Öffnung 36 des Halterings 31 kleiner als eine größte Außenabmessung des Ansatzes 32 in einer Ebene senkrecht zur Längsachse 15. Diese äußere Abmessung der Dichtungsvorrichtung 21 ist jedoch so bemessen, daß diese zumindest um die doppelte Wandstärke 13 des Aufnahmebehälters 5 größer ist als die innere Abmessung 14 des inneren Querschnitts und somit des Innenraumes 10. Nachdem der Fortsatz 30, der den Kupplungsteil 25 bildet, eine innere Öffnungsweite aufweist, welche im wesentlichen der inneren Abmessung 14 des Aufnahmebehälters 5 in seinem oberen Ende 6 entspricht, kommt es zu einer sehr guten Halterung des Ansatzes 32 in der Kappe 20 sowie zu einer guten Abdichtung zwischen dem Innenraum 10 des Aufnahmebehälters 5 und der die Aufnahmeeinrichtung 1 umgebenden Atmosphäre.

Vor allem wird die Dichtheit der Verschlußvorrichtung 9 für die offene Stirnseite 19 der Aufnahmevorrichtung 1 noch dadurch verbessert, wenn ein äußerer Durchmesser der Dichtungsvorrichtung 21 im Bereich seiner Dichtfläche 33 im entspannten Zustand außerhalb des Aufnahmebehälters 5 größer ist als die innere Abmessung 14 des Aufnahmebehälters 5 in dem der Dichtungsvorrichtung 21 zugewandten Bereich.

Weiters ist im entspannten, unmontierten Zustand eine Längs- bzw. Höhenerstreckung des Ansatzes 32 der Dichtungsvorrichtung 21 in Richtung der Längsachse 15 größer als eine Distanz einer nutförmigen Vertiefung zwischen den beiden Fortsätzen 29, 30 sowie gegebenenfalls abzüglich einer Dicke des Halterings 31. Bedingt durch die zuvor beschriebenen Maßdifferenzen zwischen der nutförmigen Vertiefung und den Längenabmessungen des Ansatzes 32 bzw. der Dicke des Halterings 31 in Richtung der Längsachse 15 kommt es zu einer Vorspannung des Ansatzes 32 zwischen den beiden Fortsätzen 29, 30. Dies bewirkt gleichzeitig eine Verdichtung sowie Vorspannung der Dichtungsvorrichtung 21 in bezug zur Kappe 20 und bewirkt gegebenenfalls zusätzlich einen festen Sitz des Halterings 31 sowie auch eine satte Anlage der beiden Stirnflächen des Ansatzes 32 im Bereich der beiden Fortsätze 29, 30.

Von Vorteil ist es dabei weiters, wenn der Kappenmantel 23 als Zylinderstumpfmantel bzw. Kegelstumpfmantel ausgebildet ist, wodurch ein Übergreifen des Kappenmantels 23 im Bereich der oberen Stirnseite 19 gewährleistet ist.

Weiters kann es sich als vorteilhaft erweisen, wenn im Bereich der offenen Stirnseite 19 des Aufnahmebehälters 5 zumindest zwei Führungsfortsätze 3 7, 3 8 angeordnet sind, die über den Außenumfang des zylinderförmigen Aufnahmebehälters 5 vorspringen. Es ist aber jede beliebige andere Anzahl von Führungsfortsätzen 37, 38 möglich, wobei diese mit auf einer dem Aufnahmebehälter 5 zugewandten Innenfläche der Kappe 20 angeordneten und über deren Oberfläche in Richtung auf die Längsachse 15 vorspringenden Führungsstegen 39, 40 zusammenwirken. Dabei ist die Anzahl sowie die z.B. gleichmäßige, winkelversetzte Aufteilung der Führungsstege 39,40 über den Umfang von der Anzahl der am Aufnahmebehälter 5 angeordneten Führungsfortsätzen 37, 38 abhängig. Diese Führungsfortsätze 37, 38 wirken mit den auf der Innenseite des Kappenmantels 23 angeordneten Führungsstegen 39, 40 zusammen, wodurch es ermöglicht wird, daß bei einem Aufschieben der Kappe 20 in Richtung der Längsachse 15 des Aufnahmebehälters 5 in die offene Stirnseite 19 desselben und einem entsprechenden Verdrehen im Uhrzeigersinn die Führungsstege 39, 40 auf die Führungsfortsätze 37, 38 auflaufen, und daß bedingt durch die kombinierte Dreh- und Längsbewegung aufgrund der Führung der Führungsstege 39, 40 entlang der Führungsfortsätze 37, 38 die Dichtungsvorrichtung 21 mit ihrer Dichtfläche 33 in den Innenraum 10 des Aufnahmebehälters 5 eingesetzt bzw. eingeschoben werden kann.

Weiters ist im Innenraum 10 des Aufnahmebehälters 5 die Trennvorrichtung 11 mit ihrem Grundkörper 41 dargestellt, welcher eine dem Aufnahmebehälter 5 zugewandte Anlagefläche 42 aufweist. Vorteilhaft ist es, wenn das Material für den Grundkörper 41 elastisch rückstellbar verformbar ist und z.B. durch einen Silikonkautschuk, Pharmagummi, Brombutylkautschuk, Gummi, ein Gel oder einen elastomeren Kunststoff gebildet ist. Unabhängig davon kann aber auch ein Kunststoff gewählt werden, welcher flüssigkeitsdicht, insbesondere wasserdicht, sowie gegebenenfalls gasdicht sein kann und beispielsweise aus der Gruppe von Polyethylenterephthalat (PET), Polypropylen (PP), Polyethylen (PE), Polystyrol (PS), High-Density-Polyethylen (PE-HD), Acrylnitril-Butadien-Styrol-Copolymere (ABS) oder dgl. bzw. einer Kombination daraus gewählt werden. Gleichfalls können aber auch unterschiedlichste Zuschlagsstoffe dem Werkstoff zur exakten Abstimmung der vorbestimmbaren Dichte zugesetzt werden. Eine Dichte soll dabei zwischen 1,02 g/cm³ und 1,07 g/cm³, bevorzugt zwischen 1,04 g/cm³ und 1,05 g/cm³, betragen.

Weiters kann es aber auch vorteilhaft sein, wenn das Material des Grundkörpers 41 flüssigkeitsdicht und durch einen gegebenenfalls mit Zuschlagsstoffen bzw. Füllstoffen versehenen Kunststoff, wie z.B. ein Duroplast, ein glasklares Polystyrol oder dgl., gebildet ist. Weiters soll der Grundkörper 41 eine Gaspermeabilität aufweisen, die den Durchtritt von Gasen zumindest in einem Zeitraum von 48 bzw. 72 Stunden nahezu verhindert. Als vorteilhaft kann es sich weiters erweisen, wenn das Gesamtgewicht des Grundkörpers 41 und/oder der Trennvorrichtung 11 veränderbar ist, wodurch es beispielsweise möglich ist, die Trennvorrichtung 11 und/oder den Grundkörper 41 auf unterschiedliche Medien 3, 4 des zu trennenden Gemisches 2 exakt abzustimmen. Um einen exakten physikalischen Trennvorgang der beiden Medien 3, 4 des Gemisches 2 während des Zentrifugiervorganges zu erreichen, muß das spezifische Gewicht bzw. die Dichte des Materials des Grundkörpers 41 einerseits kleiner sein als das höhere spezifische Gewicht bzw. die Dichte eines durch die Trennvorrichtung 11 zu trennenden Mediums 3, 4 und andererseits größer sein als das leichtere spezifische Gewicht bzw. die Dichte eines durch die Trennvorrichtung zu trennenden Mediums 3, 4.

Je nach dem zu trennenden Gemisch 2 aus den unterschiedlichen Medien 3, 4 bzw. Bestandteilen kann es sich als vorteilhaft erweisen, wenn zumindest Teilbereiche oder die gesamte innere Oberfläche des Innenraumes 10 mit einer Beschichtung 43 versehen ist, um so beispielsweise die Gleitbewegung der Trennvorrichtung 11 während des Trennvorganges zu unterstützen und/oder eine chemische und/oder physikalische Beeinflussung des Gemisches 2 oder dgl. zu bewirken. Bei der eingesetzten Stellung des Grundkörpers 41 in die Aufnahmeeinrichtung 1 im Bereich der offenen Stirnseite 19 kann zumindest die sich zwischen der Trennvorrichtung 11 und dem gegenüberliegenden Ende 7 befindliche Oberfläche mit dieser Beschichtung 43 versehen sein, welche beispielsweise bei Kontakt mit dem Gemisch 2 von der Oberfläche ablösbar bzw. auflösbar ausgebildet ist und z.B. auch gleichzeitig zur Fixierung der Trennvorrichtung 11 herangezogen werden kann.

Der Grundkörper 41 der Trennvorrichtung 11 weist bei diesem Ausführungsbeispiel in seinem Zentrum bzw. im Bereich der Längsachse 15 eine Verbindungsöffnung 44 zwischen in Richtung der Längsachse 15 voneinander distanzierten Endbereichen 45, 46 auf, wobei im hier dargestellten Endbereich 45 zusätzlich noch eine konkav ausgebildete Ausnehmung 47 angeordnet sein kann, welche in ihrer Form dem diesen zugewandten Teil der Dichtungsvorrichtung 21, beispielsweise des Dichtstopfens 22 der Verschlußvorrichtung 9 nahezu angepaßt ist bzw. dieser entspricht. Dabei ist es vorteilhaft, wenn die Ausnehmung 47 in ihrer Ausgangsstellung in einer nahezu anliegenden Position an der Dichtfläche 34 des Dichtstopfens 22 gebracht wird und so bei einem Durchstechen des Dichtstopfens 22 mit einer hier nicht näher dargestellten Kanüle eine Verbindung zwischen dieser Kanüle und der Verbindungsöffnung 44 im Grundkörper 41 hergestellt werden kann. Weiters ist hier noch vereinfacht dargestellt, daß in der Verbindungsöffnung 44 ein eigener Einsatzteil 48 eingesetzt bzw. eingebracht ist, wobei die detaillierte Ausbildung des Grundkörpers 41 sowie des Einsatzteiles 48 in den nachfolgenden Figuren noch detailliert beschrieben werden wird.

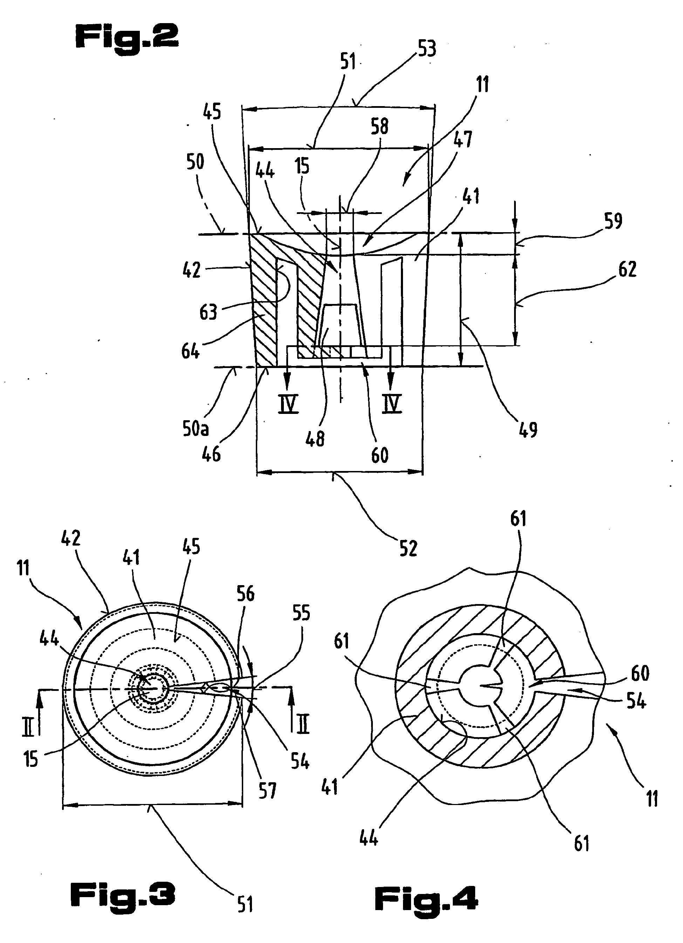

Die in den Fig. 2 und 3 gezeigte Trennvorrichtung 11 ist eine mögliche und gegebenenfalls für sich eigenständige Ausführung in einer vereinfachten und vergrößerten Darstellung, wobei für gleiche Teile gleiche Bezugszeichen wie in der Fig. 1 verwendet werden.

Der Grundkörper 41 weist in Richtung der Längsachse 15 die voneinander distanzierten Endbereiche 45, 46 auf, welche somit um eine Distanz 49 bzw. Höhe voneinander beabstandet sind. Im hier ersten bzw. oberen Endbereich 45 weist der Grundkörper 41 in einer senkrecht zur Längsachse 15 ausgerichteten Ebene 50 eine äußere Abmessung 51 auf, welche größer ist als eine weitere äußere Abmessung 52 im Endbereich 46 in einer weiteren, ebenfalls zur Längsachse 15 senkrecht ausgerichteten sowie parallel zur ersten Ebene 50 verlaufenden weiteren Ebene 50a. Da die Querschnitte in den zuvor beschriebenen Ebenen 16, 17 des Aufnahmebehälters 5 bzw. den Ebenen 50, 50a des Grundkörpers 41 nahezu kreisförmig ausgebildet sind, ist ein lageunabhängiges Einsetzen der Trennvorrichtung 11 in den Innenraum 10 des Aufnahmebehälters 5 einfach möglich. Dadurch weist der Grundkörper 41 im Bereich seiner Anlagefläche 42 die Form eines Kegelstumpfes auf, welcher einen Kegelwinkel 53 zwischen 0,1° und 3,0°, bevorzugt zwischen 0,6° und 0,8°, aufweist. Dieser Kegelwinkel 53 kann im ungespannten bzw. unverformten Zustand des Grundkörpers 41 der Verjüngung des Innenraumes 10 des Aufnahmebehälters 5 zwischen den beiden voneinander distanzierten Ebenen 16, 17 entsprechen.

Als vorteilhaft kann es sich erweisen, wenn der Kegelwinkel 53 des Grundkörpers 41 geringfügig größer der Verjüngung des Innenraumes 10 ist, da so eine Verkantung des Grundkörpers 41 im Übergangsbereich zwischen dem Endbereich 46 und der Anlagefläche 42 an der inneren Oberfläche des Aufnahmebehälters 5 gesichert verhindert wird. Durch die Wahl der Werkstoffe für den Aufnahmebehälter 5 bzw. die Trennvorrichtung 11, insbesondere den Grundkörper 41, kann eine entsprechende Abstimmung in bezug auf das Elastizitätsverhalten und damit verbunden auf die von der Anlagefläche 42 auf die innere Oberfläche des Aufnahmebehälters 11 aufgebrachte Druck- bzw. Reibungskraft und der damit verbundenen Dichtwirkung erzielt werden.

Wesentlich dabei ist, daß ein innerer Umfang bzw. eine innere Abmessung 14 des Aufnahmebehälters 5 im Bereich der ersten Ebene 16, 50 gleich oder kleiner einem äußeren Umfang bzw. einer äußeren Abmessung 51 des Grundkörpers 41 in seinem unverformten Zustand in der gleichen Ebene 16, 50 ist. Dies bedeutet, daß die äußere Abmessung 51 des Grundkörpers 41 in der senkrecht zur Längsachse 15 ausgerichteten Ebene 50 in seinem ersten Endbereich 45 im unverformten Zustand gleich oder größer der inneren Abmessung 14 des Aufnahmebehälters 5 in seinem ersten offenen Ende 6 in der gleichen Ebene 16 ist.

In der Fig. 3 ist der Grundkörper 41 in einer Ansicht von oben dargestellt, wobei in diesem ein sich zwischen den beiden Endbereichen 45, 46 erstreckender und ausgehend von seinem Zentrum bzw. der Längsachse 15 bis hin zur Anlagefläche 42 insbesondere keilförmig erweiternder Spalt 54 angeordnet ist. Wie bereits zuvor beschrieben, ist auf Grund der gewählten Umfangsgröße bzw. der äußeren Abmessung 51 des Grundkörpers 41 in bezug zur inneren Abmessung 14 des Aufnahmebehälter 5 in der in der Fig. 1 dargestellten Ebene 16 in Verbindung mit dem Spalt 54 eine gegenseitige Abstimmung zwischen diesen Grundkörpern einfach möglich. Bei gleichen inneren bzw. äußeren Abmessungen 14, 51 ist eine Anlage der Anlagefläche 42 mit geringen Haltekräften zwischen der Anlagefläche 42 und der inneren Oberfläche des Aufnahmebehälters 5 im Bereich der Ausgangsstellung möglich.

Wird die äußere Abmessung 51 bzw. der äußere Umfang des Grundkörpers 41 im unverformten bzw. ungespannten Zustand größer der inneren Abmessung 14 in der Ausgangsstellung gewählt, so ist eine vordefinierte Haltekraft durch die elastische Verformung des Grundkörpers 41 im Zusammenwirken mit dem Spalt 54 zwischen der Anlagefläche 42 des Grundkörpers 41 und der inneren Oberfläche des Aufnahmebehälters 5 erzielbar. Durch die Wahl der Vorspannung bzw. Unterschieden in den Abmessungen kann somit die infolge der Fliehkraftwirkung aufzubringende Verstellkraft in Richtung der Längsachse 15 festgelegt werden, die aufgebracht werden muß, um einen Verstellvorgang bzw. eine Verlagerung aus der Ausgangsstellung hin in Richtung der Arbeitsstellung zu erzielen.

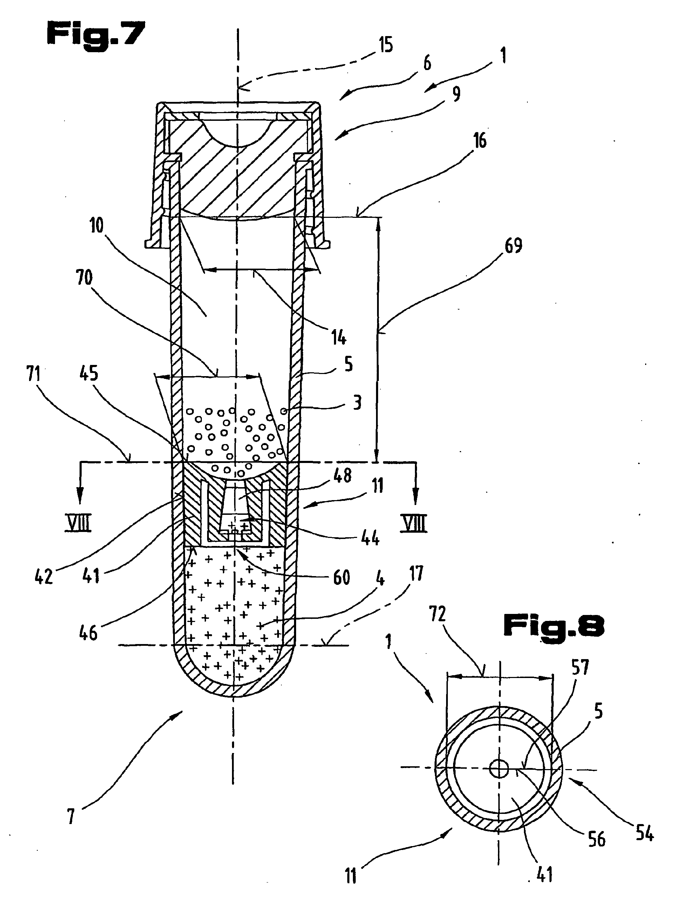

Weiters ist in der Fig. 3 dargestellt, daß eine Bogenlänge 55 des Spaltes 54 im Bereich der Anlagefläche 42 in der im Aufnahmebehälter 5 und in Fig. 1 dargestellten Ausgangsstellung gleich der Umfangsdifferenz zwischen dem inneren Umfang des Aufnahmebehälters 5 in der senkrecht zur Längsachse 15 ausgerichteten Ebene 16 im Bereich der Ausgangsstellung sowie dem in der Fig. 7 gezeigten inneren Umfang des Aufnahmebehälters 5 im Bereich der Arbeitsstellung ist, wie dies nachfolgend noch gezeigt und beschrieben werden wird.

Der Spalt 54 weist diesen begrenzende Spaltflächen 56, 57 auf, zwischen welchen während der vorbestimmbaren Verstellbewegung ausgehend von der Ausgangsstellung hin in die Arbeitsstellung ein Durchtritt für eines der beiden zu separierenden Medien - hier im vorliegenden Ausführungsbeispiel das leichtere Medium 3 - möglich ist.

Weiters ist aus einer Zusammenschau der Fig. 2 und 3 zu ersehen, daß im Grundkörper 41 in seinem Zentrum bzw. im Bereich der Längsachse 15 die in der Fig. 1 bereits kurz beschriebene Verbindungsöffnung 44 angeordnet ist. Durch diese Verbindungsöffnung 44 ist es möglich, den Befüllvorgang durch den Grundkörper 41 der Trennvorrichtung 11 hindurch in den Innenraum 10 des Aufnahmebehälters 5 zu ermöglichen. Diese Verbindungsöffnung 44 weist in dem, dem ersten Endbereich 45, zugewandten Abschnitt eine lichte Weite 58 auf, welcher derart bemessen ist, daß ein ungehindertes Hindurchfließen des in den Innenraum 10 einzufüllenden Gemisches 2 ermöglicht wird. Weiters ist hier zu ersehen, daß die Verbindungsöffnung 44, ausgehend vom ersten Endbereich 45 mit der lichten Weite 58 in Richtung des weiteren Endbereiches 46 sich erweiternd, insbesondere kegelstumpfförmig, ausgebildet ist. Wie bereits zuvor beschrieben, weist der Grundkörper 41 in seinem ersten Endbereich 45 die konkave Ausnehmung 47 auf, welche in ihrer Form der Dichtfläche 34 des Dichtstopfens 22 angepaßt ist und im Bereich der Längsachse 15, ausgehend von der Ebene 50 in Richtung der Längsachse 15 eine Tiefe 59 aufweist. Der sich erweiternde Bereich der Verbindungsöffnung 44 erstreckt sich über einen Teilbereich der Distanz 49 abzüglich der Tiefe 59 zwischen den beiden Endbereichen 45 und 46 bzw. den Ebenen 50 und 50a.

In dem sich hier kegelstumpfförmig erweiternden Abschnitt der Verbindungsöffnung 44 ist der Einsatzteil 48 dargestellt, welcher sich in einer im Abschnitt des Endbereiches 46 nähergelegenen Position befindet. In der Gebrauchs- bzw. Normalstellung der Aufnahmeeinrichtung 1 ist stets das erste Ende 6 höher als das weitere Ende 7, wobei infolge der Erdanziehung bzw. Schwerkraft des Einsatzteiles 48 dieser sich stets in einer Lage bzw. Position im Nahbereich des Endbereiches 46 befindet.

Um einen Austritt des Einsatzteiles 48 aus der Verbindungsöffnung 44 in Richtung des Innenraumes 10 des Aufnahmebehälters 5 zu verhindern, ist in der Fig. 4 eine vereinfacht dargestellte Rückhaltevorrichtung 60 dargestellt, welche im Endbereich 46 in die Verbindungsöffnung 44 hineinragt. Diese hier dargestellte Ausbildung ist nur eine von vielen Möglichkeiten, wobei dies gegebenenfalls für sich eine eigenständige erfindungsgemäße Ausbildung darstellen kann. Dadurch ist der Einsatzteil 48 in seiner Bewegungsmöglichkeit in Richtung der Längsachse 15 in diesem Abschnitt des Grundkörpers 41 gehalten, wobei die Rückhaltevorrichtung 60 derart ausgebildet ist, daß stets ein Durchfluß- bzw. Strömungskanal in jeder beliebigen Lage des Einsatzteiles 48 ausgebildet ist und durch die Verbindungsöffnung 44 hindurch eine Strömung möglich ist. Bei dem in der Fig. 4 dargestellten Ausführungsbeispiel sind mehrere Stege 61 - hier drei Stege - vorgesehen, welche am Grundkörper 41 angeordnet, insbesondere angeformt sind. Bei der Anordnung und Ausführung der Stege 61 ist auch noch auf den zuvor beschriebenen Spalt 54 Rücksicht zu nehmen, um die Schließbewegung des Spaltes 54 bis zu einer Anlage der beiden einander zugewandten Spaltflächen 56, 57 zu ermöglichen, wie dies noch nachfolgend für die Arbeitsstellung detailliert beschrieben werden wird. Dabei wird durch die Verbindungsöffnung 44 und/oder den Spalt 54 jeweils ein bedarfsweise verschließbarer Strömungskanal ausgebildet, der in zumindest einer selbsttätig wirkenden Ventilanordnung verschließbar ist. Dies kann durch die Anlage des Einsatzteils 48 an den Begrenzungswänden der Verbindungsöffnung 44 und/oder durch die Anlage der beiden Spaltflächen 56, 57 aneinander erfolgen.

Die äußere Abmessung des Einsatzteiles 48 ist in der Fig. 4 in strichlierten Linien vereinfacht dargestellt, wobei hier die einfache Durchströmmöglichkeit zwischen der äußeren Oberfläche des Einsatzteiles 48 und der Verbindungsöffnung 44 ersichtlich ist. Dadurch ist auch bei einer Anlage des Einsatzteiles 48 an der Rückhaltevorrichtung 60 eine Strömungsverbindung zwischen den beiden voneinander distanzierten Endbereichen 45, 46 des Grundkörpers 41 durch die Verbindungsöffnung 44 hindurch möglich.

Vorteilhaft ist es, wenn die lichte Weite 58 (siehe Fig. 2) der Verbindungsöffnung 44 im ersten Endbereich 45 in der senkrecht zur Längsachse 15 ausgerichteten Ebene 50 in der Ausgangsstellung des Grundkörpers 41 bzw. der Trennvorrichtung 11 gleich oder kleiner der äußeren Abmessung des Einsatzteiles 48, insbesondere des dem Endbereich 45 zugewandten Teils des Einsatzteiles 48, ist. Dadurch ist ein Durchtritt bzw. ein Austritt des Einsatzteiles 48 aus der Verbindungsöffnung 44 aus dem Grundkörper 41 auch hier verhindert.

Bei dem hier gezeigt Ausführungsbeispiel ist der Einsatzteil 48 als Kegelstumpf ausgebildet und derart bemessen, daß im unverformten Zustand des Grundkörpers 41 bzw. in jener Position, in welcher sich die Trennvorrichtung 11 in der Ausgangsstellung vor Beginn des Zentrifugiervorganges befindet, über einen Teilbereich einer Länge 62 (siehe Fig. 2) innerhalb der Verbindungsöffnung 44 in Richtung der Längsachse 15 eine Verlagerung möglich ist.

Der in den Fig. 2 und 3 dargestellte Grundkörper 41 weist zwischen der äußeren Anlagefläche 42 und der Verbindungsöffnung 44 in Richtung der Längsachse 15 eine in etwa kreisringförmig ausgebildete Vertiefung 63 auf, welche sich ausgehend vom weiteren Endbereich 46 in Richtung des ersten Endbereiches 45 erstreckt. Dadurch bildet sich ein Mantelteil 64 im Bereich des äußeren Umfanges des Grundkörpers 41 aus. Die Anordnung und Ausbildung der Vertiefung 63 kann vorgesehen sein und hängt von der Wahl des Werkstoffes zur Bildung des Grundkörpers 41, der gewählten Dichte sowie dem damit verbundenen Gewicht zusammen und ist vom Anwendungsfall zu Anwendungsfall frei wählbar.

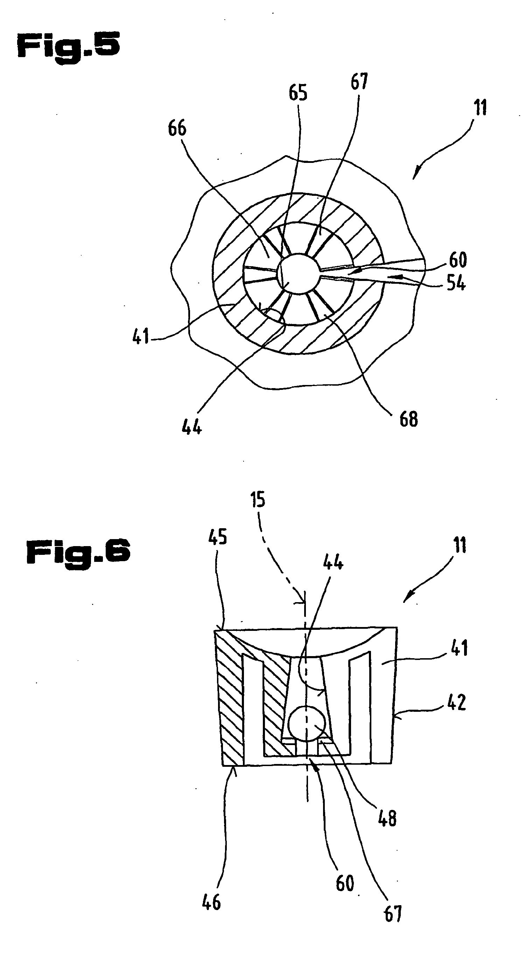

In der Fig. 5 ist eine weitere mögliche und gegebenenfalls für sich eigenständige Ausbildung der Rückhaltevorrichtung 60 für die Anordnung in der Verbindungsöffnung 44 des Grundkörpers 41 gezeigt, wobei für gleiche Teile gleiche Bezugszeichen wie in den vorangegangenen Fig. 1 bis 4 verwendet werden.

Bei diesem hier gezeigten Ausführungsbeispiel ist die Verbindungsöffnung 44 zur Aufnahme des hier nicht dargestellten Einsatzteiles 48 vorgesehen und kann auch wiederum über einen Teilbereich der Distanz 49 kegelstumpfförmig erweiternd, ausgehend vom Endbereich 45 hin zum weiteren Endbereich 46 ausgebildet sein, wie dies bereits in den Fig. 2 bis 4 detailliert beschrieben worden ist. Die Verbindungsöffnung 44 ist in ihrem Querschnitt zur Ausbildung der Rückhaltevorrichtung 60 mit einem verringerten Querschnitt zur Ausbildung eines Durchbruches 65 ausgebildet, wobei zwischen dem Durchbruch 65 und der Verbindungsöffnung 44 ein Wandteil 66 im Endbereich 46 des Grundkörpers 41 ausgebildet ist. Auf dem dem ersten Endbereich zugewandten Wandteil 66 können über diesen vorragend und/oder vertieft in diesem mehrere Stege bzw. Rippen 67 bzw. Nuten 68 angeordnet sein. Damit kann ein vollständiges Verschließen des Durchbruches 65 durch die Anlage des Einsatzteiles 48 am Wandteil 66 verhindert werden. Durch eine mögliche kombinierte Anordnung der Rippen 67 bzw. der Nuten 68 kann ein erhöhtes Durchströmvolumen zwischen dem Einsatzteil 48 und dem Wandteil 66 hin zum Durchbruch 65 erzielt werden. Gleichfalls ist aber auch eine abwechselnde Anordnung über den Umfang des Durchbruches 65 bzw. der Verbindungsöffnung 44 zwischen den Rippen 67 bzw. den Nuten 68 möglich.

In der Fig. 6 ist eine ähnliche Ausführungsform des Grundkörpers 41 zur Bildung der Trennvorrichtung 11 gezeigt, wie dies bereits in den Fig. 2 bis 4 beschrieben worden ist, jedoch im Gegensatz dazu der Einsatzteil 48 eine andere Raumform aufweist.

Im vorliegenden Ausführungsbeispiel weist hier der Einsatzteil 48 die Raumform einer Kugel auf, welche in einer Stellung gezeigt ist, die unmittelbar benachbart dem Endbereich 46 ist. Auch hier ist es wiederum für die Strömungsverbindung zwischen den beiden Endbereichen 45, 46 durch die Verbindungsöffnung 44 hindurch notwendig, die Rückhaltevorrichtung 60 gemäß einer der zuvor beschriebenen Ausführungsformen auszubilden. Dies kann beispielsweise durch die Anordnung mehrerer Stege 61 bzw. des Wandteiles 66 mit den darauf angeordneten Rippen 67 und/oder vertieft angeordneten Nuten 68 erfolgen.

In den Fig. 7 und 8 ist die Aufnahmeeinrichtung 1 mit der darin angeordneten Trennvorrichtung 11 dargestellt, bei welcher das in den Innenraum 10 eingefüllte Gemisch 2 gemäß der Fig. 1 durch Beaufschlagung mit Fliehkraft, insbesondere eines Zentrifugiervorganges, auf die beiden Medien 3, 4 aufgeteilt bzw. getrennt worden ist. Dabei ist das leichtere Medium 3 im Innenraum 10 zwischen der Trennvorrichtung 11 und dem ersten Ende 6 bzw. der Verschlußvorrichtung 9 und das weitere und schwerere Medium 4 zwischen der Trennvorrichtung 11 und dem hier verschlossenen Ende 7 im Aufnahmebehälter 5 beinhaltet.

Wie bereits zuvor beschrieben, weist der Spalt 54 (siehe Fig. 3) in der ersten Ebene 16 zwischen den Spaltflächen 56, 57 die Bogenlänge 55 im Bereich der Anlagefläche 42 auf. Ausgehend von der Ebene 16 erfolgt die Verstellung der Trennvorrichtung 11 in Richtung des weiteren Endes 7, wodurch, bedingt durch die Konizität des Innenraumes 10, eine stetige Abnahme des inneren Umfanges erfolgt und nach einer Verstellung um einen Verstellweg 69 die beiden den Spalt 54 ausbildenden Spaltflächen 56, 57 zur Anlage aneinander gebracht werden. Dadurch ist es nunmehr möglich, während der Verstellung bzw. Verlagerung der Trennvorrichtung 11, ausgehend von der Ausgangsstellung bis hin zu der in der Fig. 7 dargestellten Arbeitsstellung durch die stete Abnahme von der inneren Abmessung 14 eine kontinuierliche Abnahme der Bogenlänge 55 (siehe Fig. 3) des Spaltes 54 festzulegen, bis daß eine satte und bevorzugt dichtende Anlage der beiden Spaltflächen 56, 57 aneinander erzielt ist.

Beim Erreichen des Aneinanderliegens ist die einfache elastische Verstellung bzw. Verringerung der Bogenlänge 55 des Spaltes 54 beendet und es entspricht eine lichte innere Abmessung 70 bzw. der innere Umfang des Aufnahmebehälters 5 in der im Bereich der Arbeitsstellung verlaufenden und senkrecht zur Längsachse 15 ausgerichteten Ebene 71 einer äußeren Abmessung 72 bzw. dem Umfang des Grundkörpers 41 in der geschlossenen Stellung des Spaltes 54.

Durch die vorbestimmbare Konizität des Innenraumes des Aufnahmebehälters 5 sowie der vorwählbaren Bogenlänge 55 des Spaltes 54 im Grundkörper 41 ist der Verstellweg 69 exakt vorherbestimmbar, um welchen die Trennvorrichtung 11, ausgehend von der Ausgangsstellung hin zur Arbeitsstellung verlagerbar ist, wodurch eine mechanische Sperre bzw. Verriegelung oder Halterung innerhalb des Aufnahmebehälters 5 gewährleistet ist. Durch den vorherbestimmbaren Verstellweg 69 ist es möglich, unabhängig von der Befüllmenge die Lage und damit die verbundene Position der Trennvorrichtung für die Arbeitsstellung festzulegen, ohne daß dabei Bestandteile des Gemisches 2, insbesondere das Medium 4, in den Raum zwischen der Trennvorrichtung 11 und dem ersten Ende 6 bzw. der Verschlußvorrichtung 9 gelangen kann. Bevorzugt liegt dieser Verstellweg 69 ca. in der Hälfte des Abstandes zwischen den Ebenen 16 und 17.

Während des Zentrifugiervorganges wandert die Trennvorrichtung 11 entlang der inneren Oberfläche des Aufnahmebehälters 5 in Richtung der Längsachse 15 hin zur Arbeitsstellung, wobei ein Durchtritt des Mediums 3 durch den Spalt 54 in den Raum zwischen der Trennvorrichtung 11 und der Verschlußvorrichtung 9 bzw. dem ersten Ende 6 möglich ist. Weiters kann auch noch ein Durchtritt des hier leichteren Mediums 3 durch die Verbindungsöffnung 44 möglich sein, da auf Grund der auf den Einsatzteil 48 wirkenden Fliehkraft dieser in den Bereich der Rückhaltevorrichtung 60 verlagert wird. Vorteilhaft ist es, wenn die Dichte des Einsatzteiles 48 einen Wert aufweist, welcher geringer ist als das schwerere der beiden Medien 3, 4 und größer ist als das des leichteren Mediums.

Unabhängig davon ist es aber auch möglich, die Dichte des Einsatzteiles 48 geringer als die Dichte des leichteren Mediums - hier im vorliegenden Fall des Mediums 3 - zu wählen, da so auf alle Fälle der Einsatzteil 48 auf diesem Medium schwimmt und in der Verbindungsöffnung 44 in Richtung des Endbereiches 45 verlagert wird. Durch die gegengleich konische Ausbildung wird eine Abdichtung der Verbindungsöffnung 44 zwischen den beiden voneinander distanzierten Endbereichen 45, 46 erzielt. Dies wird noch dadurch verstärkt, daß durch die Verringerung des Spaltes 54 bis hin zur Anlage der beiden Spaltflächen 56, 57 aneinander der Querschnitt der Verbindungsöffnung 44 geringfügig verringert wird und so eine zusätzliche Klemmkraft zwischen dem Grundkörper 41 und dem Einsatzteil 48 im Abschnitt der gegenseitigen Anlageflächen erzielbar ist. Somit liegt der Einsatzteil 48 in der Arbeitsstellung dichtend, insbesondere flüssigkeitsdicht, an den Begrenzungswänden des sich erweiternden Abschnittes der Verbindungsöffnung 44 an.

Als Material bzw. Werkstoff für den Einsatzteil 48 kann bevorzugt ein Kunststoff gewählt werden, welcher flüssigkeitsdicht, insbesondere wasserdicht, sowie gegebenenfalls gasdicht sein kann und beispielsweise aus der Gruppe von Polyethylenterephthalat (PET), Polypropylen (PP), Polyethylen (PE), High Density Polyethylen (PE-HD), Acrymitril-Butadien-Styrol-Copolymere (ABS), Polystyrol (PS) oder dgl. bzw. eine Kombination daraus gewählt werden. Gleichfalls können aber auch unterschiedlichste Zuschlagsstoffe dem Werkstoff zur exakten Abstimmung der vorbestimmbaren Dichte zugesetzt werden. Eine Dichte soll dabei zwischen 1,02 g/cm³ und 1,07 g/cm³, bevorzugt zwischen 1,04 g/cm³ und 1,05 g/cm³ betragen. Vorteilhaft ist es weiters, wenn die Dichte des Einsatzteils 48 in bezug zur Dichte des Grundkörpers 41 geringfügig größer gewählt wird, da so bis kurz vor dem Erreichen der Arbeitsstellung stets ein Durchfluß durch die Verbindungsöffnung 44 zwischen den beiden durch die Trennvorrichtung voneinander getrennten Innenräume des Aufnahmebehälters 5 möglich ist.

Als vorteilhaft hat sich auch noch erwiesen, wenn der Grundkörper 41 und/oder der Einsatzteil 48 zumindest bereichsweise mit einer Beschichtung, wie beispielsweise einer Silikonschicht versehen ist, da so während des Zentrifugiervorganges keine Blutzellen an diesen Grundkörpern haften bleiben und so eine Verunreinigung des zwischen der Verschlußvorrichtung 9 und der Trennvorrichtung 11 separierten Mediums 3 verhindert wird.

Für den Zusammenbau der gesamten Aufnahmeeinrichtung 1 kann wie folgt vorgegangen werden:
Dabei wird in den vorbereiteten Aufnahmebehälter 5 in das offene Ende desselben die Trennvorrichtung 11 eingesetzt, daran anschließend der Innenraum 10 des Aufnahmebehälters 5 auf einen gegenüber dem atmosphärischen Druck geringeren Druck abgesenkt, wobei hier vorteilhaft der gesamte Umgebungsraum um den Aufnahmebehälter 5 auf diesen Unterdruck evakuiert bzw. abgesenkt wird und daran anschließend zur Bewahrung des Unterdruckes die Verschlußvorrichtung 9 dichtend in das offene Ende des Aufnahmebehälters 5 eingesetzt wird. Durch die zuvor beschriebene Anordnung des Spaltes 54 im Grundkörper 41 kann nach dem Einsetzen derselben in den Innenraum 10 dieser noch auf den gewünschten Unterdruck evakuiert werden und erst anschließend daran die dichtende Verschlußvorrichtung zur Aufrechterhaltung des Unterdruckes auf den Aufnahmebehälter 5 an dessen offenen Stirnseite 19 aufgesetzt bzw. eingesetzt werden.

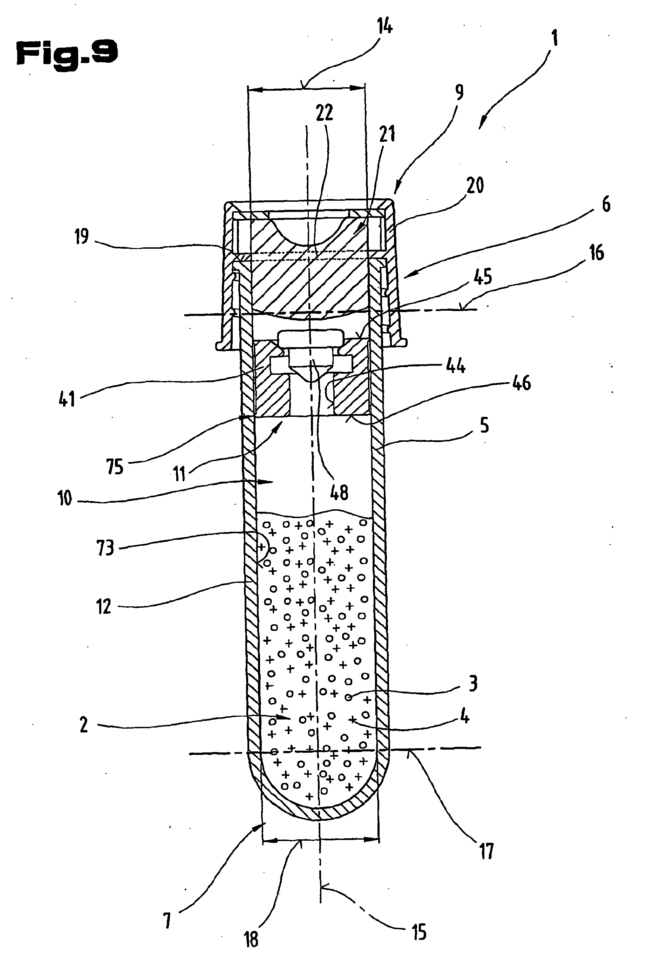

In den Fig. 9 bis 13 ist eine weitere mögliche und gegebenenfalls für sich eigenständige Ausbildung der Aufnahmeeinrichtung 1 mit der darin angeordneten Trennvorrichtung 11 gezeigt, welche jedoch nicht einen Teil der Erfindung darstellt und wobei wiederum für gleiche Teile die gleichen Bezugszeichen, wie in den vorangegangenen Fig. 1 bis 8, verwendet werden. Um unnötige Wiederholung zu vermeiden wird für die Ausbildung sowie die Wahl der Werkstoffe, insbesondere des Aufnahmebehälters 5, der Trennvorrichtung 11 sowie der Verschlußvorrichtung 9 auf die detaillierte Beschreibung der vorangegangenen Fig. 1 bis 8 hingewiesen bzw. bezug genommen.

Der Aufnahmebehälter 5 weist bei diesem Ausführungsbeispiel das erste und hier offen ausgebildete Ende 6, sowie das in Richtung der Längsachse 15 distanzierte und hier verschlossen ausgebildete, weitere Ende 7 auf. Die Behälterwand 12 umgrenzt den Innenraum 10 und bildet eine diesem zugewandte innere Oberfläche 73 aus. Weiters ist hier noch dargestellt, daß die innere Abmessung 14 im Bereich der ersten Ebene 16, welche dem ersten Ende 6 des Aufnahmebehälters 5 zugewandt ist, nahezu gleich bis identisch der inneren Abmessung 18 im Bereich der weiteren, im Bereich des Endes 7, schematisch dargestellten Ebene 17 ist. Durch die beiden gleichen inneren Abmessungen 14, 18 entspricht die innere Oberfläche 73 einer zylinderförmig ausgebildeten Wandfläche der Behälterwand 12 zwischen den beiden Ebenen 16, 17.

Der Grundkörper 41 der Trennvorrichtung 11 weist die beiden in Richtung der Längsachse 15 voneinander distanzierten Endbereiche 45, 46 auf, welche durch die Distanz 49 voneinander distanziert angeordnet sind. Am äußeren Umfang ist der Grundkörper 41 zwischen den beiden Endbereichen 45, 46 durch eine Außenfläche 74 begrenzt, wobei diese Außenfläche 74 bei dem zylinderförmig ausgebildeten Aufnahmebehälter 5 in etwa der inneren Abmessung 14 bzw. 18 entsprechen kann. Ist die Abmessung des Grundkörpers 41 derart gewählt, daß die Außenfläche 74 an der inneren Oberfläche 73 des Aufnahmebehälters 5 zur Anlage kommt, bildet die gesamte Außenfläche 74 eine Dichtungsvorrichtung 75 zwischen dieser und der inneren Oberfläche 73 im gegenseitigen Anlagebereich aus.

Dabei kann es auch möglich sein, eine äußere Abmessung bzw. einen Durchmesser der Außenfläche 74 größer der inneren Abmessung 14 bzw. 18 zu wählen, wodurch bei entsprechender Wahl des Werkstoffes für den Grundkörper 41 eine ausreichende Anpreßkraft der Außenfläche 74 an der inneren Oberfläche 73 zur Erzielung einer Abdichtung zwischen den beiden Grundkörper erreicht werden kann.

Unabhängig davon ist es auch möglich, die Dichtungsvorrichtung 75 durch zumindest eine umlaufende und die Außenfläche 74 des Grundkörpers 41 überragende Dichtlippe 76 zu bilden, wobei diese Dichtlippe 76 in einem der beiden Endbereiche 45, 46 angeordnet sein kann. Dabei kann eine äußere Abmessung der Außenfläche 74 geringer der inneren Abmessungen 14, 18 des Aufnahmebehälters 5 sein und nur die Dichtlippe 76 anliegend an der inneren Oberfläche 73 ausgebildet sein. Zur Erzielung einer besseren Abdichtung bzw. einer positionsgenaueren Führung des Grundkörpers 41 während der Verstellbewegung von der Ausgangsstellung hin in die Arbeitsstellung kann es vorteilhaft sein, wenn die Dichtungsvorrichtung 75 durch mehrere, bevorzugt jeweils in einem Endbereich 45, 46 des Grundkörpers 41, angeordnete und die Außenfläche 74 überragenden Dichtlippen 76 gebildet ist. Dies bedeutet, daß zumindest in jedem der Endbereiche 45, 46 eine rundum durchlaufende Dichtlippe 76 ausgebildet ist.

Selbstverständlich ist es aber auch möglich, in Richtung der Distanz 49 zwischen den beiden Endbereichen 45, 46 mehrere dieser Dichtlippen 76 anzuordnen und so eine Art Lamellendichtung auszubilden. Je nach Anzahl und Ausbildung der Dichtlippen 76 kann so jene Verstellkraft festgelegt werden, welche durch die Fliehkraft auf den Grundkörper 41 ausgeübt werden muß, um die zuvor beschriebenen Verstellbewegung durchführen zu können.

Innerhalb des Grundkörpers 41 ist im Bereich der Längsachse 15 die Verbindungsöffnung 44 zur Bildung des Strömungskanals zwischen den beiden Endbereichen 45, 46 angeordnet. Dabei weist die Verbindungsöffnung 44, ausgehend vom weiteren Endbereich 46 in Richtung des ersten Endbereiches 45 in einer senkrecht zur Längsachse 15 ausgerichteten Ebene, eine innere Abmessung 77 auf, welche zumindest gleich oder kleiner einer äußeren Abmessung 78 eines in die Verbindungsöffnung 44 einzusetzenden Tragkörpers 79 des Einsatzteils 48 ist.

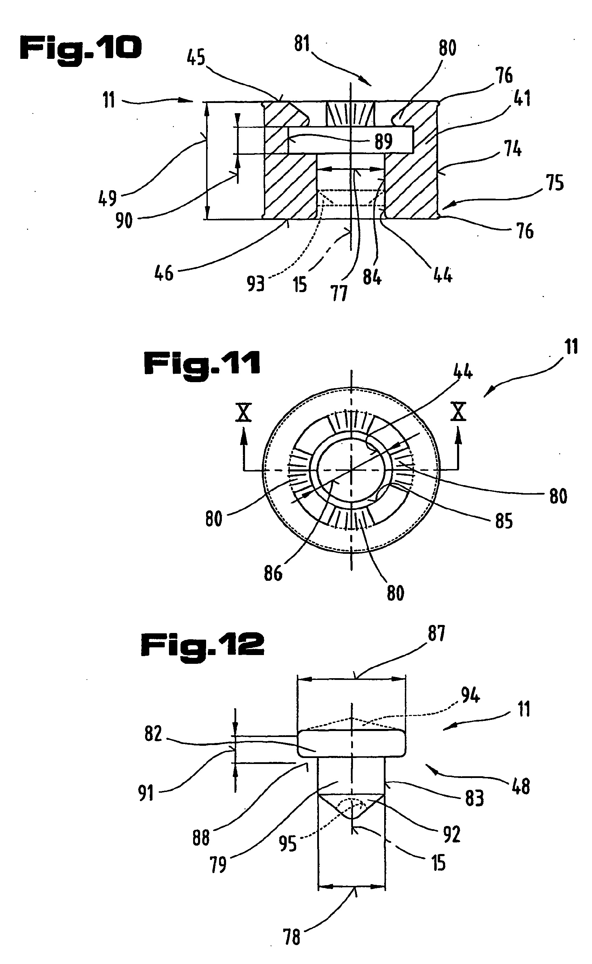

In den Fig. 10 bis 12 sind die Einzelteile zur Bildung der Trennvorrichtung 11 in einem größeren Maßstab dargestellt, wobei in der Ausgangsstellung der Trennvorrichtung 11 innerhalb des Aufnahmebehälters 5 der Einsatzteil 48, insbesondere der Tragkörper 79, bereichsweise in die Verbindungsöffnung 44 hineinragt, jedoch in der Ausgangsstellung der Einsatzteil 48 zwischen dem Grundkörper 41 und dem ersten bzw. offenen Ende 6 des Aufnahmebehälters 5 angeordnet ist. Dadurch ist eine Befüllung des Innenraums 10 durch den Dichtstopfen 22 der Dichtungsvorrichtung 21 mittels eines Durchstechvorganges, wie beispielsweise mit einer Hohlnadel bzw. einer Kanüle, möglich.

Um einen Durchtritt des in den Innenraum einzufüllenden Gemisches 2 auch im Bereich des Einsatzteiles 48 und des Grundkörpers 41 zu ermöglichen, sind am Grundkörper 41 im ersten Endbereich 45 mehrere bereichsweise über den Umfang der Verbindungsöffnung 44 verteilt angeordnete Rastelemente 80 einer Rückhaltevorrichtung 81 angeordnet, zwischen welchen bereichsweise ein Durchtritt des Gemisches 2 in die Verbindungsöffnung 44 und weiter in den Innenraum 10 möglich ist. Diese einzelnen Rastelemente 80 sind derart ausgebildet, daß bei einer Anlage eines den Tragkörper 79 des Einsatzteils 48 überragenden Ansatzes 82 an den Rastelementen 80 eine Anlagefläche 83 des Einsatzteils 48 distanziert von einer Dichtfläche 84 der Verbindungsöffnung 44 ist, wodurch die beiden Endbereiche 45, 46 über die Verbindungsöffnung 44 in Strömungsverbindung stehen. Dabei wirkt der Ansatz 82 mit den einzelnen Rastelementen 80 der Rückhaltevorrichtung 81 zusammen und positioniert den Einsatzteil 48 relativ zum Grundkörper 41 in der zuvor beschriebenen Stellung, wodurch ein Durchfluß zwischen dem teilweisen in die Verbindungsöffnung 44 ragenden Tragkörper 79 und der Dichtfläche 84 der Verbindungsöffnung 44 möglich ist.

Diese Stellung zur Befüllung des Innenraums 10 ist in der Fig. 9 dargestellt, wobei noch zusätzlich das bereits in den Innenraum 10 eingefüllte Gemisch 2 aus den beiden unterschiedlichen Medien 3, 4 ebenfalls vereinfacht dargestellt worden ist. Dabei ist die Rückhaltevorrichtung 81 am Grundkörper 41 an der dem ersten Ende 6 des Aufnahmebehälters 5 zugewandten Seite angeordnet. Die einzelnen Rastelemente 80 - hier im vorliegenden Ausführungsbeispiel vier Rastelemente 80 - bilden an dem der Längsachse 15 zugewandten Ende eine Hüllkurve 85 aus, welche in einer senkrecht zur Längsachse 15 ausgerichteten Ebene eine lichte Weite 86 aufweist, die kleiner einer äußeren Abmessung 87 des den Tragkörper 79 überragenden Ansatzes 82 des Einsatzteils 48 ist. Dadurch ist zumindest bereichsweise eine Anlage einer Bundfläche 88 des Ansatzes 82 an den hier nasenförmig ausgebildeten Rastelementen 80 gewährleistet.

Weiters ist im Bereich der Verbindungsöffnung 44 im Grundkörper 41 eine nutförmige Vertiefung 89 angeordnet, deren Breite 90 in Richtung der Längsachse 15 zumindest einer Dicke 91 des in diese Vertiefung 89 einzusetzenden Ansatzes des Einsatzteils 48 in der gleichen Richtung entspricht.

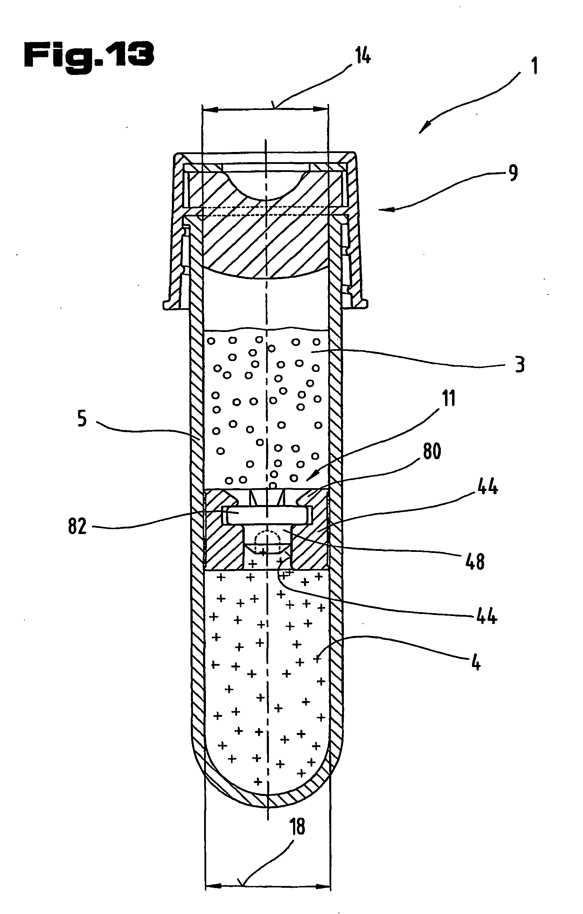

Diese dichtende Position des Einsatzteils 48 im Grundkörper 41 ist in der Fig. 13 zu ersehen, wobei hier der Ansatz 82 an den einzelnen Rastelementen 80 während des Zentrifugiervorganges vorbeibewegt worden ist und der Tragkörper 79 des Einsatzteils 48 in einer dichtenden, insbesondere flüssigkeitsdichten, Stellung, in der Verbindungsöffnung 44 angeordnet ist.

Dabei wirkt die Anlagefläche 83 des Tragkörpers 79 mit der Dichtfläche 84 der Verbindungsöffnung 44 zusammen, wodurch der Strömungskanal zwischen den beiden Endbereichen 45, 46 nach Beendigung des Zentrifugiervorganges verschlossen worden ist. Durch die zuvor beschriebenen Abmessungsunterschiede, insbesondere des Durchmessers zwischen der lichten Weite 86 der Hüllkurve 85 und der äußeren Abmessung 87 des Ansatzes 82 hintergreifen die Rastelemente 80 zumindest bereichsweise den Ansatz 82, wodurch eine zusätzliche Lagefixierung des Einsatzteils 48 in seiner dichtenden Stellung innerhalb der Verbindungsöffnung 44 gewährleistet ist. Dabei ist die nutförmige Vertiefung 89 auf der dem ersten Endbereich 45 bzw. dem ersten Ende 6 des Aufnahmebehälters 5 zugewandten Seite zumindest bereichsweise durch die Rastelemente 80 der Rückhaltevorrichtung 81 begrenzt. Dies hat den Vorteil, daß ausgehend von der Arbeitsstellung, in welcher eine Strömungsverbindung durch die Verbindungsöffnung 44 zwischen den beiden Endbereichen 45, 46 möglich ist, nur ein kurzer Verstellweg in Richtung der Längsachse 15 relativ zwischen den beiden Teilen, nämlich dem Einsatzteil 48 und dem Grundkörper 41, notwendig ist, um einerseits einen dichtenden Abschluß im Bereich der Trennvorrichtung 11 zwischen den beiden voneinander getrennten Medien 3, 4 zu erzielen und andererseits in der Ausgangsstellung bis hin zur Arbeitsstellung einen nahezu ungehinderten Durchfluß durch die Verbindungsöffnung 44 sicher zu stellen.

Zur Erleichterung dieser relativen Verstellbewegung ist es vorteilhaft, wenn die Rastelemente 80 auf der von der nutförmigen Vertiefung 89 abgewandten Seite, ausgehend vom Bereich der Längsachse 15 in Richtung der Außenfläche 74, keilförmig bzw. kegelig erweiternd ausgebildet sind. Durch diese Rastelemente bildet sich im Endbereich 45, ausgehend von diesem hin in Richtung des weiteren Endbereiches 46, eine sich trichterförmig verjüngende Aufnahmeöffnung hin zur Verbindungsöffnung 44 aus, wodurch, je nach Steilheit des Winkels, die aufzubringende, notwendige Durchtrittskraft zwischen dem Einsatzteil 48 und dem Grundkörper 41 festlegbar ist und damit auch der Zeitpunkt im Zuge des Zentrifugiervorganges festgelegt werden kann, bei welchem die relative Verlagerung des Einsatzteiles 48 hin zur dichtenden Stellung innerhalb des Grundkörpers 41 erfolgt.

Weiters weist der Einsatzteil 48 an der vom Ansatz 82 abgewandten Seite einen an den Tragkörper 79 anschließenden, sich kegelförmig verjüngenden Ansatzteil 92 auf, der dazu vorgesehen sein kann, um die relative Verstellbewegung zwischen dem Einsatzteil 48 und dem Grundkörper 41 hin zur dichtenden Stellung zu erleichtern. Gleichfalls kann aber auch dadurch der Einsetzvorgang für die Stellung des Einsatzteiles 48 in seiner Ausgangsstellung vor Beginn des Zentrifugiervorganges in den Grundkörper 41 erleichtert werden, da so die jeweils schräg zur Längsachse 15 ausgerichteten Flächen des Ansatzteiles 92 bzw. der Rastelemente 80 dies erleichtern.

Um eine zusätzliche Abdichtung zwischen dem Einsatzteil 48 und dem Grundkörper 48 im Bereich der Verbindungsöffnung 44 zu erzielen, ist weiters, wie in der Fig. 10 im Bereich der Verbindungsöffnung 44, in strichlierten Linien dargestellt, ein gegengleich zum kegelförmig verjüngenden Ansatzteil 92 des Einsatzteils 48 ausgebildeter und mit diesem in der Arbeitsstellung zusammenwirkender Anschlagring 93 angeordnet. Durch die gegengleiche Ausführung der zur Längsachse 15 geneigten Flächen des kegelförmig ausgebildeten Einsatzteils 92 bzw. Anschlagrings 93, ist auch in diesem Bereich eine Abdichtung des Durchströmkanals 44 zwischen den beiden Endbereichen 45, 46 möglich. Dabei ist es möglich, diese Abdichtung zusätzlich zu der Abdichtung zwischen der Anlagefläche 83 sowie Dichtfläche 84 auszubilden oder die Abdichtung ausschließlich zwischen dem Ansatzteil 92 und Anschlagring 93 zu wählen. Dabei liegt in der Arbeitsstellung der Trennvorrichtung 11 der kegelförmig ausgebildete sich verjüngende Ansatzteil 92 des Einsatzteils 48 dichten, insbesondere flüssigkeitsdicht, am Anschlagring 93 an.

Zusätzlich ist es noch möglich, um den Einfüllvorgang gesichert durchführen zu können, daß auf der vom Tragkörper 79 abgewandten Seite des Ansatzes 82 ein in der Fig. 12 in strichlierten Linien dargestellter erhöhter Grundkörper 94 an den Ansatz 82 angeformt ist, der ausgehend von der Längsachse 15 in Richtung der Randbereiche des Ansatzes 82 abfallend ausgebildet ist, wodurch bei einem Einfüllvorgang des Gemische 2 dieses entlang der zur Längsachse 15 geneigten Flächen ablaufen kann und so in weiterer Folge durch die Verbindungsöffnung 44 in den Innenraum 10 weiterfließen kann. Dieser Einfüllvorgang wird auch noch durch das im Innenraum herrschende Vakuum, bzw. den im Innenraum herrschenden Unterdruck erleichtert, wodurch dies in Form eines Ansaugvorganges erfolgt und durch die Höhe des gewählten Unterdruckes auch der Füllstand innerhalb des Aufnahmebehälters, bzw. die Einfüllmenge, einfach festlegbar ist.

Nach dem erfolgten Einfüllvorgang befindet sich die gesamte Trennvorrichtung 11 in ihrer zuvor beschriebenen Ausgangsstellung, also nahe dem offenen Ende des Aufnahmebehälters 5 bzw. nahe der Verschlußvorrichtung 9. Das Gemisch 2 aus den beiden voneinander zu trennenden Medien 3, 4 ist zwischen der Trennvorrichtung 11 und dem hier verschlossenen Ende 7 im Aufnahmebehälter 5 bevorratet. Bei Beginn des Zentrifugiervorganges erfolgt aufgrund der Wahl der Dichte des Grundkörpers 41 bzw. des Einsatzteils 48 ein relativer Verstellvorgang des Grundkörpers 41 entlang der inneren Oberfläche 73 des Aufnahmebehälters 5 in Richtung des Endes 7. Dabei beträgt eine Dichte des Grundkörpers 41 zwischen 1,04 g/cm³ und 1,05 g/cm³ und des Einsatzteils 48 zwischen 1,06 g/cm³ und 1,07 g/cm³. Nach Überwindung eines gewissen Verstellweges erreicht der Endbereich 46 des Grundkörpers 41 die Oberseite des Gemisches 2, wobei aufgrund der einwirkenden Fliehkräfte bereits eine Trennung der Medien 3, 4 aufgrund deren unterschiedlichen Dichtewerte erfolgt ist. Dabei weist das Gemisch, wie beispielsweise das Vollblut, eine Dichte zwischen 1,05 g/cm³ und 1,06 g/cm³ auf. Die Dichte des Serums bzw. Plasmas beträgt zwischen 1,02 g/cm³ und 1,03 g/cm³ und die der Blutzellen in etwa 1,08 g/cm³.

Das hier mit dem Bezugszeichen 3 versehene leichtere Medium 3 verbleibt an der dem Ende 6 zugewandten Seite des Aufnahmebehälters 5, wobei das hier mit Kreuzen dargestellte schwerere Medium 4 in Richtung des hier verschlossen ausgebildeten Endes 7 verbracht wird. Der Grundkörper 41 wird durch die auf ihn einwirkende Fliehkraft und der zuvor beschriebenen Möglichkeit des Durchtritts des leichteren Mediums 3 durch die Verbindungsöffnung 44 auf die der Verschlußvorrichtung 9 zugewandten Seite des Grundkörpers 41 in Richtung des weiteren Endes 7 des Aufnahmebehälters 5 bewegt. Dies erfolgt solange, bis daß der Endbereich 46 des Grundkörpers 41 an der Trennfläche zwischen dem schwereren und dem leichteren Medium 4, 3, aufgrund der zuvor beschriebenen Dichteunterschiede, zur Anlage kommt und die relative Verstellbewegung zwischen dem Grundkörper 41 und dem Aufnahmebehälter 5 beendet ist. Nun erfolgt eine weitere relative Verlagerung des Einsatzteils 48 gegenüber dem Grundkörper 41 in die dichtende Lage innerhalb des Grundkörpers 41, wodurch ein Durchtritt bzw. eine nachträgliche Vermischung der beiden voneinander getrennten Medien 3, 4 gesichert verhindert ist. Zur Erleichterung dieser Verstellbewegung kann, ohne ein weiteres Verdrängen von einem der beiden Medien 3, 4 bewirken zu müssen, innerhalb des Einsatzteils 48 im Bereich des Ansatzteiles 92 bzw. Tragkörpers 79 eine, in der Fig. 12 in strichlierten Linien dargestellte, Ausnehmung 95 angeordnet sein, die zur Aufnahme des Mediums 3 und/oder 4 dient und so der Ansatz 82 über die Rastelemente 80 in die im Bereich der Verbindungsöffnung 44 angeordnete nutförmige Vertiefung 89 hin verstellt werden kann.

Damit ist eine dichtende Lage sowohl im Bereich zwischen der inneren Oberfläche 73 des Aufnahmebehälters 5 und der Außenfläche 7, bzw. den Dichtlippen 7, des Grundkörpers 41, sowie zwischen dem Grundkörper 41 und dem Einsatzteil 48, erzielt.

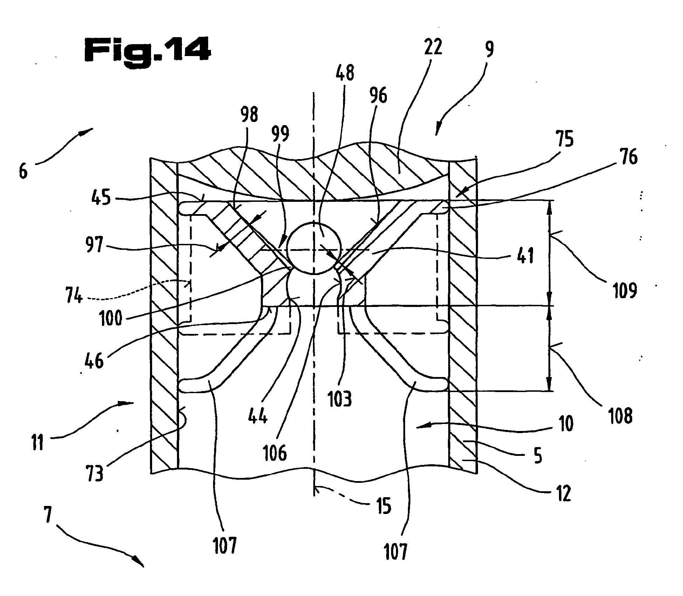

In den Fig. 14 bis 16 ist eine weitere und gegebenenfalls für sich eigenständige Ausbildung der Trennvorrichtung 11 innerhalb des Aufnahmebehälters 5 der Aufnahmeeinrichtung 1 dargestellt, welche jedoch nicht einen Teil der Erfindung darstellt und wobei wiederum für gleiche Teile gleiche Bezugszeichen, wie in den vorangegangenen Fig. 1 bis 13, verwendet werden. Um unnötige Wiederholung, insbesondere für die Ausbildung des Aufnahmebehälters 5, dessen Verschlußvorrichtung 9, sowie einzelne Ausbildungen der Trennvorrichtung 11, zu vermeiden, wird auf die vorangegangenen Fig. 1 bis 13 hingewiesen bzw. bezug genommen.

Der Aufnahmebehälter 5 mit der den Innenraum 10 umgrenzenden Behälterwand 12, ist im Bereich seiner inneren Oberfläche 73 zwischen den beiden voneinander distanzierten Enden 6, 7 wiederum zylindrisch ausgebildet, wie dies bereits in den Fig. 9 bis 13 beschrieben worden ist. Somit weist der Innenraum 10 zwischen den beiden Enden 6, 7 die gleichen Abmessungen 14, 18 auf (siehe Fig. 9).

Zwischen dem Grundkörper 41 und der inneren Oberfläche 73 des Aufnahmebehälters 5 ist wiederum die Dichtungsvorrichtung 75, bevorzugt rundum durchlaufend, am Grundkörper 41 angeordnet, wodurch in diesem Bereich, zwischen dem Grundkörper 41 und der Behälterwand 12 ein Durchtritt von den Medien 3, 4 , welche das Gemisch 2(siehe Fig. 9) ausbilden, gesichert verhindert ist. Die Dichtungsvorrichtung kann wiederum durch zumindest eine umlaufende und dem Grundkörper 41 überragende Dichtlippe 76 gebildet sein, welche aus einem zum Grundkörper 41 gleichen oder unterschiedlichen Werkstoff gebildet sein kann. Dabei kann die Dichtlippe 76, wenn diese aus einem zum Grundkörper 41 unterschiedlichen Material gebildet ist, am Grundkörper gehaltert, an diesem angeformt bzw. einstückig mit diesem verbunden sein. Dies kann frei nach dem bekannten Stand der Technik gewählt werden.

Gleichfalls ist es aber auch möglich, den Grundkörper 41, sowie die Dichtlippe 76 aus dem gleichen Werkstoff, jedoch mit unterschiedlichen Dichten bzw. Elastizitätswerten herzustellen, wodurch die Dichtlippe 76 zur einwandfreien Realisierung einer sicheren Trennung nach erfolgtem Trennvorgang zwischen den beiden Medien 3, 4 ausgebildet ist.

Der hier dargestellte Grundkörper 41, insbesondere im linken Teil der Fig. 14, kann in dem der inneren Oberfläche 73 zugewandten Bereich gleichartig zu der in den Fig. 10 dargestellten Raumform gewählt sein, wie dies in strichlierten Linien vereinfacht dargestellt worden ist. Weiters weist der Grundkörper 41 der Trennvorrichtung 11 bei diesem Ausführungsbeispiel, ausgehend vom ersten Endbereich 45 hin zu seinem weiteren Endbereich 46, ausgehend von den Randbereichen, eine sich kegelförmig in Richtung der Längsachse 15 verjüngende Ausnehmung 96 auf, welche im Bereich der Längsachse 15 in die Verbindungsöffnung 44 mündet. Besonders vorteilhaft ist es, wenn der Grundkörper 41, ausgehend vom ersten Endbereich 45 hin zu seinem weiteren Endbereich 46, insbesondere in Richtung der Längsachse 15 trichterförmig ausgebildet ist, wodurch ein hoher Grad an Materialeinsparung zur Ausbildung des Grundkörpers 41 erzielt werden kann. Durch die trichterförmige Ausbildung weist der Grundkörper 41 eine in etwa gleiche Wandstärke 97 auf und kann beispielsweise durch einen einfachen Spritzgußvorgang hergestellt werden.

Die Ausnehmung 96 ist, wie bereits zuvor beschrieben, kegelförmig verjüngend ausgebildet und durch eine Begrenzungsfläche 98 in ihrer Raumform festgelegt. Der Einsatzteil 48 ist in der Ausgangsstellung der Trennvorrichtung 11 wiederum zwischen dem Grundkörper 41, insbesondere der Ausnehmung 96 und dem Dichtstopfen 22 der Verschlußvorrichtung 9 angeordnet. Dabei ist bei diesem Ausführungsbeispiel der Einsatzteil 48 durch eine Kugel gebildet, welche in der in der Fig. 14 dargestellten Ausgangsstellung durch eine Rückhaltevorrichtung 99 derart relativ zum Grundkörper 41 positioniert wird, so daß stets ein Durchfluß für das in den Innenraum 10 einzufüllende Gemisch 2 vom bedarfsweise verschließbaren ersten Ende 6 in den Innenraum 10 durch die Trennvorrichtung 11 hindurch möglich ist.

Diese Rückhaltevorrichtung 99 ist in einem Übergangsbereich zwischen der Verbindungsöffnung 44 und der sich kegelförmig verjüngenden Ausnehmung 96 am Grundkörper 41 angeordnet. Ausgehend von der Verbindungsöffnung 44 bzw. der Längsmittelachse 15 ist die Ausnehmung 96 in Richtung der Außenfläche 74 bzw. dem Endbereich 45 kegelförmig erweiternd ausgebildet. Die Rückhaltevorrichtung 99 ist durch mehrere, über den Umfang der Verbindungsöffnung 44 verteilt angeordnete und in diese von der Außenfläche 74 in Richtung der Längsachse 15, vorragende Rastelemente 100 bzw. Rippen gebildet. Diese Rastelemente 100 begrenzen in ihren der Längsachse 15 zugewandten Endbereichen in einer senkrecht zur Längsachse 15 ausgerichteten Ebene eine Hüllkurve mit einer lichten Weite 101, welche kleiner einer äußeren Abmessung 102, insbesondere einem Durchmesser des kugelförmigen Einsatzteils 48, ist.

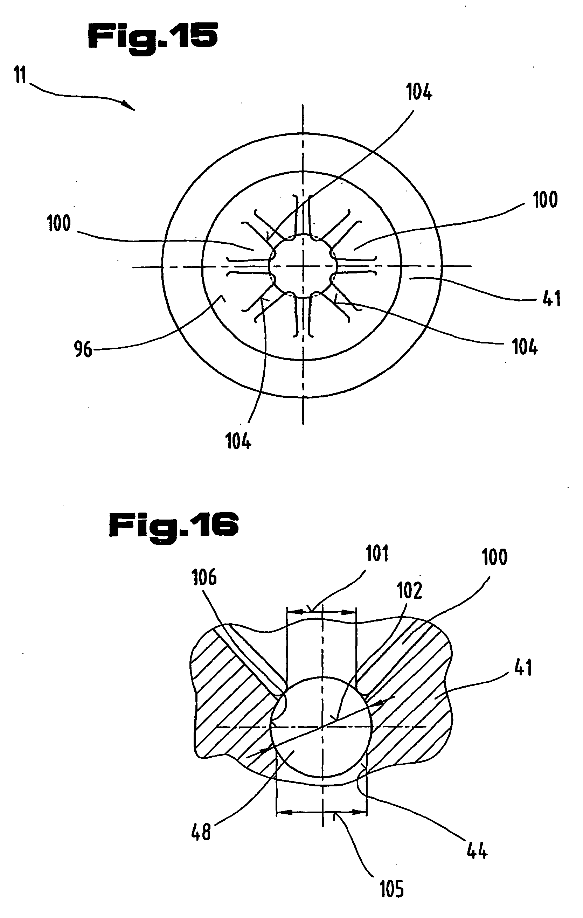

Weiters ist noch aus der Darstellung der Fig. 14 bzw. 15 zu ersehen, daß die einzelnen Rastelemente 100 im Übergangsbereich zwischen der Begrenzungsfläche 98 der kegelförmig ausgebildeten Ausnehmung 96 und der Verbindungsöffnung 44 über diese Begrenzungsfläche 98 vorragend ausgebildet sind. Durch die mehrfache Anordnung der Rastelemente 100 über den Umfang der Verbindungsöffnung 44 und dem weiters, ausgehend von der Verbindungsöffnung 44 hin in Richtung des ersten Endbereiches 45 des Grundkörpers 41 gegenüber der Begrenzungsfläche 98, abnehmenden Überstandes 103, bilden sich zwischen den einzelnen Rastelementen 100 Durchströmkanäle 104 aus.

Dadurch ist ein gesicherter Durchfluß des in den Innenraum einzufüllenden Gemisches 2 durch diese Durchströmkanäle 104 auch bei einer Anlage des Einsatzteils an den Rastelementen 100 möglich. Dies erfolgt solange, bis daß der Einsatzteil 48 von seiner Ausgangsstellung hin in die Arbeitsstellung und die in der Fig. 16 dichtend dargestellte Arbeitsstellung, verstellt worden ist. In der Ausgangsstellung ist der Einsatzteil 48 an der von der Verbindungsöffnung 44 abgewandten Seite der Rastelemente 100 an diesen abgestützt. Die äußere Abmessung 102, insbesondere der Durchmesser des kugelförmigen Einsatzteils 48, ist bei diesem Ausführungsbeispiel größer einem Durchmesser 105 der Verbindungsöffnung 44 gewählt

Wie nun am besten aus der Fig. 14 zu ersehen ist, ist zur Aufnahme und Abstützung, insbesondere der dichtenden Lage des Einsatzteils 48 im Grundkörper 41 im Übergangsbereich zwischen der Verbindungsöffnung 44 und der Rückhaltevorrichtung 99 bzw. der Begrenzungsfläche 98 der sich kegelförmig verjüngenden Ausnehmung 96, eine gegengleich zum kugelförmigen Einsatzteil 48, ausgebildete Anlagefläche 106 angeordnet. Diese Anlagefläche 106 dient dazu, um im Zusammenwirken mit dem kugelförmigen Einsatzteil 48, sowie den Rastelementen 100 der Rückhaltevorrichtung 99 einen dichtenden Abschluß zwischen den voneinander getrennten Medien 3, 4 nach Beendigung des Zentrifugiervorganges zu erzielen. Dabei dient die Anlagefläche 106 als Dichtfläche zwischen der äußeren Oberfläche des Einsatzteils 48 und dem Grundkörper 41.

Durch die zuvor beschriebenen Abmessungsunterschiede, insbesondere der lichten Weite 101 des Hüllkreises bzw. der Hüllkurve der Rastelemente 100 und der äußeren Abmessung 102 des kugelförmigen Einsatzteils 48, kommt es nach dem Hindurchtritt des Einsatzteils 48 durch die Rückhaltevorrichtung 99 zu einer Lagefixierung des Einsatzteils 48 relativ zum Grundkörper 41, wobei zusätzlich noch durch die einzelnen Rastelemente 100 eine gerichtete Anlagekraft des Einsatzteils 48 an die Anlagefläche 106 aufgebracht werden kann.

Weiters ist noch dargestellt, daß am Grundkörper 41 im weiteren Endbereich 46 unmittelbar benachbart zur Verbindungsöffnung 44 mehrere, bevorzugt drei, Führungselemente 107 angeordnet sind, welche sich auf die von der Längsachse 15 abgewandte Seite erstrecken und eine in der senkrecht zur Längsachse 15 ausgerichteten Ebene und durch Enden der Führungselemente 107 verlaufende äußere Hüllkurve in etwa einer äußeren Abmessung des Grundkörpers 41 in seinem ersten Endbereich 45 entspricht. Diese Ausbildung und Anordnung der Führungselemente 107 dient dazu, den beispielsweise nur trichterförmig ausgebildeten Grundkörper 41 in seiner Lage relativ zum Aufnahmebehälter 5 festzulegen, um beispielsweise ein Kippen während der Verstellbewegung, und damit eine Undichtheit im Bereich der Dichtlippen 76, zu verhindern. Diese Führungselemente 107 können jede beliebige Raumform aufweisen, wobei ein bogenförmig gekrümmter Verlauf vorteilhaft ist, da so eine gewisse Feder- bzw. Vorspannwirkung, ausgehend von diesen Führungselementen 107, auf die innere Oberfläche 73 des Aufnahmebehälters 5 aufgebaut werden kann.

Selbstverständlich ist es aber auch möglich, im ungespannten Zustand die Abmessung der äußeren Hüllkurve größer der äußeren Abmessung des Grundkörpers 41 bzw. der inneren Abmessung 14, 18 des Innenraums 10 zu wählen. Zur Erzielung einer ausreichenden Kippsicherheit des Grundkörpers 41 ist es vorteilhaft, wenn die Führungselemente 107 den Grundkörper 41 um einen Überstand 108 in Richtung der Längsachse 15 auf die vom ersten Endbereich 45 abgewandte Seite überragen, der in etwa einer Höhe 109 des Grundkörpers 41 in der gleichen Richtung entspricht.

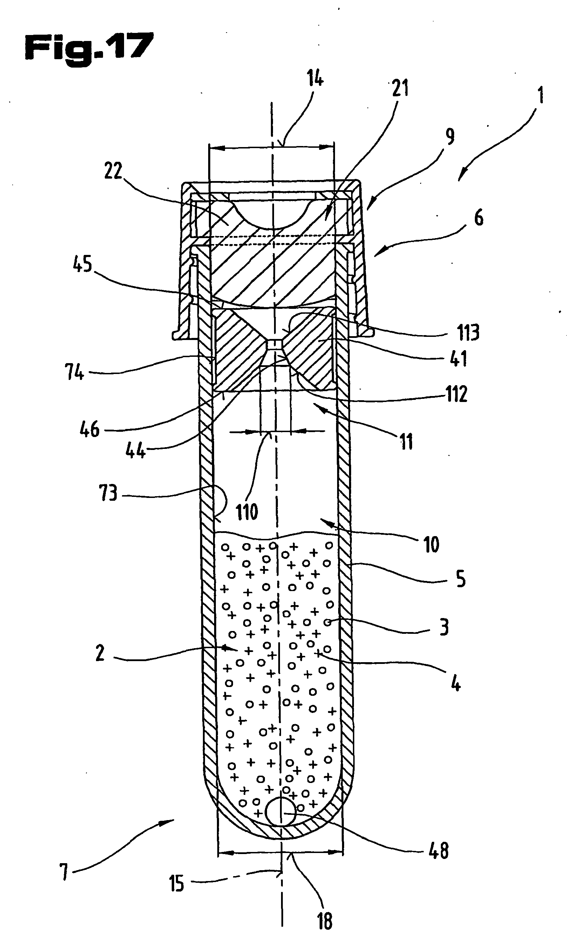

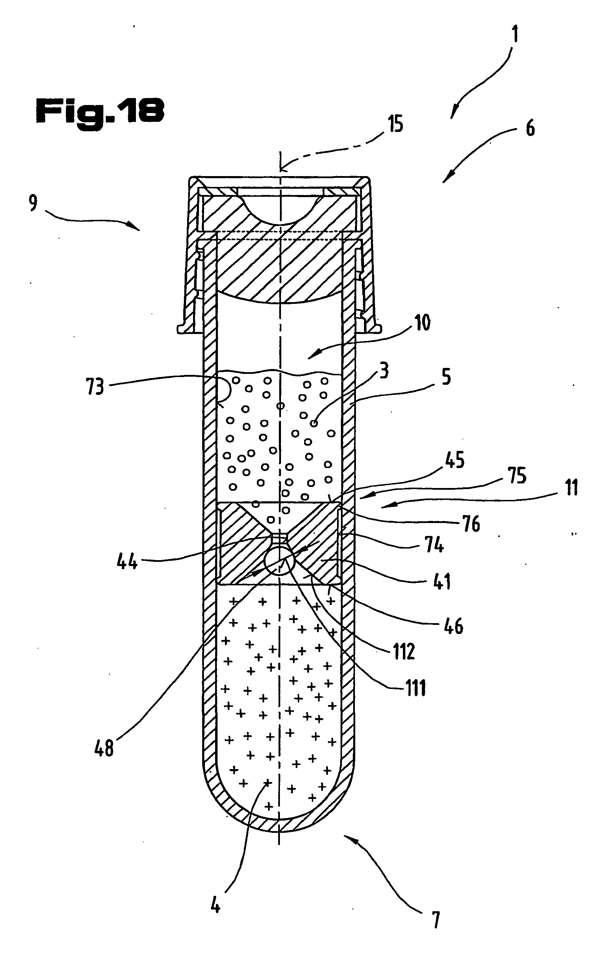

In den Fig. 17 und 18 ist eine weitere und gegebenenfalls eine für sich eigenständige Ausbildung der Trennvorrichtung 11 innerhalb des Aufnahmebehälters 5 dargestellt, welche jedoch nicht einen Teil der Erfindung darstellt und wobei wiederum für gleiche Teile gleiche Bezugszeichen, wie in den vorangegangenen Fig. 1 bis 16 verwendet werden. Die Ausbildung des Aufnahmebehälters 5, dessen Verschlußvorrichtung 9 mit der Dichtungsvorrichtung 21, insbesondere dem Dichtstopfen 22, kann gemäß einer der zuvor in den Fig. 1 bis 16 beschriebenen Ausbildungen gebildet sein. Deshalb wird, um unnötige Wiederholungen zu vermeiden, auf diese vorangegangenen Fig. hingewiesen bzw. bezug genommen.

Der Aufnahmebehälter 5 umgrenzt den Innenraum 10 und weist in Richtung seiner Längsachse 15 die voneinander distanzierten Enden 6, 7 auf, von denen zumindest eines mit einer Öffnung ausgebildet ist. In den Innenraum 10 ist wiederum die Trennvorrichtung 11 eingesetzt, die den Grundkörper 41 mit dem darin angeordneten, bedarfsweise verschließbaren, Strömungskanal umfaßt. Der Grundkörper 41 weist in Richtung der Längsachse 15 die voneinander distanzierte Endbereiche 45, 46 auf, zwischen welchen sich die den Grundkörper 41 begrenzende Außenfläche 74 erstreckt. Zwischen dem Grundkörper 41 und der inneren Oberfläche 73 des Aufnahmebehälters 5 ist zumindest eine Dichtungsvorrichtung 75 mit zumindest einer umlaufend am Grundkörper 41 angeordneten Dichtlippe 76 vorgesehen. Die Ausbildung der Dichtungsvorrichtung 75 mit zumindest einer, bevorzugt jedoch mehreren Dichtlippen 76, kann gemäß der Beschreibung in den Fig. 9 bis 13 erfolgen.

In der Fig. 17 ist die Aufnahmeeinrichtung 1 mit der darin angeordneten Trennvorrichtung 11 sowie dem in den Innenraum 10 eingebrachten Gemisch 2 aus den Medien 3, 4 in der Ausgangsstellung gezeigt, wobei sich wiederum der Grundkörper 41 naher der Verschlußvorrichtung 9 befindet. Während der Beaufschlagung mit der Zentrifugalkraft ist der Grundkörper 41 in Richtung des weiteren Endes 7 in seine mit dem Einsatzteil 48 und in der Fig. 18 dargestellte Arbeitsstellung verlagerbar.

Die Trennvorrichtung 11 umfaßt bei diesem Ausführungsbeispiel zusätzlich zum Grundkörper 41 zumindest einen Einsatzteil 48, der in der Ausgangsstellung der Trennvorrichtung 11 zwischen dem Grundkörper 41 und dem weiteren hier verschlossen dargestellten Ende 7 im Innenraum 10 des Aufnahmebehälters 5 angeordnet ist. Der Strömungskanal ist durch die im Grundkörper 41 angeordnete, bevorzugt als Bohrung, rund ausgeführte Verbindungsöffnung 44 ausgebildet, welche in Richtung der Längsachse 15 vom weiteren Endbereich 46 hin zum ersten Endbereich 45 eine zumindest bereichsweise abnehmende innere Öffnungsweite 110 aufweist, wobei in einer senkrecht zur Längsachse 15 ausgerichteten Ebene eine äußere Abmessung 111 des Einsatzteils 48 größer der kleinsten inneren Öffnungsweite 110 der Verbindungsöffnung 44 ist, um eine sichere Abdichtung durch den Einsatzteil 48 im Bereich der Verbindungsöffnung 44 zu erzielen.

Durch die zuvor beschriebene abnehmende innere Öffnungsweite 110 ist in diesem Abschnitt die Verbindungsöffnung 44 kegelförmig verjüngend ausgebildet, wobei es vorteilhaft ist, die größte innere Öffnungsweite 110 größer der äußeren Abmessung 111 des Einsatzteils 48 zu wählen. Dadurch ist ein Eintreten des Einsatzteils 48 in die Verbindungsöffnung 44 bei oder kurz nach Beendigung des Zentrifugiervorganges möglich, wobei durch das Ausmaß der Verringerung der inneren Öffnungsweite 110 der Kegelwinkel der Verbindungsöffnung 44 festgelegt werden kann. Je nach der Steilheit des Winkels kann eine selbsthemmende Klemmung des Einsatzteils 48 in diesem Abschnitt der Verbindungsöffnung 44 sichergestellt werden.

Wesentlich bei dieser Ausführung ist, daß der Grundkörper 41 eine Dichte zwischen 1,06 g/cm³ und 1,07 g/cm³ und der Einsatzteil 48 eine Dichte zwischen 1,04 g/cm³ und 1,05 g/cm³ aufweist. Dadurch ist gewährleistet, daß in Folge der fortschreitenden Trennung der beiden Medien 3, 4 des Gemisches 2 und der zuvor beschriebenen Dichtewerte für die beiden Medien 3, 4 ein Absinken des Grundkörpers 41 durch das leichtere Medium 3 hin bis zum Erreichen der Trennfläche zwischen den beiden Medien 3, 4 möglich ist. Durch die Wahl der Dichte des Einsatzteils wird dieser im ungetrennten Gemisch 2 aufschwimmend bzw. in einem Schwebezustand innerhalb des Gemisches 2 gehalten, wobei bei beginnender Trennung der beiden Medien 3,4 der leichtere Bestandteil oberhalb der schwereren Blutzellen aufschwimmt und der Einsatzteil 48 durch die geringere Dichte gegenüber dem Serum bzw. Plasma bis in den Bereich der Trennfläche zwischen den beiden Medien 3, 4 aufsteigt.

Bei Beaufschlagung mit der Zentrifugalkraft wird der Grundkörper 41 aus seiner Ausgangsstellung in Richtung der Längsachse 15 hin zum hier verschlossenen Ende 7 bewegt, wobei ein Durchtritt des leichteren Mediums, hier im vorliegenden Fall des Mediums mit dem Bezugszeichen 3, durch die noch nicht verschlossene Verbindungsöffnung 44 in den zwischen dem Grundkörper 41 und der Verschlußvorrichtung 9 angeordneten Innenraum 10 der Aufnahmeinrichtung 1 möglich ist, wobei eine Abdichtung der zuvor beschriebenen Verbindungsöffnung 44 beim Auftreffen des Grundkörpers 41 im Bereich der Trennfläche zwischen den beiden Medien 3, 4 durch den Einsatzteil 48 erfolgt. Durch die höhere Dichte sinkt der Grundkörper 41 tiefer in das Medium 4 ein, wodurch der Einsatzteil 48 gesichert in einer dichtenden, insbesondere flüssigkeitsdichten, Stellung in der Verbindungsöffnung 44 nach Beendigung des Zentrifugiervorganges angeordnet und in Art eines Klemmsitzes gehalten ist.

Dabei ist es vorteilhaft, wenn der Einsatzteil 48 durch eine Kugel gebildet ist. Um ein gesichertes Eintreten des Einsatzteils 48 nach Beendigung bzw. kurz vor Abschluß des Zentrifugiervorganges zu erreichen ist es vorteilhaft, wenn der Grundkörper 41 der Trennvorrichtung 11, ausgehend vom weiteren Endbereich 46 hin zum ersten Endbereich 45, eine sich kegelförmig verjüngende Ausnehmung 112 aufweist, welche im Bereich der Längsmittelachse 15 in die Verbindungsöffnung 44 mündet. Diese Ausnehmung 112 erstreckt sich über eine Teillänge des Grundkörpers 41 in Richtung der Längsachse 15.

Für den Einfüllvorgang ist es vorteilhaft, wenn der Grundkörper 41 der Trennvorrichtung 11, ausgehend vom ersten Endbereich 45 hin zum weiteren Endbereich 46 eine weitere, sich kegelförmig verjüngende Ausnehmung 113 aufweist, welche ebenfalls im Bereich der Längsachse 15 in die Verbindungsöffnung 44 mündet und ebenfalls über eine Teillänge des Grundkörpers 41 in Richtung der Längsachse 15 erstreckt.

Bei den in den Fig. 9 bis 18 gezeigten Ausführungsformen weist der Aufnahmebehälter 5 im Bereich seiner inneren Oberfläche 73 stets annähernd die gleichen inneren Abmessungen 14, 18 auf, wodurch eine ziemlich exakte Zylinderwand den Innenraum 10 umgrenzt.

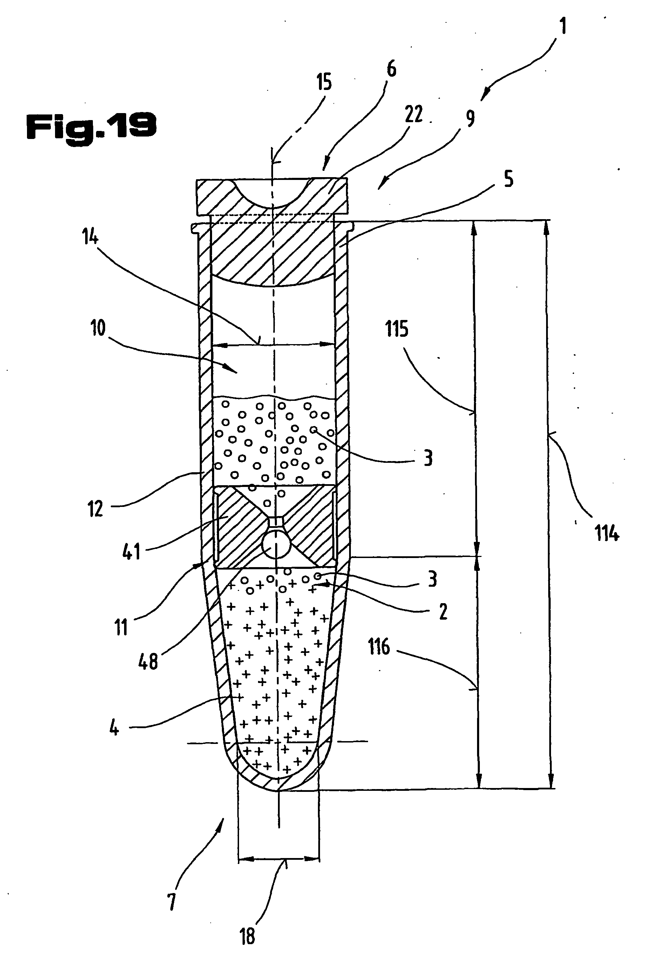

In der Fig. 19 ist eine weitere Möglichkeit zur Ausbildung eines Aufnahmebehälters 5 gezeigt, wobei wiederum für gleiche Teile gleiche Bezugszeichen wie in den vorhergehenden Fig. 1 bis 18 verwendet werden. Gleichfalls wurde nur ein Teil der Verschlußvorrichtung 9, nämlich nur der Dichtstopfen 22, dargestellt.

Der Aufnahmebehälter 5 weist in Richtung seiner Längsachse 15 eine Gesamtlänge 114 auf, wobei dieser über eine erste Teillänge 115, ausgehend vom hier offen dargestellten Ende 6 hin zum hier verschlossen dargestellten Ende 7, in einer senkrecht zur Längsachse 15 ausgerichteten Ebene die gleiche innere Abmessung 14 aufweist und somit in diesem Abschnitt zylindrisch ausgebildet ist. Eine weitere Teillänge 116 des Aufnahmebehälters 5 weist im Anschluß an die erste Teillänge 115, ausgehend von der inneren Abmessung 14, eine abnehmende innere Abmessung 18 auf, welche im Bereich des weiteren Endes 7 kleiner der ersten Abmessung 14 ist.

Durch die Wahl der einzelnen Teillängen 115, 116, im Verhältnis zur Gesamtlänge 114, kann so auf einfache Art und Weise eine vorbestimmbare Position der Trennvorrichtung 11 innerhalb des Aufnahmebehälters 5 nach Beendigung des Zentrifugiervorganges festgelegt werden, in welcher die Trennvorrichtung 11 relativ in ihrer Position zum Aufnahmebehälter 5 festgelegt ist. Dabei hängt die Wahl der Teillängen 115, 116 vom Gesamt-Füllvolumen des Gemisches 2 im Innenraum 10 ab, wobei dadurch auf alle Fälle eine gesicherte Trennung zwischen den beiden voneinander separierten Medien 3, 4 innerhalb des Innenraums 10 gewährleistet ist, ohne daß eine nachträgliche Vermischung nach Beendigung des Zentrifugiervorganges noch erfolgen kann. Dabei ist es auch unwesentlich, ob noch eine Teilmenge des hier leichter dargestellten Mediums 3 im Innenraum 10 zwischen der Trennvorrichtung 11 und dem verschlossenen Ende 7 des Aufnahmebehälters 5 vorhanden ist. Dies ist schematisch durch einige Teile des Mediums 3 in diesem Abschnitt des Aufnahmebehälters 5 dargestellt. Die Ausbildung der Trennvorrichtung 11 kann bei Verwendung dieses Aufnahmebehälters 5 gemäß der Beschreibung einer der Fig. 1 bis 18 entsprechen.

Die jeweils in den einzelnen Fig. dargestellten Mengen des Gemisches 2, bzw. dessen Bestandteilen - Medien 3, 4 - sind nur beispielhaft wiedergegeben und müssen weder in der Gesamtmenge noch in den einzelnen Teilmengen den tatsächlichen Mengen entsprechen.

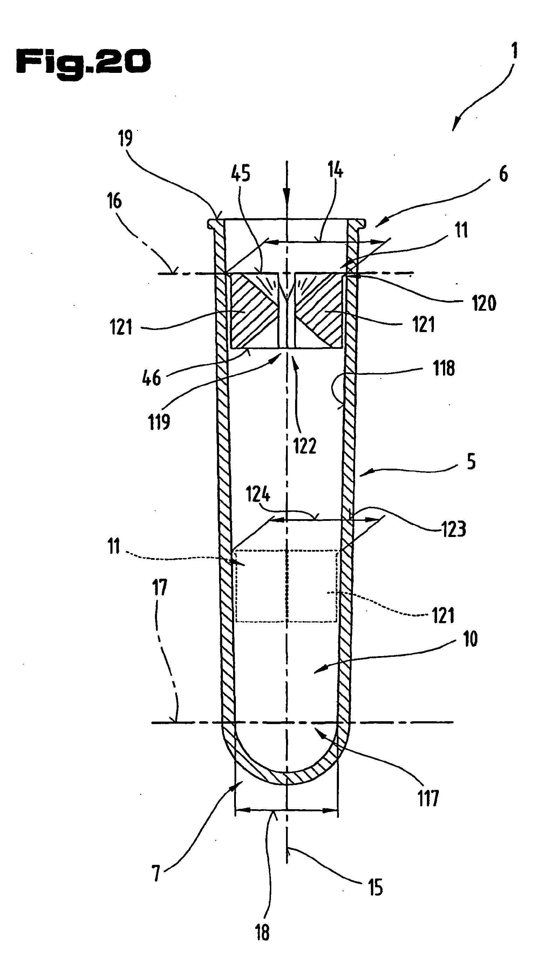

In den Fig. 20 bis 22 ist eine weitere Aufnahmeeinrichtung 1 für ein zuvor beschriebenes Gemisch 2 gezeigt, wobei wiederum für gleiche Teile gleiche Bezugszeichen, wie in den vorangegangenen Fig. 1 bis 19 verwendet werden. Gleichfalls wird, um unnötige Wiederholungen zu vermeiden, auf die Beschreibung der vorangegangenen Fig. 1 bis 19 hingewiesen bzw. bezug genommen.

Die Aufnahmeeinrichtung 1 umfaßt wiederum zumindest den Aufnahmebehälter 5, der einen Aufnahmeraum 117 mit einer diesen begrenzenden bzw. umgrenzenden Innenwandung 18 aufweist. Weiters weist der Aufnahmebehälter 5 zwei in Richtung seiner Längsachse 15 voneinander distanzierte Enden 6, 7 auf, von denen zumindest eines mit einer Öffnung ausgebildet ist. Im Bereich der Innenwandung 118 ist eine innere Abmessung 14 des Aufnahmeraums 117 im Bereich des ersten Endes 6 in einer senkrecht zur Längsachse 15 ausgerichteten Ebene 16 größer der inneren Abmessung 18 im Bereich des weiteren Endes 7 in der dazu parallelen Ebene 17 in der gleichen Raumrichtung, wodurch je nach Abnahme der Abmessung der Aufnahmeraum 117 kegelig bzw. konisch verjüngend ausgebildet ist. An zumindest einem der offenen Enden 6, 7 ist eine hier nicht näher dargestellte, bedarfsweise öffenbare Verschlußvorrichtung 9 zum bedarfsweisen Verschließen des Aufnahmebehälters 5 vorgesehen.

In den Aufnahmeraum 117 bzw. Innenraum 10 ist wiederum die Trennvorrichtung 11 eingesetzt, welche je nach Ausbildung des an der Innenwandung 118 anliegenden Dichtstopfens der Verschlußvorrichtung, ausgehend vom hier offenen Ende in Richtung des weiteren Endes 7, um ein vorbestimmbares Ausmaß von der Stirnseite 19 distanziert angeordnet ist. Die Trennvorrichtung 11 ist in Richtung der Längsachse 15 von den beiden voneinander distanzierten Endbereichen 45, 46 begrenzt. Zwischen diesen voneinander distanzierten Endbereichen 45, 46 ist zumindest ein verschließbarer Strömungskanal 119 angeordnet. Weiters ist zwischen der Trennvorrichtung 11 und dem Aufnahmebehälter 5, insbesondere dessen Innenwandung 118, zumindest eine Dichtungsvorrichtung 120, angeordnet.

Die Trennvorrichtung 11 umfaßt mindestens einen, im vorliegenden Ausführungsbeispiel zwei Grundkörper 121, welche durch mindestens ein Druckelement 122 in der Ausgangsstellung zumindest bereichsweise an die Innenwandung 118 des Aufnahmebehälters 5 angedrückt sind.

Die beiden hier die Trennvorrichtung 11 bildenden Grundkörper 121 bilden in Richtung der Längsachse 15 gesehen jeweils annähernd eine Halbkreisfläche aus, wobei in der Arbeitsstellung, also in der dichtenden Stellung der Strömungskanal 119, welcher hier zwischen den einander zugewandten Bereichen der Grundkörper 121 ausgebildet ist, dichtend, insbesondere flüssigkeitsdicht, verschlossen ist. Diese Schließbewegung des Strömungskanals 119 kann durch die zuvor beschriebene Abnahme, ausgehend von der größeren inneren Abmessung 14 des Aufnahmeraums 117, hin zur kleineren inneren Abmessung 18 im Bereich des weiteren Endes 7 maßlich derart abgestimmt werden, daß die Trennvorrichtung 11 nach Verstellung, ausgehend von der Ausgangslage bzw. Startposition hin zu ihrer Arbeitsstellung dort eindeutig positioniert festgelegt ist, ohne daß ein ungewünschter Durchtritt des schwereren Mediums hin zum leichteren Medium während des Zentrifugiervorganges und auch nach Beendigung desselben erfolgen kann.

Das hier zwischen den Grundkörpern 121 angeordnete Druckelement 122 bewirkt eine radial in Richtung zur Innenwandung 118 gerichtete Druckkraft auf die beiden Grundkörper 121, wodurch die Dichtungsvorrichtung 120 bereits in der Ausgangsstellung zumindest bereichsweise über den Umfang an der Innenwandung 118 zur Anlage gebracht wird.

In der Ausgangsstellung ist durch den Strömungskanal 119 der zwischen der Trennvorrichtung 11 und dem weiteren Ende 7 angeordnete Aufnahmeraum 117 evakuierbar. Nach erfolgter Evakuierung wird dann die Verschlußvorrichtung 9, insbesondere der Dichtstopfen 22, in den Aufnahmeraum 117 des Aufnahmebehälters 5 eingesetzt und so in diesem Zustand gelagert. Diese Aufnahmeeinrichtung 1 steht nunmehr beispielsweise für eine Aufnahme von Körperflüssigkeiten, Gewebeteilen bzw. Gewebekulturen, insbesondere Blut, zur Verfügung, wobei der Dichtstopfen mit einer Nadel durchstechbar ist und durch den im Aufnahmeraum 117 herrschenden Unterdruck eine Befüllung der Aufnahmeeinrichtung 1 erfolgen kann.

Die innere Abmessung 14 bzw. ein innerer Umfang einer Hüllinie des Aufnahmeraums 117 ist in der ersten Ebene 16 größer einer äußeren Abmessung 123 bzw. einem äußeren Umfang einer Hüllinie des bzw. der Grundkörper 121 in seiner bzw. deren Arbeitsstellung und der gleichen Raumrichtung. Dadurch ist gewährleistet, daß in der Ausgangsstellung durch den Strömungskanal 119 ein Durchfließen des in den Aufnahmeraum 117 einzubringenden Gemisches möglich ist. Nach dem Befüllen wird der zuvor beschriebene Zentrifugiervorgang durchgeführt und das Gemisch 2 in die beiden Medien 3, 4 separiert. Dazu ist in der Ausgangsstellung der Strömungskanal 119 zwischen den Enden 6,7 des Aufnahmebehälters 5 im Bereich der Trennvorrichtung 11 ausgebildet. Durch die auf die Trennvorrichtung 11 einwirkende Zentrifugalkraft erfolgt, ausgehend von der Ausgangsstellung, eine Verlagerung der Trennvorrichtung 11 hin zu der davon distanzierten Arbeitsstellung, wobei hier eine innere Abmessung 124 bzw. ein innerer Umfang einer Hüllinie des Aufnahmeraums 117 gleich oder kleiner dem äußeren Umfang einer Hüllinie des oder der Grundkörper 121 bzw. der äußeren Abmessung 123 in der gleichen Stellung ist.

Der oder die Grundkörper 121 der Trennvorrichtung 11 dichten in der Arbeitsstellung den oder die Strömungskanäle 119 selbsttätig ab, wobei dies hier durch die maßliche Abnahme des Aufnahmeraums 117 in Art einer Steuerkurve erfolgt. Dabei kann beispielsweise die Abnahme der inneren Abmessung 14 hin zur inneren Abmessung 124 im Bereich der Arbeitsstellung gleichmäßig bzw. stetig erfolgen. Gleichfalls ist es aber auch möglich, einen Teilabschnitt der Strecke zwischen der Ausgangsstellung und der Arbeitsstellung, ausgehend von der Ausgangsstellung, zylindrisch und den weiteren Teilabschnitt konisch bzw. kegelig verjüngend auszubilden.

Um eine gesicherte Verstellbewegung während der aufzubringenden Zentrifugalkraft auf die Trennvorrichtung 11 zu erzielen, ist die Dichte derselben abhängig von den Dichtewerten der einzelnen zu trennenden Medien 3, 4. Besteht das Gemisch 2 aus Blut, ist die Dichte der Trennvorrichtung größer 1,05 g/cm³ zu wählen. Je nach Höhe der gewählten Zentrifugalkraft für den Zentrifugiervorgang kann die Trennvorrichtung 11 eine Dichte zwischen 1,5 g/cm³ und 3,5 g/cm³, bevorzugt zwischen 2,0 g/cm³ und 2,5 g/cm³, aufweisen.

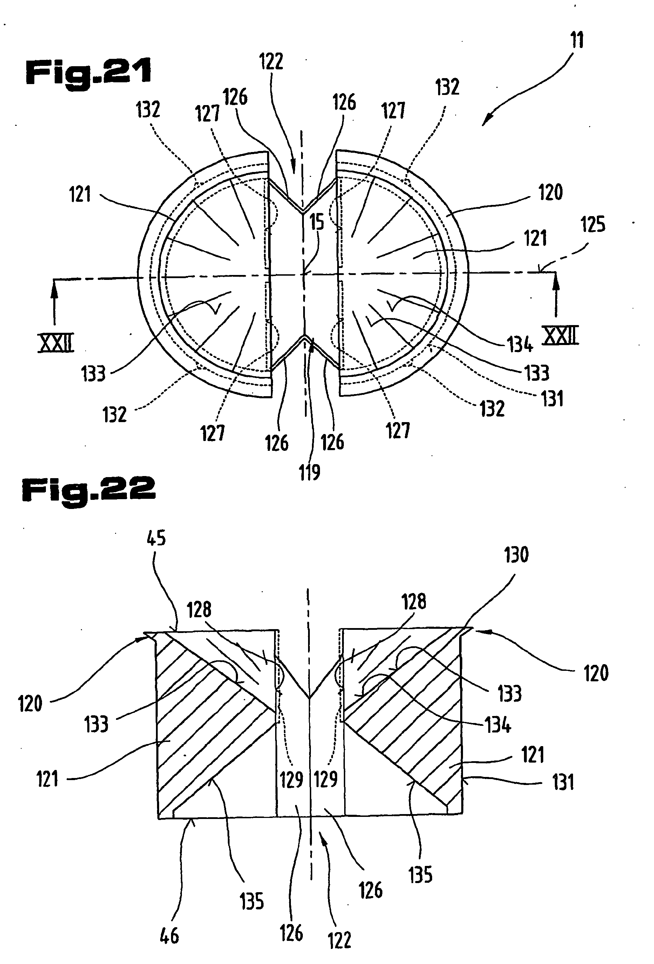

Wie nun besser aus den Fig. 21 und 22 zu ersehen ist, ist bei diesem Ausführungsbeispiel gezeigt, daß die Trennvorrichtung 11 aus zwei Grundkörpern 121 und bevorzugt zentrisch zwischen diesen angeordneten Druckelementen 122 gebildet ist. Je nach Größe der äußeren Abmessung 123 der Trennvorrichtung 11 kann diese aber auch mehrere dieser Grundkörper 121 aufweisen. Wesentlich dabei ist aber, daß die Grundkörper 121 während ihrer gesamten Verstellbewegung gegenüber dem Aufnahmebehälter 5 stets die gleiche relative Verstellgeschwindigkeit in bezug zum Aufnahmebehälter 5 aufweisen, wodurch eine gemeinsame Verstellung während des Zentrifugiervorganges erfolgt und so auch in der Arbeitsstellung ein dichtender, insbesondere flüssigkeitsdichter Abschluß zwischen den beiden voneinander getrennten Aufnahmeräumen 117 im Aufnahmebehälter 5 gewährleistet ist.

Zur Erzielung einer möglichst gleichmäßigen Druckkraft ist in bezug zu einer durch die Längsachse 15 verlaufenden und senkrecht zum Strömungskanal 119 ausgerichteten Symmetrieebene 125 beidseits dieser jeweils ein Druckelement 122 den Grundkörpern 121 der Trennvorrichtung 11 zugeordnet und somit zwischen den einander zugewandten Bereichen der Grundkörper 121 angeordnet. Um eine dichtende Lage der Grundkörper 121 in deren Arbeitsstellung zu erzielen, sind die die Trennvorrichtung 11 bildenden Grundkörper 121 in einer senkrecht zur Längsachse 15 ausgerichteten Ebene relativ zueinander verstellbar, wodurch diese stets zueinander die gleiche relative Lage zum Aufnahmebehälter 5 einnehmen und so auch gleichzeitig verstellbar sind.

Durch die Anordnung des oder der Druckelemente 122 sind die Grundkörper 121 der Trennvorrichtung 11 zueinander in ihrer relativen Lage positioniert gehaltert und somit auch miteinander bewegungsverbunden. In vorteilhafter Weise sind die Druckelemente 122 symmetrisch zur Längsachse 15 angeordnet und können beispielsweise durch in Richtung der Längsachse 15 gesehen V-förmig und in Richtung zur Längsachse 15 hin zusammenlaufend durch miteinander verbundene Federstege 126 gebildet sein. Bevorzugt ist der Grundkörper 121 bzw. die Grundkörper 121 sowie das Druckelement 122 bzw. die Druckelemente 122 aus einem zueinander gleichartigen Werkstoff gebildet, wodurch die Trennvorrichtung 11 beispielsweise in einem einzige Fertigungsvorgang, wie beispielsweise in einem Spritzgußwerkzeug durch einen Spritzgußvorgang, hergestellt werden kann.

Zur Erzielung einer dichtenden Anlage der einander zugewandten Bereiche der Grundkörper 121 kann eine entsprechende, in der Fig. 21 in strichlierten Linien angedeutete Ausnehmung 127 angeordnet bzw. vorgesehen sein. Dadurch können die einzelnen Federstege 126 während der Bewegung von der Ausgangsstellung in die Arbeitsstellung in die Ausnehmung bzw. Ausnehmungen 127 eingeklappt werden, wodurch eine ebenflächige Anlage zur Abdichtung des Strömungskanals 119 zwischen den Grundkörpern 121 erzielbar ist. Die das Druckelement 122 bildenden Federstege 126 sind an den einander zugewandten Endbereichen miteinander verbunden und an den davon abgewandten Endbereichen mit den Grundkörpern 121 bewegungsverbunden. Bedingt durch die V-förmige Ausbildung wird eine auf die vom Strömungskanal 119 abgewandte Richtung ausübende Druckkraft, ausgehend von den Druckelementen 122 aufgebaut, welche dazu dient, bis zum gegenseitigen Aneinanderliegen der Grundkörper 121 im Bereich der einander zugewandten Bereiche einen Durchfluß durch den Strömungskanal 119 zu ermöglichen. Dies ist einerseits für den Einfüllvorgang in den Aufnahmeraum 117 und andererseits für das Hindurchtreten eines der zu trennenden Medien während dem Zentrifugiervorgang notwendig.

Die einander zugewandten Bereiche der Grundkörper 121 bilden bevorzugt im Endbereich 45 bevorzugt plan aneinanderliegende Dichtflächen 128 aus. Zusätzlich dazu ist es aber noch möglich, zwischen den Grundkörpern 121 der Trennvorrichtung 11, im Bereich des dem ersten Ende 6 des Aufnahmebehälters 5 zugewandten Endbereiches 45, eine Dichtungsanordnung 129 zur Abdichtung des oder der Strömungskanäle 119 vorzusehen. Diese Dichtungsanordnung 129 ist in der Fig. 22 im Bereich der Dichtflächen 128 in strichlierten Linien angedeutet und kann durch die unterschiedlichsten Ausbildungen realisiert sein. Dies können beispielsweise ineinandergreifende bzw. überlappende Dichtlippen, Lamellendichtungen usw. sein.

Die Dichtungsvorrichtung 120, welche zwischen der Trennvorrichtung 11 und der Innenwandung 118 des Aufnahmeraums 117 angeordnet ist, soll im Bereich des dem ersten Endes 6 des Aufnahmebehälters 5 zugewandten Endbereiches 45 angeordnet sein, um bereits am obersten Ende der Trennvorrichtung 11 eine Ansammlung des Gemisches 2 zwischen den Grundkörpern 121 und der Innenwandung 118 zu vermeiden, was zu späteren Vermischungen von bereits getrennten Medien führen würde. Dies wäre dann der Fall, wenn beispielsweise die Dichtungsvorrichtung 120 vom ersten Endbereich 45 in Richtung des weiteren Endbereiches 46 distanziert angeordnet wäre und so bereits während dem Einfüllvorgang beide Bestandteile des Gemisches in diesen Zwischenraum eindringen könnten, welcher während des gesamten Zentrifugiervorganges nicht mehr entleert und auch nicht getrennt werden kann und somit beide Bestandteile zumindest in Teilmengen dann in dem Aufnahmeraum 117 zwischen der Trennvorrichtung 11 und der Verschlußvorrichtung 9 verbleiben würden, was zu einem Verunreinigen des hier leichteren Mediums führen würde.

Die Dichtungsvorrichtung 120 wird bevorzugt durch zumindest eine über den äußeren Umfang des Grundkörpers 121 durchlaufende Dichtlippe 130 gebildet, welche den Grundkörper 121 radial nach außen in die von der Längsachse 15 abgewandte Richtung überragt. Dadurch, daß die Dichtlippe 130 in einem gewissen Ausmaß elastisch verformbar ist, können gewisse Fertigungstoleranzen, insbesondere Durchmesserunterschiede, zwischen den Grundkörpern 11 und dem Aufnahmebehälter 5 aufgefangen werden. Wesentlich dabei ist, daß die Dichtlippe 130 auf alle Fälle in der Arbeitsstellung den Bereich zwischen der Trennvorrichtung 11 und der Innenwandung 118 des Aufnahmeraums 117 vollständig gegeneinander, insbesondere flüssigkeitsdicht, abdichtet.

Der oder die Grundkörper 121 können zwischen den voneinander distanzierten Endbereichen 45, 46, beispielsweise bei der Wahl von zwei Grundkörpern 121 jeweils als Halbzylinder ausgebildet sein, über deren Außenumfang die Dichtlippe 130 vorragend ausgebildet ist.

Unabhängig davon ist es aber auch möglich, die Grundkörper 121 in einem der Innenwandung 118 des Aufnahmebehälters 5 zugeordneten Bereich durch einen Abschnitt eines Hohlzylinders bzw. Hohlkegelstumpfes auszubilden, wodurch Materialeinsparungen erzielbar sind. Wird nur ein Hohlzylinder bzw. Hohlkegelstumpf verwendet, so ist auf die Anordnung der zwischen den Grundkörpern 121 auszubildenden Dichtflächen 128 bedacht zu nehmen, um in der aneinander anliegenden Position den Strömungskanal 119 selbsttätig abdichten zu können.

Wie bereits zuvor beschrieben, überragt die Dichtlippe 130 die Grundkörper 121, welche somit eine geringere Außenabmessung gegenüber der den Aufnahmeraum 17 begrenzenden Innenwandung 118 über den gesamten Verstellweg aufweisen. Um ein Verkanten bzw. Kippen der Trennvorrichtung 11 während des Verstellvorganges zu vermeiden, ist es vorteilhaft, an den Grundkörpern 121 mehrere über den äußeren Umfang derselben und eine Außenfläche 131 in die von der Längsachse 15 abgewandte Richtung überragende Stützelemente 132 anzuordnen. Diese Stützelemente 132 sind bevorzugt symmetrisch zur Längsachse 15 verteilt, über den äußeren Umfang an der Außenfläche 131 angeordnet und können beispielsweise durch parallel zur Längsachse 15 ausgerichtete Stege gebildet sein. Diese Stützelemente 132 können aber auch durch über die Außenfläche 131 vorragende Noppen, kalottenförmige Vorsprünge usw. gebildet sein, welche in beliebiger Anordnung über die Außenfläche 131 verteilt angeordnet sein können.

Zur Verbesserung der Fließbedingungen zwischen den beiden voneinander distanzierten Endbereichen 45, 46 und der Verhinderung von Totvolumen, können die Grundkörper 121 im Bereich des dem ersten Ende des Aufnahmebehälters 5 zugewandten ersten Endbereiches 45 eine bevorzugt aus Kegelabschnitten 133 gebildete und in Richtung zur Längsachse 15 sowie hin zum weiteren Endbereich 46 verjüngend ausgebildete Leitfläche 134 aufweisen. Weiters ist es noch vorteilhaft, wenn die Grundkörper 121 im Bereich des dem weiteren Ende 7 des Aufnahmebehälters 5 zugewandten zweiten Endbereiches 46 eine in Richtung zur Längsachse 15 sowie hin zum ersten Endbereich 45 geneigt verlaufende Anströmfläche 135 aufweisen.

Dadurch kann ein ungehindertes Einströmen des in den Aufnahmeraum 117 einzufüllenden Gemisches in Richtung des weiteren Endes 7 des Aufnahmebehälters 5 erfolgen, wodurch dieses auch von den Randbereichen, also aus dem Bereich der Innenwandungen 118 in Richtung des Strömungskanals 119, geleitet wird. Zusätzlich wird durch die geneigten Anströmflächen 135 ein Durchtritt des leichteren Mediums durch den Strömungskanal 119 während des Separiervorganges ermöglicht und auch hier die Ausbildung eines Totvolumens verhindert.

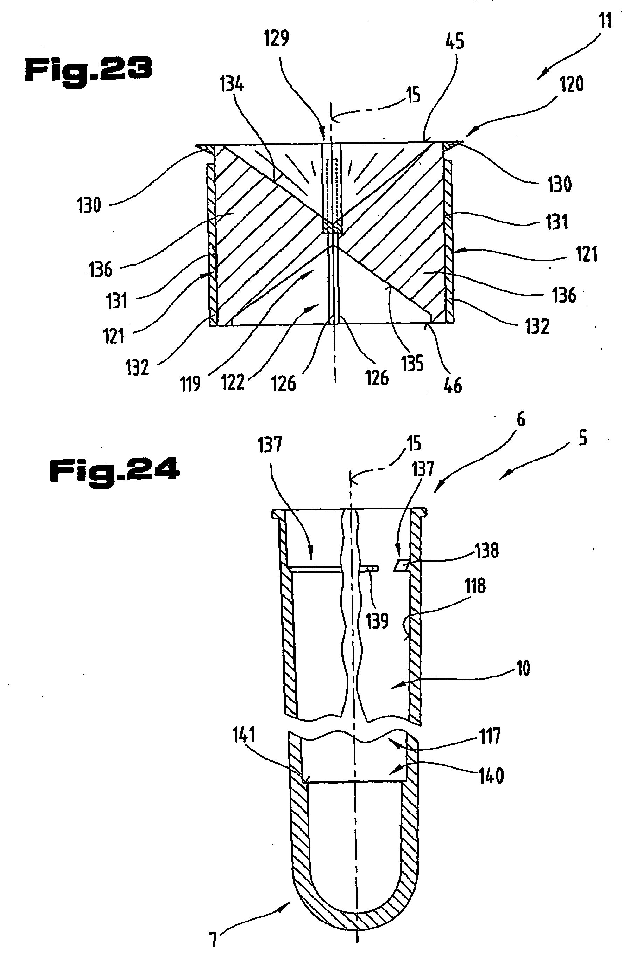

In der Fig. 23 ist eine weitere mögliche und gegebenenfalls für sich eigenständige Ausbildung der Trennvorrichtung 11 gezeigt, wobei wiederum für gleiche Teile gleiche Bezugszeichen wie in den vorangegangen Fig. 1 bis 22 verwendet werden. Da sich diese hier gezeigte Trennvorrichtung 11 nur in einigen Details von jener Ausführungsform, wie diese in den Fig. 20 bis 22 beschrieben worden ist, unterscheidet, wird auf die dort detaillierte Beschreibung hingewiesen bzw. Bezug genommen.

Auch bei dieser Ausführungsform ist die Trennvorrichtung 11 aus mehreren, bevorzugt zwei Grundkörpern 121 gebildet, welche durch das oder die Druckelemente 122 in Form der Federstege 126 miteinander bewegungsverbunden sind. Weiters sind die beiden Grundkörper 121 in einer dichtenden Stellung im Bereich des Strömungskanals 119 gezeigt. Zur besseren Variierung der Dichte der gesamten Trennvorrichtung 11 ist bei diesem Ausführungsbeispiel gezeigt, daß der oder die Grundkörper 121 der Trennvorrichtung 11 jeweils durch einen Tragkörper 136 und die daran angeordnete Dichtungsvorrichtung 120 und/oder Dichtungsanordnung 129 gebildet sind. Dabei ist es vorteilhaft, wenn die Werkstoffe zur Bildung des Tragkörpers 136 bzw. der Dichtungsvorrichtung 120 und/oder Dichtungsanordnung 129 zueinander unterschiedlich sind.

Der Tragkörper 136 soll gegenüber der Dichtungsvorrichtung 120 und/oder der Dichtungsanordnung 129 eine höhere Dichte, bei gegebenenfalls einem höheren Elastizitätsmodul, aufweisen. Dadurch kann bei gleichem Volumen des Tragkörpers 136 bei Einsatz eines Werkstoffes mit höherer Dichte eine höhere Masse erzielt werden, wodurch die Verstellbewegung bei Beaufschlagung auch mit einer geringeren Zentrifugalkraft gesichert durchführbar ist.

Die Dichtungsvorrichtung 120 bzw. Dichtungsanordnung 129 kann dabei aus einem Silikonkautschuk, Pharmagummi, Brombutylkautschuk, Gummi einem Gel, einem Thermoplastischen Elastomer (TPE), Thermoplastischen Polyurethan (TPU) oder einem anderen elastomeren Kunststoff und der Tragkörper 136 aus der Gruppe von Polyethylenterephthalat (PET), Polypropylen (PP), Polyethylen (PE), Polystyrol (PS), High-Density-Polyethylen (PE-HD), Acrylnitril-Butadien-Styrol-Copolymere (ABS), Thermoplastische Elastomere (TPE), Thermoplastisches Polyurethan (TPU), Ultrahochmolekulares Polyethylen mit sehr hoher molarer Masse (PE-UHMW), Polycarbonat (PC), Polyamid (PA) Polyoxymethylen (POM) bzw. einem anderen thermoplastischen Kunststoff sowie gegebenenfalls einer Kombination daraus gebildet sein. Die Dichtungsanordnung 129 kann, muß jedoch nicht zwingend, vorgesehen sein. Selbstverständlich können aber auch die Werkstoffe zur Bildung der Dichtungsvorrichtung 120 bzw. Dichtungsanordnung 129 zwischen den Tragkörpern 136 zueinander unterschiedlich ausgebildet sein. Die die Dichtungsvorrichtung 120 bildenden Dichtlippen 130 überragen zur besseren Abdichtung die Außenfläche 131 auf die von der Längsachse 15 abgewendete Seite und bilden, wie zuvor bereits detailliert beschrieben worden ist, den dichtenden Abschluß nach Beendigung des Zentrifugiervorganges zwischen der Trennvorrichtung 11 und der Innenwandung 118 aus. Die weitere Abdichtung des Strömungskanals 119 zwischen den Grundkörpern 121, insbesondere den Tragkörpern 136 erfolgt durch die dem ersten Endbereich 45 zugeordnete Dichtungsanordnung 129, welche hier vereinfacht schematisch durch Dichtstreifen dargestellt worden ist. Diese Dichtungsanordnung 129 kann wiederum unterschiedlichst ausgebildet sein, wobei diese zur dichtenden Anlage der einander zugewandten Bereiche der Grundkörper 121 im Bereich des Strömungskanals 119 ausgebildet sein können.

Gleichfalls ist wiederum zwischen den Grundkörpern 121 das oder die Druckelemente 122 angeordnet, welche der Einfachheit halber wiederum durch hier aneinanderliegende Federstege 126 dargestellt sind. Selbstverständlich kann aber auch das Druckelement 122 jede beliebige andere Form aufweisen wobei jedoch sicher gestellt sein muß, daß einerseits eine ausreichende entgegengesetzte Druckkraft auf die einzelnen Grundkörper 121 ausgeübt und andererseits in der Arbeitsstellung ein dichtender Verschluß des Strömungskanals 119 ermöglicht wird.

Weiters ist in dieser Figur noch dargestellt, daß zur Lagestabilisierung während des Verstellvorganges im Bereich der Außenfläche 131 diese zumindest bereichsweise überragende Stützelemente 132, beispielsweise in Form von längsstegen- bzw. rippen- oder kalottenförmigen Ansätzen verteilt über den Umfang zur Abstützung an der Innenwandung 118 vorgesehen sein können. Diese Stützelemente 132 sind in ihrem Überstand über die Außenfläche 131 derart bemessen, daß diese während der gesamten Verstellbewegung bis hin zum Erreichen der Arbeitsstellung an der Innenwandung 118 anliegen und die Dichtungsvorrichtung 120, insbesondere die Dichtlippe 130, auch noch die Hüll-Linie um die Stützelemente 132 nach außen in Richtung zur Innenwandung 118 überragt. Bedingt durch die Elastizität der Dichtlippen 130 werden diese im Überstandsbereich über die Hüll-Linie um die Stützelemente 132 auf die von der Trennvorrichtung 11 abgewandte Seite verformt. Je nach Größe des Überstandes kann auch noch die notwendige Verstellkraft, welche zur Verstellung von der Ausgangslage hin zur Arbeitsstellung aufzubringen ist, festgelegt werden.

Wird als Stützelement 132 ein durchlaufender Steg verwendet, ist darauf Bedacht zu nehmen daß dieser in jedem Fall von der Dichtlippe 130 getrennt ausgeführt ist, um eine ungehinderte Verformungsbewegung der Dichtlippe 130 zur Erzielung einer dichtenden Anlage an der Innenwandung 118 sicherstellen zu können. Eine äußere Umhüllende im Bereich der Stützelemente 132 ist dabei kleiner als der äußere Durchmesser der Dichtlippen 130 der Dichtungsvorrichtung 120 in deren unverformten Zustand. Durch den Überstand der Dichtlippen 130 über die Umhüllende durch die Stützelemente 132 erfolgt bereits in der Ausgangsstellung bedingt durch die Aufbringung der Druckkraft über das oder die Druckelemente 122 eine gewisse Verformung der Dichtlippen 130. Das Ausmaß der Verformung ist vom Überstand der Dichtlippen 130 über die Umhüllende um die Stützelemente 132 abhängig. Ein Festsitzen der Trennvorrichtung 11 im Bereich der Arbeitsstellung erfolgt einerseits durch die Anlage der einzelnen Stützelemente 132 an der Innenwandung 118 des Aufnahmebehälters 11 und andererseits durch die verformten Dichtlippen 130 in der dichtenden Lage gegenüber der Innenwandung 118.

In der Fig. 24 sind unterschiedliche Ausbildungsmöglichkeiten des Aufnahmebehälters 5 in einer einzigen Figur vereinfacht dargestellt, wobei diese selbstverständlich aber auch beliebig miteinander kombinierbar sind. Der besseren Übersichtlichkeit halber wird in dieser Fig. auf die Darstellung der Trennvorrichtung 11 sowie der Verschlußvorrichtung 9 verzichtet.

In dem dem Ende 6 des Aufnahmebehälters 5 benachbarten Bereich der Ausgangsstellung für die in den Innenraum 10 bzw. Aufnahmeraum 117 einzusetzende Trennvorrichtung 11 sind unterschiedliche Ausbildungen von Rückhaltevorrichtungen 137 gezeigt. Dabei ist hier im rechten Teil der Fig. dargestellt, daß die Rückhaltevorrichtung 137 durch zumindest einen über den Umfang der Innenwandung 118 in Richtung zur Längsachse 15 vorragenden Ansatz 138 und/oder durch einen zumindest bereichsweise über den Umfang der Innenwandung 118 in Richtung zur Längsachse 15 vorragenden Steg 139 gebildet ist. Dabei kann sowohl der Ansatz 138 und/oder der Steg 139 nur bereichsweise über den Umfang sowie gegebenenfalls auch durchlaufend über den gesamten Umfang der Innenwandung 118 durchlaufend angeordnet sein.

Im hier linken oberen Bereich der Fig. 24 ist eine weitere Ausbildung der Rückhaltevorrichtung 137 gezeigt, welche durch eine Verkleinerung der inneren Abmessung 14 des Aufnahmeraums 117 gebildet ist. Diese Verkleinerung kann dadurch bewirkt werden, daß beispielsweise ausgehend vom Ende 6 des Aufnahmebehälters 5 dieser bis hin zur Rückhaltevorrichtung 137 die normale Wandstärke des Aufnahmebehälters 5 und ab der Rückhaltevorrichtung 137 in Richtung des weiteren Endes 7 eine dazu größere Wandstärke aufweist, wobei die Vergrößerung der Wandstärke durch einen Versatz der Innenwandung 118 in Richtung zur Längsachse 15 erfolgt. Unabhängig davon ist es aber auch möglich, die Wandstärke des Aufnahmebehälters 5 zwischen der Ausgangsstellung und dem weiteren Ende 7 im Bereich der üblichen Wandstärke zu wählen und lediglich die Wandstärke zwischen der Ausgangsstellung und dem hier offenen Ende 6 des Aufnahmebehälters 5 geringer auszubilden.

Je nach Ausbildung der Rückhaltevorrichtung 137 ist eine Lagepositionierung der Trennvorrichtung 11 bis zum Erreichen einer vorbestimmbaren Zentrifugalkraft, bei der die Rückhaltekräfte überwunden werden und die Verlagerung der Trennvorrichtung 11 relativ zum Aufnahmebehälter 5 bis hin zum Erreichen der Arbeitsstellung erfolgt, vorbestimmbar.

Zur Erzielung einer anderen gesicherten Positionierung bzw. relativen Lagefixierung der Trennvorrichtung 11 im Bereich der Ausgangsstellung kann zwischen dem Aufnahmebehälter 5 und der Trennvorrichtung 11 die Rückhaltevorrichtung 137 durch eine hier nicht näher dargestellte nutförmige Vertiefung, welche umlaufend über den inneren Umfang der Innenwandung 118 in dieser vertieft angeordnet ist, gebildet sein.

Zur Erzielung einer gesicherten Positionierung bzw. relativen Lagefixierung der Trennvorrichtung 11 im Bereich der Arbeitsstellung kann zwischen dem Aufnahmebehälter 5 und der Trennvorrichtung 11 eine Positioniervorrichtung 140 angeordnet sein. Diese Positioniervorrichtung 140 kann beispielsweise durch eine Verkleinerung der inneren Abmessung 124 des Aufnahmeraums 117 und unter Bildung einer in etwa in einer senkrecht zur Längsachse 15 ausgerichteten Anschlagfläche 141 gebildet sein. An dieser Anschlagfläche 141 kann sowohl der weitere Endbereich 46 der Trennvorrichtung 11 bzw. dessen Grundkörper 121 oder aber auch die im ersten Endbereich 45 angeordnete Dichtungsvorrichtung 120, insbesondere die Dichtlippen 130 zur Anlage gebracht werden. Damit wird eine dichtende, insbesondere flüssigkeitsdichte, Abdichtung der voneinander getrennten Medien nach Beendigung des Zentrifugiervorganges auch über eine längere Lagerdauer erzielt.

Die Verkleinerung des Innenraums 10 ausgehend von der Ausgangsstellung hin zur Arbeitsstellung ist auch bei diesem hier gezeigten Aufnahmebehälter 5 realisiert, wie dies bereits zuvor beschrieben worden ist und bildet somit die Steuerkurve für das selbsttätige Schließen des oder der Strömungskanäle 119 im Bereich der Trennvorrichtung 11 aus.

Die zuvor beschriebene Verjüngung des Aufnahmebehälters 5 in seinem Innenraum 10 bzw. Aufnahmeraum 117 zwischen den beiden voneinander distanzierten Ebenen 16, 17 kann zwischen 0,1° und 3,0° bevorzugt zwischen 0,6° und 0,8°, betragen.

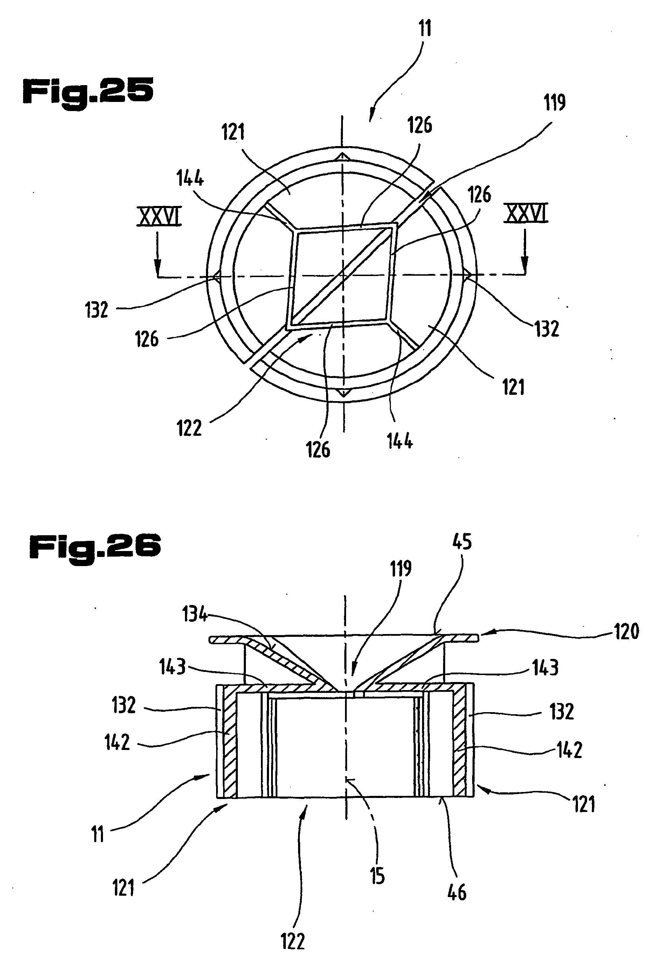

In den Fig. 25 und 26 ist eine weitere mögliche und gegebenenfalls für sich eigenständige Ausbildung der Trennvorrichtung 11 mit dem Druckelement 122 dargestellt, wobei wiederum für gleiche Teile gleiche Bezugszeichen wie in den vorangegangenen Fig. 1 bis 24 verwendet werden. Gleichfalls wird um unnötige Wiederholungen zu vermeiden, auf die detaillierte Beschreibung in den vorangegangenen Fig. 1 bis 24 hingewiesen bzw. Bezug genommen.

Die Trennvorrichtung 11 ist wiederum aus den Grundkörpern 121 gebildet, wobei zwischen den einander zugewandten Grundkörpern der Strömungskanal 119 ausgebildet ist. Die Dichtungsvorrichtung 120 ist wiederum im ersten Endbereich 45 im Bereich des äußeren Umfangs der Grundkörper 121 zur Abdichtung der voneinander zu trennenden Aufnahmeräume 117 angeordnet und kann gemäß den zuvor in den Fig. 20 bis 23 beschriebenen Ausführungsformen entsprechen. Gleiches gilt auch für die Ausbildung der als Kegelabschnitt ausgebildeten Leitfläche 134, welche von den Randbereichen weg hin in Richtung zur Längsachse 15 verjüngend ausgebildet ist und in den Strömungskanal 119 einmündet.

Ausgehend vom weiteren Endbereich 46 der Trennvorrichtung 11 sind die Grundkörper 121 jeweils als Hohlzylindersegmente 142 - im vorliegenden Fall bei zwei Grundkörpern sich in etwa jeweils über einen Halbkreis erstreckend - ausgebildet. Im Bereich der zusammenlaufenden Leitflächen 134 sind zur Verbindung mit den Hohlzylindersegmenten 142 Stimwandteile 143 angeordnet, welche in etwa in einer senkrecht zur Längsachse 15 ausgerichteten Ebene verlaufen.

Das Druckelement 122 ist wiederum durch die miteinander in Verbindung stehenden Federstege 126 gebildet, welche in Richtung der Längsachse 15 gesehen zueinander parallelogrammförmig angeordnet sind. Die den jeweils gegenüberliegenden Grundkörpern 121 zugehörigen Federstege 126 sind im Bereich des Strömungskanals 119 miteinander verbunden und in einer zum Strömungskanal 119 in etwa um 90° versetzten Ebene, gegebenenfalls über Haltestege 144 an den gegenüberliegenden Hohlzylindersegmenten 142 abgestützt.
Durch die symmetrische Aufteilung der Federstege 126 in Bezug zum Strömungskanal 119 sowie die dazu in etwa rechtwinkelig versetzte Abstützung der Federstege 126 an den Hohlzylindersegmenten 142 werden die Grundkörper 121 in etwa symmetrisch zum Strömungskanal 119 an die jeweils gegenüberliegenden Innenwandungen 118 des Aufnahmebehälters 5 während deren gesamten Anordnung derselben innerhalb des Aufnahmebehälters 5 angedrückt.

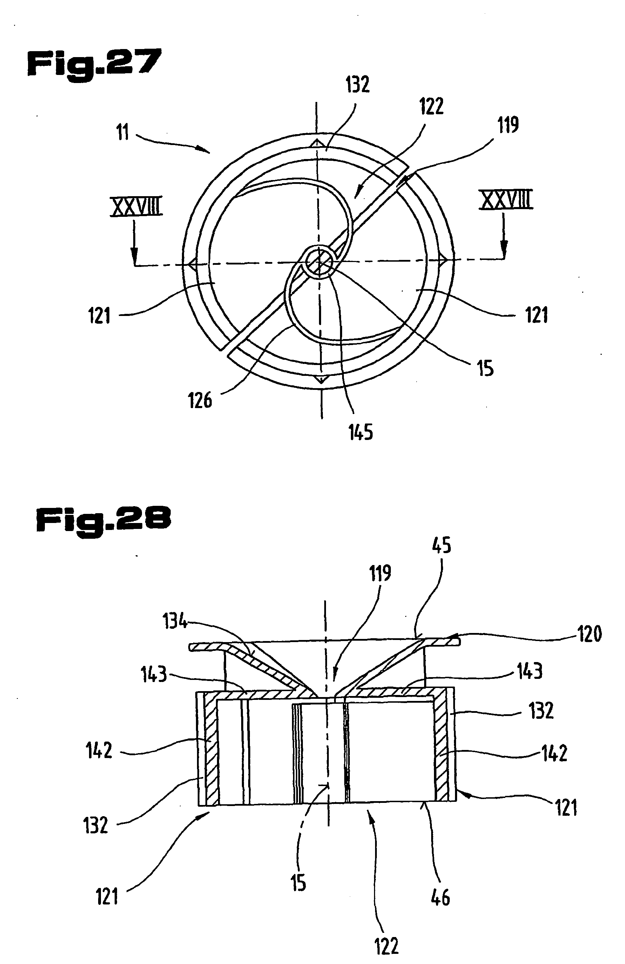

In den Fig. 27 und 28 ist eine weitere mögliche Anordnung des Druckelements 122 für die die Trennvorrichtung 11 bildenden Grundkörper 121 gezeigt, wobei die Ausbildung der Grundkörper 121 gleich wie in den vorangegangen Fig. 25 und 26 ist, wodurch hier um unnötige Wiederholungen zu vermeiden, auf diese Beschreibung hingewiesen bzw. Bezug genommen wird.

Das Druckelement 122 ist wiederum zwischen den Hohlzylindersegmenten 142 zentrisch zur Längsachse 15 zwischen den Grundkörpern 121 angeordnet, wobei die Federstege 126 in Richtung der Längsachse 15 gesehen einen gekrümmt ausgebildeten Längsverlauf aufweisen und durch die gegenläufig ausgebildete Krümmung die notwendige Druckkraft in etwa senkrechter Richtung zum Strömungskanal 119 auf die Grundkörper 121 aufbringen. Im Bereich der Längsachse 15 ist ein hier kreisförmig ausgebildeter Verbindungsteil 145 vorgesehen, wobei die einander zugewandten Enden der Federstege 126 mit diesem in einer etwa senkrecht zum Strömungskanal 119 ausgerichteten Ebene verbunden sind. Die weiteren Enden der bogenförmig gekrümmten Federstege 126 sind an der Innenseite der Hohlzylindersegmente 142 in etwa der gleichen Ebene mit diesen verbunden.

Bei den zuvor in den Fig. 25 bis 28 beschriebenen Druckelementen 122 stehen die Federstege 126 jeweils nur an gegenüberliegenden Bereichen in Bezug zum Strömungskanal 119 ausschließlich mit den Hohlzylindersegmenten 142 in Verbindung, um die Federwirkung ungehindert auf die Grundkörper 121 übertragen zu können. Eine Verbindung der Federstege 126 mit den Stirnwandteilen 143 ist in allen Fällen zu vermeiden.

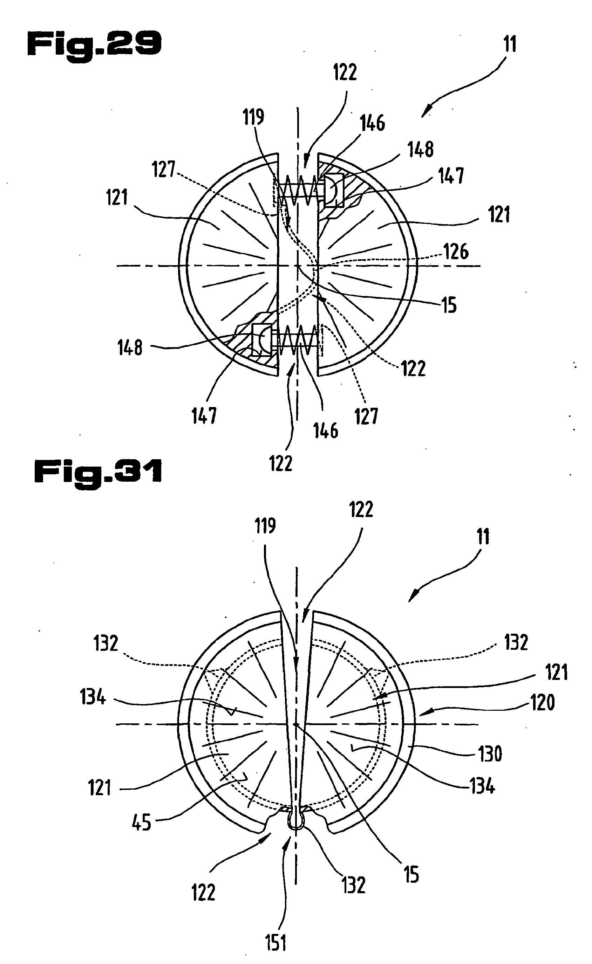

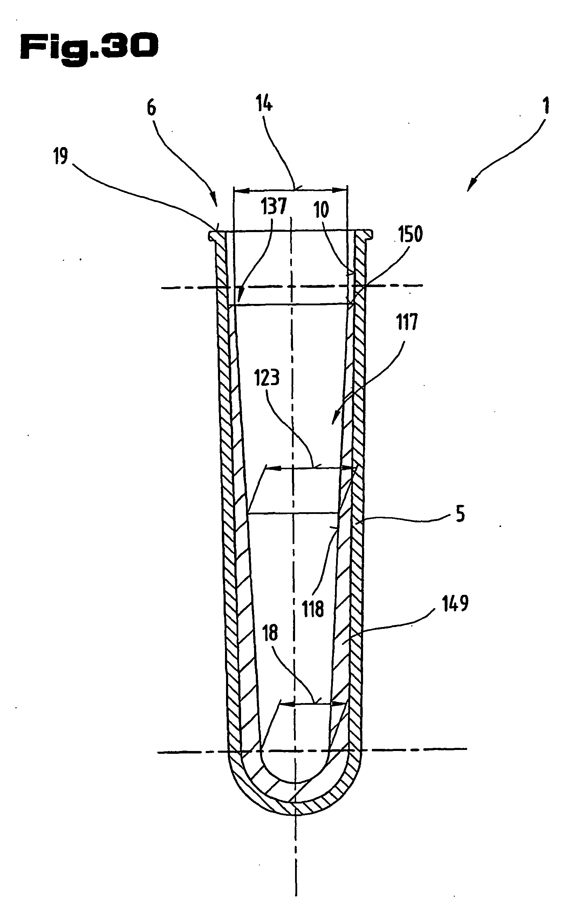

In der Fig. 29 ist eine weitere Anordnungsmöglichkeit der Druckelemente 122 zwischen den Grundkörpern 121 in einer vereinfachten Darstellung gezeigt, wo wiederum für gleiche Teile gleiche Bezugszeichen wie in den vorangegangenen Fig. 1 bis 28 verwendet werden.

Bei diesem hier gezeigten Ausführungsbeispiel sind die einzelnen Druckelemente 122 beispielsweise durch Spiralfedern gebildet, welche auf den einander zugewandten Bereichen der Grundkörper 121 abgestützt sind. Zur Aufnahme derselben in den einander zugewandten Wandteilen der Grundkörper 121 in der geschlossenen und dichtenden Stellung des Strömungskarials 119 können wiederum entsprechende Ausnehmungen 127 in zumindest einer dieser Flächen vertieft angeordnet sein.

Zur Erleichterung der Montage und der gegenseitigen Halterung der einzelnen Grundkörper 121 relativ zueinander ist hier noch zusätzlich dargestellt, daß im Bereich der Druckelemente 122 ausgehend von zumindest einem der Grundkörper 121 sich in Richtung des gegenüberliegenden Grundkörpers 121 zumindest ein Führungsteil 146 erstreckt und dieser in eine im anderen Grundkörper 121 vertiefte Aufnahmeöffnung 147 eingreift. Weiters ist es noch vorteilhaft, wenn an dem in die Aufnahmeöffnung 147 ragenden Endbereich des Führungsteils 146 ein Rückhaltefortsatz 148 angeordnet ist, der in seiner äußeren Abmessung in radialer Richtung den Führungsteil 146 überragt. Die Aufnahmeöffnung 147 weist in radialer Richtung zum Führungsteil 146 im Bereich des Rückhaltefortsatzes 148 eine größere Abmessung auf als in dem unmittelbar an den Strömungskanal 119 angrenzenden Bereich. In diesem Bereich weist die Aufnahmeöffnung 147 in etwa die Abmessung des Führungsteils 146 auf. Durch die elastische Verformung ist ein Einschieben des einen größeren Durchmesser aufweisenden Rückhaltefortsatzes 148, in den ersten Teil der Aufnahmeöffnung 147 möglich und schnappt dann in die zur Aufnahme des Rückhaltefortsatzes 148 ausgebildete größere Aufnahmeöffnung 147 ein. Durch das Zusammenwirken mit dem Druckelement 122 werden die beiden Grundkörper 121 im Bereich des Strömungskanals 119 auseinander gedrückt, wobei eine Begrenzung und damit ein Auseinanderfallen der Grundkörper 121 durch das Zusammenwirken des Rückhaltefortsatzes 148 mit der verkleinerten Aufnahmeöffnung 147 verhindert wird.

Weiters ist es aber auch noch möglich, das oder die Druckelemente 122 nicht wie hier gezeigt im Bereich der Führungsteile 146 anzuordnen, sondern ein eigenes Druckelement 122 an einem der Grundkörper 121 zu haltern, wie dies in strichlierten Linien vereinfacht dargestellt worden ist.

Dieses Druckelement 122 weist einen gekrümmten Längsverlauf auf und ist als Federsteg 126 ausgebildet, der an einem Endbereich mit einem der Grundkörper 121 verbunden ist und sich in Richtung der Längsachse 15 gesehen bogenförmig im Bereich des Strömungskanals 119 in Richtung des gegenüberliegenden Grundkörpers 121 erstreckt. Durch die Anordnung der Führungsteile 146 und der damit zusammenwirkenden Aufnahmeöffnung 147 ist eine gegenseitige Ausrichtung der Grundkörper 121 möglich, wobei die Druckvorrichtung wiederum zwischen den beiden einander zugewandten Bereichen der Grundkörper 121 jedoch getrennt von den Führungsteilen 146 zur gegenseitigen Distanzierung und Ausbildung des Strömungskanals 119 angeordnet ist.

In der Fig. 30 ist eine weitere mögliche Ausbildung der Aufnahmeeinrichtung 1 gezeigt, welche bei diesem Ausführungsbeispiel aus dem Aufnahmebehälter 5 und einem in dessen Innenraum 10 eingesetzten Behälter 149 gebildet ist. Auf die Darstellung der Verschlußvorrichtung 9 sowie der Trennvorrichtung 11 wurde der besseren Übersichtlichkeit halber verzichtet.

Diese Aufnahmeeinrichtung 1 kann für alle jene Ausbildungen der Trennvorrichtung 11 eingesetzt werden, denen zum Verschließen des Strömungskanals 119 bzw. der Verbindungsöffnung 44 das Prinzip der Verringerung der Innenabmessung des Aufnahmeraums 117 ausgehend von der Ausgangsstellung hin zu deren Arbeitsstellung zu Grunde liegt.

Der Aufnahmebehälter 5 überragt mit seinem hier offen ausgebildeten Ende 6 eine Stirnseite 150 des Behälters 149 um eine vorbestimmbare Distanz, welche derart gewählt sein kann, daß die Stirnseite 150 die zuvor beschriebene Rückhaltevorrichtung 137 für die in den Aufnahmeraum 117 einzusetzende Trennvorrichtung 11 ausbildet. Die innere Abmessung 14 im Bereich der Stirnseite 150 ist zur Ausbildung des Strömungskanals 119 bzw. der Verbindungsöffnung 44 im Bereich der Trennvorrichtung 11 derart im Verhältnis zur Trennvorrichtung 11 bzw. deren Grundkörpern 121 gewählt, daß stets ein Durchtritt für das in den Aufnahmeraum 117 einzufüllende Gemisch 2 ermöglicht wird.

Im Bereich der Arbeitsstellung der Trennvorrichtung 11 weist der Behälter 149 eine innere Abmessung 123 auf, welche kleiner gegenüber der inneren Abmessung 14 im Bereich der Stirnseite 150 gewählt ist. Dadurch ist es beispielsweise möglich, bei gleichen äußeren Abmessungen für den Aufnahmebehälter 5, z.B. unterschiedliche Größen für den Aufnahmeraum 117 im Bereich des Behälters 149 zu schaffen, wobei gleichzeitig auch noch durch die Wahl der Abstimmung der inneren Abmessungen 14, 123 bzw. 18 zueinander die Lage der Arbeitsstellung der Trennvorrichtung 11 relativ zum Aufnahmebehälter 5 festlegbar ist. Die Ausbildung der einander zugewandten äußeren bzw. inneren Oberfläche des Aufnahmebehälters 5 bzw. Behälters 149 sowie die Wahl der Werkstoffe kann gemäß der EP 0 735 921 B1, der AT 402 365 B bzw. der US 5,871,700 A entsprechend gewählt werden.

In der Fig. 31 ist eine weitere mögliche und gegebenenfalls für sich eigenständige Ausbildung der Trennvorrichtung 11 gezeigt, welche aus den Grundkörpern 121 gebildet ist. Um unnötige Wiederholungen zu vermeiden wird auf die Beschreibung in den vorangegangen Fig. 1 bis 30 hingewiesen bzw. Bezug genommen, sowie für gleiche Teile gleiche Bezugszeichen verwendet.

Der Strömungskanal 119 ist auch bei diesem Ausführungsbeispiel zwischen einander zugewandten Bereichen der Grundkörper 121 ausgebildet, wobei im ersten Endbereich 45 wiederum die Dichtungsvorrichtung 120 mit der Dichtlippe 130 bevorzugt umlaufend über den Umfang der einzelnen Grundkörper 121 angeordnet ist. Die Ausbildung der Leitfläche 134 im ersten Endbereich 45 kann gemäß den in den Fig. 20 bis 29 beschriebenen Ausführungsformen erfolgen.

Die beiden Grundkörper 121 sind in einem Endbereich des Strömungskanals 119 durch ein Scharniergelenk 151 miteinander schwenkbar verbunden, wobei dieses Scharniergelenk 151 beispielsweise gleichzeitig auch eines der Stützelemente 132 ausbilden kann. Weiters sind über den Umfang verteilt noch weitere Stützelemente 132 im Bereich des Außenumfangs vereinfacht dargestellt. Die Ausbildung der Stützelemente 132 sowie die Teilung über den Umfang kann frei nach den gegebenen Anforderungen gewählt werden.

Das Scharniergelenk 151 kann zusätzlich auch noch gleichzeitig das Druckelement 122 ausbilden, wodurch die Grundkörper 121 zur Bildung des Strömungskanals 119 während deren Einsatz innerhalb der Aufnahmeeinrichtung 1 stets an die Innenwandung 118 angedrückt werden.

Zusätzlich ist es aber auch noch möglich indem dem Scharniergelenk 151 gegenüberliegenden Endbereich des Strömungskanals 119 ein oder mehrere zusätzliche Druckelemente 122 anzuordnen, wie dies in strichlierten Linien angedeutet ist. Dadurch wird eine zusätzliche gerichtete Kraft auf die einander zugewandten Grundkörper 121 ausgeübt und der Strömungskanal 119 in der Ausgangsstellung bis zum Erreichen der dichtenden Arbeitsstellung für ein Hindurchströmen offen gehalten.

Das Scharniergelenk 151 kann aus einem zum Grundkörper 121 gleichartigen aber auch davon unterschiedlichen Werkstoff gebildet sein. Bevorzugt wird dieses Scharniergelenk 151 im gleichen Arbeitsgang mit der Herstellung der Grundkörper 121 gefertigt, wodurch nachfolgende Fügevorgänge für den Zusammenbau der Trennvorrichtung 11 eingespart werden können. Auch ist dadurch der Montageaufwand für das Einsetzen der Trennvorrichtung 11 in die Aufnahmeeinrichtung 1 geringer, da die Trennvorrichtung 11 zwar mehrere Grundkörper aufweisen kann, jedoch als ein einzigen Stück in den Aufnahmeraum 117 eingesetzt werden kann.

Wesentlich ist bei den zuletzt beschriebenen Ausbildungen gemäß der Fig. 20 bis 31, daß eine Verjüngung des Aufnahmebehälters 5 bzw. des Behälters 149 in seinem Innenraum 10 bzw. Aufnahmeraum 117 zwischen den beiden Ebenen 16, 17 in etwa zwischen 0,1 ° und 3,0°, bevorzugt zwischen 0,6° und 0,8°, beträgt. Dabei sind auch noch Abweichungen von plus/minus 10% möglich. Der Aufnahmebehälter 5 und/oder der Behälter 149 und/oder der Grundkörper 121 und/oder die Dichtungsvorrichtung 120 bzw. Dichtungsanordnung 129 und/oder das Druckelement 122 können aus einem flüssigkeitsdichten, insbesondere wasserdichten sowie gegebenenfalls gasdichten Kunststoff gebildet sein. Dieser Kunststoff ist aus der Gruppe von Polyethylenterephthalat (PET), Polypropylen (PP), Polyethylen (PE), Polystyrol (PS), High-Density-Polyethylen (PE-HD), Acrylnitril-Butadien-Styrol-Copolymere (ABS), Thermoplastische Elastomere (TPE), Thermoplastisches Polyurethan (TPU), Ultrahochmolekulares Polyethylen mit sehr hoher molarer Masse (PE-UHMW), Polycarbonat (PC), Polyamid (PA) Polyoxymethylen (POM), Silikonkautschuk, Pharmagummi, Brombutylkautschuk, Gummi, ein Gel bzw. einer Kombination daraus gewählt.

Der oder die den Grundkörper bildenden Grundkörper 121 sind bevorzugt aus der Gruppe der Werkstoffe PE-UHMW, PC, PA, POM oder anderen thermoplastischen Kunststoffen gewählt. Das Druckelement 122 kann aus dem weicheren Material, wie z.B. für die Dichtungsvorrichtung 120 oder Dichtungsanordnung 129 angegeben, oder auch aus dem gleichen Material wie für den Grundkörper bzw. dessen Grundkörper 121 gebildet sein. Gleichfalls können aber auch jene Werkstoffe Anwendung finden, wie diese in den Fig. 1 bis 19 beschrieben worden sind.

Weiters kann der Grundkörper 121 bzw. auch nur Teilbereiche desselben zumindest bereichsweise mit einer Beschichtung versehen sein, wobei die Beschichtung z.B. durch eine Silikonschicht gebildet ist. Der Innenraum 10 bzw. der Aufnahmeraum 117 des Aufnahmebehälters 5 kann vor dem Aufsetzen und Verschließen durch die Verschlußvorrichtung 9 auf einen gegenüber dem atmosphärischen Druck geringeren Druck evakuiert werden.

Der Ordnung halber sei abschließend darauf hingewiesen, daß zum besseren Verständnis des Aufbaus des Aufnahmebehälters, der Verschlußvorrichtung sowie der Trennvorrichtung 11 diese bzw. deren Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

### Bezugszeichenaufstellung

- 1: Aufnahmeeinrichtung
- 2: Gemisch
- 3: Medium
- 4: Medium
- 5: Aufnahmebehälter

- 6: Ende
- 7: Ende
- 8: Stirnwand
- 9: Verschlußvorrichtung
- 10: Innenraum

- 11: Trennvorrichtung
- 12: Behälterwand
- 13: Wandstärke
- 14: Abmessung
- 15: Längsachse

- 16: Ebene
- 17: Ebene
- 18: Abmessung
- 19: Stirnseite
- 20: Kappe

- 21: Dichtungsvorrichtung
- 22: Dichtstopfen
- 23: Kappenmantel
- 24: Kupplungsteil
- 25: Kupplungsteil

- 26: Kupplungsteil
- 27: Kupplungsteil
- 28: Kupplungsvorrichtung
- 29: Fortsatz
- 30: Fortsatz

- 31: Haltering
- 32: Ansatz
- 33: Dichtfläche
- 34: Dichtfläche
- 35: Vertiefung

- 36: Öffnung
- 37: Führungsfortsatz
- 38: Führungsfortsatz
- 39: Führungssteg
- 40: Führungssteg

- 41: Grundkörper
- 42: Anlagefläche
- 43: Beschichtung
- 44: Verbindungsöffnung
- 45: Endbereich

- 46: Endbereich
- 47: Ausnehmung
- 48: Einsatzteil
- 49: Distanz
- 50: Ebene
- 50a: Ebene

- 51: Abmessung
- 52: Abmessung
- 53: Kegelwinkel
- 54: Spalt
- 55: Bogenlänge

- 56: Spaltfläche
- 57: Spaltfläche
- 58: Weite
- 59: Tiefe
- 60: Rückhaltevorrichtung

- 61: Steg
- 62: Länge
- 63: Vertiefung
- 64: Mantelteil
- 65: Durchbruch

- 66: Wandteil
- 67: Rippe
- 68: Nut
- 69: Verstellweg
- 70: Abmessung

- 71: Ebene
- 72: Abmessung
- 73: Oberfläche
- 74: Außenfläche
- 75: Dichtungsvorrichtung

- 76: Dichtlippe
- 77: Abmessung
- 78: Abmessung
- 79: Tragkörper
- 80: Rastelement

- 81: Rückhaltevorrichtung
- 82: Ansatz
- 83: Anlagefläche
- 84: Dichtfläche
- 85: Hüllkurve

- 86: Weite
- 87: Abmessung
- 88: Bundfläche
- 89: Vertiefung
- 90: Breite

- 91: Dicke
- 92: Ansatzteil
- 93: Anschlagring
- 94: Bauteil
- 95: Ausnehmung

- 96: Ausnehmung
- 97: Wandstärke
- 98: Begrenzungsfläche
- 99: Rückhaltevorrichtung
- 100: Rastelement

- 101: Weite
- 102: Abmessung
- 103: Überstand
- 104: Durchströmkanal
- 105: Durchmesser

- 106: Anlagefläche
- 107: Führungselement
- 108: Überstand
- 109: Höhe
- 110: Öffnungsweite

- 111: Abmessung
- 112: Ausnehmung
- 113: Ausnehmung
- 114: Gesamtlänge
- 115: Teillänge

- 116: Teillänge
- 117: Aufnahmeraum
- 118: Innenwandung
- 119: Strömungskanal
- 120: Dichtungsvorrichtung

- 121: Grundkörper
- 122: Druckelement
- 123: Abmessung
- 124: Abmessung
- 125: Symmetrieebene

- 126: Federsteg
- 127: Ausnehmung
- 128: Dichtfläche
- 129: Dichtungsanordnung
- 130: Dichtlippe

- 131: Außenfläche
- 132: Stützelement
- 133: Kegelabschnitt
- 134: Leitfläche
- 135: Anströmfläche

- 136: Tragkörper
- 137: Rückhaltevorrichtung
- 138: Ansatz
- 139: Steg
- 140: Positioniervorrichtung

- 141: Anschlagfläche
- 142: Hohlzylindersegment
- 143: Stirnwandteil
- 144: Haltesteg
- 145: Verbindungsteil

- 146: Führungsteil
- 147: Aufnahmeöffnung
- 148: Rückhaltefortsatz
- 149: Behälter
- 150: Stirnseite

- 151: Scharniergelenk

## Patentansprüche

1. Trennvorrichtung (11) zum Einsetzen in einen Innenraum (10) eines Aufnahmebehälters (5) einer Aufnahmeeinrichtung (1) für Körperflüssigkeiten, Gewebeteile bzw. Gewebekulturen, welche mindestens zwei Grundkörper (121) mit einer einer Innenwandung (118) des Aufnahmebehälters (5) zuwendbaren Dichtungsvorrichtung (120) sowie in Richtung einer Längsachse (15) voneinander distanzierte Endbereiche (45, 46) aufweist, zwischen welchen sich zumindest ein verschließbarer Strömungskanal (119) erstreckt, **dadurch gekennzeichnet, daß** die Grundkörper (121) durch mindestens ein Druckelement (122), wie z.B. Federstege, Spiralfedern, bereichsweise an die Innenwandung (118) des Aufnahmebehälters (5) andrückbar sind und in einer Ausgangsstellung der Strömungskanal (119) zwischen den benachbart angeordneten und durch das Druckelement (122) voneinander distanzierten Grundkörpern (121) ausgebildet ist, und in einer Arbeitsstellung, bei der eine Abmessung (124) des Innenraums (10) des Aufnahmebehälters (5) kleiner dem äußeren Umfang einer Hüll-Linie der Trennvorrichtung (11) in der gleichen Stellung ist, die Grundkörper (121) den Strömungskanal (119) verschließen.

2. Trennvorrichtung nach Anspruch 1, **dadurch gekennzeichnet, daß** diese eine Dichte von größer 1,05 g/cm³ aufweist.

3. Trennvorrichtung nach Anspruch 2, **dadurch gekennzeichnet, daß** die Dichte zwischen 1,5 g/cm³ und 3,5 g/cm³, bevorzugt zwischen 2,0 g/cm³ und 2,5 g/cm³, beträgt.

4. Trennvorrichtung nach einem der vorhergehenden Ansprüche, **dadurch gekennzeichnet, daß** diese mehrere, bevorzugt zwei Grundkörper (121) umfaßt, die während ihrer gesamten Verstellbewegung gegenüber dem Aufnahmebehälter (5) die gleiche relative Verstellgeschwindigkeit aufweisen.

5. Trennvorrichtung nach einem der vorhergehenden Ansprüche, **dadurch gekennzeichnet, daß** die Grundkörper (121) in einer senkrecht zur Längsachse (15) ausgerichteten Ebene relativ zueinander verstellbar sind.

6. Trennvorrichtung nach Anspruch 1, **dadurch gekennzeichnet, daß** das Druckelement (122) zwischen den einander zugewandten Bereichen der Grundkörper (121) angeordnet ist.

7. Trennvorrichtung nach einem der vorhergehenden Ansprüche, **dadurch gekennzeichnet, daß** die Grundkörper (121) über das Druckelement (122) zueinander in ihrer relativen Lage positioniert gehalten sind.

8. Trennvorrichtung nach einem der vorhergehenden Ansprüche, **dadurch gekennzeichnet, daß** die Grundkörper (121) über das oder die Druckelemente (122) miteinander bewegungsverbunden sind.

9. Trennvorrichtung nach einem der vorhergehenden Ansprüche, **dadurch gekennzeichnet, daß** das oder die Druckelemente (122) symmetrisch zur Längsachse (15) angeordnet sind.

10. Trennvorrichtung nach einem der vorhergehenden Ansprüche, **dadurch gekennzeichnet, daß** das Druckelement (122) durch in Richtung der Längsachse (15) gesehen V-förmig und in Richtung zur Längsachse (15) zusammenlaufende Federstege (126) gebildet ist.

11. Trennvorrichtung nach einem der Ansprüche 1 bis 9, **dadurch gekennzeichnet, daß** das Druckelement (122) durch in Richtung der Längsachse (15) gesehen V-förmig und auf die von der Längsachse (15) abgewandte Richtung zusammenlaufende Federstege (126) gebildet ist.

12. Trennvorrichtung nach Anspruch 11, **dadurch gekennzeichnet, daß** das Druckelement (122) in Richtung der Längsachse (15) gesehen jeweils durch zwei V-förmig und auf die von der Längsachse (15) abgewandte Richtung zusammenlaufende Federstege (126) gebildet ist und die weiteren einander zugewandten Enden der Federstege (126) miteinander verbunden sind.

13. Trennvorrichtung nach einem der Ansprüche 1 bis 9, **dadurch gekennzeichnet, daß** das Druckelement (122) in Richtung der Längsachse (15) gesehen und in einer in etwa senkrecht zum Strömungskanal (119) ausgerichteten Ebene durch jeweils gegengleich gekrümmte Federstege (126) gebildet ist, die an ihren einander zugewandten Endbereichen gegebenenfalls unter Anordnung eines kreisförmig ausgebildeten Verbindungsteils (145) miteinander verbunden sind.

14. Trennvorrichtung nach einem der vorhergehenden Ansprüche, **dadurch gekennzeichnet, daß** die Grundkörper (121) und das oder die Druckelemente (122) aus einem zueinander gleichartigen Werkstoff gebildet sind.

15. Trennvorrichtung nach einem der vorhergehenden Ansprüche, **dadurch gekennzeichnet, daß** zur Aufnahme des Druckelements (122) in zumindest einem der einander zugewandten Bereiche der Grundkörper (121) eine entsprechende Ausnehmung (127) vorgesehen ist.

16. Trennvorrichtung nach einem der vorhergehenden Ansprüche, **dadurch gekennzeichnet, daß** zwischen den Grundkörpern (121) im Bereich des einem ersten Endes (6) des Aufnahmebehälters (5) zuwendbaren Endbereiches (45) eine Dichtungsanordnung (129) zur Abdichtung des oder der Strömungskanäle (119) vorgesehen ist.

17. Trennvorrichtung nach einem der vorhergehenden Ansprüche, **dadurch gekennzeichnet, daß** die der Innenwandung (118) zuwendbare Dichtungsvorrichtung (120) im Bereich des dem ersten Ende (6) des Aufnahmebehälters (5) zuwendbaren Endbereiches (45) angeordnet ist.

18. Trennvorrichtung nach einem der vorhergehenden Ansprüche, **dadurch gekennzeichnet, daß** die Dichtungsvorrichtung (120) durch zumindest eine über den Umfang des Grundkörpers (121) durchlaufende Dichtlippe (130) gebildet ist.

19. Trennvorrichtung nach Anspruch 18, **dadurch gekennzeichnet, daß** die Dichtlippe (130) den Grundkörper (121) radial nach außen in die von der Längsachse (15) abgewandte Richtung überragt.

20. Trennvorrichtung nach Anspruch 18 oder 19, **dadurch gekennzeichnet, daß** die dem Strömungskanal (119) benachbarten Abschnitte der Dichtlippen (130) sich zumindest in der Arbeitsstellung derselben einander übergreifen bzw. überlappen.

21. Trennvorrichtung nach einem der vorhergehenden Ansprüche, **dadurch gekennzeichnet, daß** die Dichtungsvorrichtung (120) und/oder die Dichtungsanordnung (129) flüssigkeitsdicht ausgebildet ist.

22. Trennvorrichtung nach einem der vorhergehenden Ansprüche, **dadurch gekennzeichnet, daß** die Grundkörper (121) in einem der Innenwandung (118) des Aufnahmebehälters (5) zuordenbaren Bereiches durch einen Abschnitt eines Hohlzylinders bzw. Hohlkegelstumpfes ausgebildet sind.

23. Trennvorrichtung nach einem der vorhergehenden Ansprüche, **dadurch gekennzeichnet, daß** an den Grundkörper (121) mehrere über den äußeren Umfang derselben und eine Außenfläche (131) in die von der Längsachse (15) abgewandte Richtung überragende Stützelemente (132) angeordnet sind.

24. Trennvorrichtung nach Anspruch 23, **dadurch gekennzeichnet, daß** die Stützelemente (132) durch parallel zur Längsachse (15) ausgerichtete Stege gebildet sind.

25. Trennvorrichtung nach einem der vorhergehenden Ansprüche, **dadurch gekennzeichnet, daß** die Grundkörper (121) im Bereich des dem ersten Ende (6) des Aufnahmebehälters (5) zuwendbaren ersten Endbereiches (45) eine bevorzugt aus Kegelabschnitten (133) gebildete und in Richtung zur Längsachse (15) sowie hin zum weiteren Endbereich (46) verjüngend ausgebildete Leitfläche (134) aufweisen.

26. Trennvorrichtung nach einem der vorhergehenden Ansprüche, **dadurch gekennzeichnet, daß** die Grundkörper (121) im Bereich des dem weiteren Ende (7) des Aufnahmebehälters (5) zuwendbaren zweiten Endbereiches (46) eine in Richtung zur Längsachse (15) sowie hin zum ersten Endbereich (45) geneigt verlaufende Anströmfläche (135) aufweisen.

27. Trennvorrichtung nach einem der vorhergehenden Ansprüche, **dadurch gekennzeichnet, daß** zwischen den einander zugewandten Bereichen der Grundkörper (121) zumindest ein vorragender Führungsteil (146) an einem der Grundkörper (121) und im anderen Grundkörper (121) eine damit zusammenwirkende Aufnahmeöffnung (147) für den Führungsteil (146) angeordnet ist.

28. Trennvorrichtung nach Anspruch 27, **dadurch gekennzeichnet, daß** am Führungsteil (146) ein mit der Aufnahmeöffnung (147) zusammenwirkender Rückhaltefortsatz (148) angeordnet ist.

29. Trennvorrichtung nach einem der vorhergehenden Ansprüche, **dadurch gekennzeichnet, daß** die Grundkörper (121) durch ein im Randbereich derselben angeordnetes und den Strömungskanal (119) übergreifendes Scharniergelenk (151) miteinander schwenkbar verbunden sind.

30. Trennvorrichtung nach Anspruch 29, **dadurch gekennzeichnet, daß** das Scharniergelenk (151) das Druckelement (122) bildet.

31. Trennvorrichtung nach Anspruch 29 oder 30, **dadurch gekennzeichnet, daß** das Scharniergelenk (151) ein Stützelement (132) bildet.

32. Trennvorrichtung nach einem der vorhergehenden Ansprüche, **dadurch gekennzeichnet, daß** die Grundkörper (121) jeweils durch einen Tragkörper (136) und die daran angeordnete Dichtungsvorrichtung (120) und/oder Dichtungsanordnung (129) gebildet sind.

33. Trennvorrichtung nach Anspruch 32, **dadurch gekennzeichnet, daß** der Tragkörper (136) gegenüber der Dichtungsvorrichtung (120) bzw. der Dichtungsanordnung (129) eine höhere Dichte sowie einen höheren Elastizitätsmodul aufweist.

34. Trennvorrichtung nach einem der vorhergehenden Ansprüche, **dadurch gekennzeichnet, daß** der Grundkörper (121) zumindest bereichsweise mit einer Beschichtung versehen ist.

35. Trennvorrichtung nach Anspruch 34, **dadurch gekennzeichnet, daß** die Beschichtung durch eine Silikonschicht gebildet ist.

36. Aufnahmeeinrichtung (1) mit einem Aufnahmebehälter (5), der einen Innenraum (10) mit einer Innenwandung (118) umgrenzt, sowie zwei in Richtung einer Längsachse (15) voneinander distanzierte Enden (6, 7) aufweist, von denen zumindest eines mit einer Öffnung ausgebildet ist, und im Bereich der Innenwandung (118) eine innere Abmessung (14) des Aufnahmebehälters (5) im Bereich des ersten offenen Endes (6) in einer senkrecht zur Längsachse (15) ausgerichteten Ebene (16) größer einer inneren Abmessung (18) im Bereich des weiteren Endes (7) in einer dazu parallelen Ebene (17) in der gleichen Raumrichtung ist, mit zumindest einer öffenbaren Verschlußvorrichtung (9) für das offene Ende (6) des Aufnahmebehälters (5), mit einer in den Innenraum (10) eingesetzten Trennvorrichtung (11), wobei diese von einer sich im Bereich der Verschlußvorrichtung (9) befindlichen Ausgangsstellung in eine davon in Richtung des weiteren Endes (7) distanzierte Arbeitsstellung verlagerbar ist, **dadurch gekennzeichnet, daß** die Trennvorrichtung (11) nach einem der Ansprüche 1 bis 35 ausgebildet ist und mindestens zwei Grundkörper (121) umfaßt, die durch mindestens ein Druckelement (122) bereichsweise an die Innenwandung (118) des Aufnahmebehälters (5) angedrückt sind, und daß die innere Abmessung (14) bzw. ein innerer Umfang einer Hüll-Linie des Innenraums (10) in der ersten Ebene (16) größer einer äußeren Abmessung (123) bzw. einem äußeren Umfang einer Hüll-Linie der Trennvorrichtung (11) in ihrer Arbeitsstellung und der gleichen Raumrichtung ist, und in der Ausgangsstellung ein Strömungskanal (119) zwischen den Enden (6, 7) des Aufnahmebehälters (5) im Bereich der Trennvorrichtung (11) ausgebildet ist und eine innere Abmessung (124) bzw. ein innerer Umfang einer Hüll-Linie des Innenraums (10) im Bereich der Arbeitsstellung der Trennvorrichtung (11) kleiner dem äußeren Umfang einer Hüll-Linie der Trennvorrichtung (11) in der gleichen Stellung ist und die Grundkörper (121) der Trennvorrichtung (11) in der Arbeitsstellung den Strömungskanal (119) verschließen.

37. Aufnahmeeinrichtung nach Anspruch 36, **dadurch gekennzeichnet, daß** im Bereich der Ausgangsstellung eine Rückhaltevorrichtung (137) für die in den Innenraum (10) eingesetzte Trennvorrichtung (11) angeordnet ist.

38. Aufnahmeeinrichtung nach Anspruch 37, **dadurch gekennzeichnet, daß** die Rückhaltevorrichtung (137) durch zumindest einen über den Umfang der Innenwandung (118) in Richtung zur Längsachse (15) vorragenden Ansatz (138) gebildet ist.

39. Aufnahmeeinrichtung nach Anspruch 37 oder 38, **dadurch gekennzeichnet, daß** die Rückhaltevorrichtung (137) durch einen zumindest bereichsweise über den Umfang der Innenwandung (118) in Richtung zur Längsachse (15) vorragenden Steg (139) gebildet ist.

40. Aufnahmeeinrichtung nach Anspruch 39, **dadurch gekennzeichnet, daß** der Steg (139) durchlaufend über den Umfang der Innenwandung (118) angeordnet ist.

41. Aufnahmeeinrichtung nach einem der Ansprüche 37 bis 40, **dadurch gekennzeichnet, daß** die Rückhaltevorrichtung (137) durch eine Verkleinerung der inneren Abmessung (14) des Innenraums (10) gebildet ist.

42. Aufnahmeeinrichtung nach einem der Ansprüche 37 bis 41, **dadurch gekennzeichnet, daß** die Rückhaltevorrichtung (137) durch eine über den Umfang der Innenwandung (118) durchlaufend ausgebildete nutförmige Vertiefung gebildet ist.

43. Aufnahmeeinrichtung nach einem der Ansprüche 36 bis 42, **dadurch gekennzeichnet, daß** im Bereich der Arbeitsstellung zwischen dem Aufnahmebehälter (5) und der Trennvorrichtung (11) eine Positioniervorrichtung (140) angeordnet ist.

44. Aufnahmeeinrichtung nach Anspruch 43, **dadurch gekennzeichnet, daß** die Positioniervorrichtung (140) durch eine Verkleinerung der inneren Abmessung (124) des Innenraums (10) und unter Bildung einer in etwa in einer senkrecht zur Längsachse (15) ausgerichteten Anschlagfläche (141) gebildet ist.

45. Aufnahmeeinrichtung nach einem der Ansprüche 36 bis 44, **dadurch gekennzeichnet, daß** eine Verjüngung des Aufnahmebehälters (5) in seinem Innenraum (10) zwischen den beiden Ebenen (16, 17) zwischen 0,1° und 3,0°, bevorzugt zwischen 0,6° und 0,8°, beträgt.

46. Aufnahmeeinrichtung nach einem der Ansprüche 36 bis 45, **dadurch gekennzeichnet, daß** der Aufnahmebehälter (5) und/oder der Behälter (149) und/oder der Grundkörper (121) und/oder die Dichtungsvorrichtung (120) bzw. Dichtungsanordnung (129) und/oder das Druckelement (122) aus einem flüssigkeitsdichten sowie gegebenenfalls gasdichten Kunststoff gebildet ist bzw. sind.

47. Aufnahmeeinrichtung nach Anspruch 46, **dadurch gekennzeichnet, daß** der Kunststoff aus der Gruppe von Polyethylenterephthalat (PET), Polypropylen (PP), Polyethylen (PE), Polystyrol (PS), High-Density-Polyethylen (PE-HD), Acrylnitril-Butadien-Styrol-Copolymere (ABS), Thermoplastische Elastomere (TPE), Thermoplastisches Polyurethan (TPU), Ultrahochmolekulares Polyethylen mit sehr hoher molarer Masse (PE-UHMW), Polycarbonat (PC), Polyamid (PA) Polyoxymethylen (POM), Silikonkautschuk, Pharmagummi, Brombutylkautschuk, Gummi, ein Gel bzw. einer Kombination daraus gewählt ist.

48. Aufnahmeeinrichtung nach einem der Ansprüche 36 bis 47, **dadurch gekennzeichnet, daß** der Innenraum (10) des Aufnahmebehälters (5) auf einen gegenüber dem atmosphärischen Druck geringeren Druck evakuiert ist.

49. Trennvorrichtung (11) zum Einsetzen in einen Innenraum (10) eines Aufnahmebehälters (5) einer Aufnahmeeinrichtung (1) für Körperflüssigkeiten, Gewebeteile bzw. Gewebekulturen, welche einen elastisch verformbaren Grundkörper (41) mit einer einer Innenwandung (118) des Aufnahmebehälters (5) zuwendbaren Anlagefläche (42) sowie in Richtung einer Längsachse (15) voneinander distanzierte Endbereiche (45, 46) aufweist, zwischen welchen sich zumindest ein verschließbarer Strömungskanal (119) sowie die Anlagefläche (42) erstrecken, **dadurch gekennzeichnet, daß** in einer Ausgangsstellung im Grundkörper (41) ein sich zwischen den beiden Endbereichen (45, 46) erstreckender und ausgehend von seinem Zentrum hin zu der Anlagefläche (42) sich erweiternder Spalt (54) angeordnet ist und in einer Arbeitsstellung, bei der eine innere Abmessung des Innenraums (10) des Aufnahmebehälters (5) kleiner dem äußeren Umfang einer Hüll-Linie der Trennvorrichtung (11) in der gleichen Stellung ist, der Strömungskanal (119) in zumindest einer selbsttätig wirkenden Ventilanordnung verschließbar ist, und die beiden einander zugewandten Spaltflächen des Spaltes (54) zur Anlage aneinander gebracht werden.

50. Trennvorrichtung nach Anspruch 49, **dadurch gekennzeichnet, daß** der sich erweiternde Spalt (54) keilförmig ausgebildet ist.

51. Trennvorrichtung nach Anspruch 49 oder 50, **dadurch gekennzeichnet, daß** der Grundkörper (41) in einer senkrecht zur Längsachse (15) ausgerichteten Ebene (50) in seinem ersten Endbereich (45) eine größere äußere Abmessung (51) aufweist als in seinem weiteren Endbereich (46).

52. Trennvorrichtung nach einem der Ansprüchen 49 bis 51, **dadurch gekennzeichnet, daß** der Grundkörper (41) als Kegelstumpf ausgebildet ist, welcher in seinem ersten Endbereich (45) in der senkrecht zur Längsachse (15) ausgerichteten Ebene (50) die größere äußere Abmessung (51) aufweist.

53. Trennvorrichtung nach Anspruch 52, **dadurch gekennzeichnet, daß** der Kegelstumpf einen Kegelwinkel (53) zwischen 0,1° und 3,0°, bevorzugt zwischen 0,6° und 0,8°, aufweist.

54. Trennvorrichtung nach einem der Ansprüche 49 bis 53, **dadurch gekennzeichnet, daß** in der Arbeitsstellung der Trennvorrichtung (11) einander zugewandte Spaltflächen (56, 57) des Spaltes (54) dichtend aneinander liegen.

55. Trennvorrichtung nach einem der Ansprüche 49 bis 54, **dadurch gekennzeichnet, daß** der Grundkörper (41) im seinem ersten Endbereich (45) eine konkav ausgebildete Ausnehmung (47) aufweist, welche in ihrer Form einem diesen zuwendbaren Teil einer Verschlußvorrichtung (9) nahezu angepaßt ist.

56. Trennvorrichtung nach einem der Ansprüche 49 bis 55, **dadurch gekennzeichnet, daß** der Grundkörper (41) eine im Bereich des Zentrums angeordnete und sich in Richtung der Längsachse (15) und zwischen den Endbereichen (45, 46) erstreckende Verbindungsöffnung (44) aufweist.

57. Trennvorrichtung nach Anspruch 56, **dadurch gekennzeichnet, daß** die Verbindungsöffnung (44) ausgehend vom ersten Endbereich (45) in Richtung des weiteren Endbereiches (46) sich erweiternd ausgebildet ist.

58. Trennvorrichtung nach Anspruch 57, **dadurch gekennzeichnet, daß** die Verbindungsöffnung kegelstumpfförmig ausgebildet ist.

59. Trennvorrichtung nach Anspruch 57 oder 58, **dadurch gekennzeichnet, daß** der sich erweiternde Bereich der Verbindungsöffnung (44) nur über einen Teilbereich einer Distanz (49) zwischen den beiden Endbereichen (45, 46) erstreckt.

60. Trennvorrichtung nach einem der Ansprüche 56 bis 59, **dadurch gekennzeichnet, daß** in den erweiternden Abschnitt der Verbindungsöffnung (44) ein Einsatzteil (48) eingebracht ist.

61. Trennvorrichtung nach einem der Ansprüche 56 bis 60, **dadurch gekennzeichnet, daß** die Verbindungsöffnung (44) im ersten Endbereich (45) in der senkrecht zur Längsachse (15) ausgerichteten Ebene (50) in der Ausgangsstellung eine lichte Weite (58) aufweist, die gleich oder kleiner einer äußeren Abmessung des Einsatzteils (48) ist.

62. Trennvorrichtung nach einem der Ansprüche 56 bis 61, **dadurch gekennzeichnet, daß** am Grundkörper (41) im weiteren Endbereich (46) eine in die Verbindungsöffnung (44) ragende Rückhaltevorrichtung (60) für den Einsatzteil (48) angeordnet ist.

63. Trennvorrichtung nach einem der Ansprüche 56 bis 62, **dadurch gekennzeichnet, daß** die beiden Endbereiche (45, 46) des Grundkörpers (41) bei Anlage des Einsatzteils (48) an der Rückhaltevorrichtung (60) über die Verbindungsöffnung (44) in Strömungsverbindung stehen.

64. Trennvorrichtung nach einem der Ansprüche 56 bis 63, **dadurch gekennzeichnet, daß** der Einsatzteil (48) in der Arbeitsstellung dichtend an Begrenzungswänden des sich erweiternden Abschnitts der Verbindungsöffnung (44) anliegt.

65. Trennvorrichtung nach einem der Ansprüche 49 bis 64, **dadurch gekennzeichnet, daß** der Grundkörper (41) eine Dichte zwischen 1,02 g/cm³ und 1,07 g/cm³, bevorzugt zwischen 1,04g/cm³ und 1,05 g/cm³, aufweist.

66. Trennvorrichtung nach einem der Ansprüche 60 bis 64, **dadurch gekennzeichnet, daß** der Einsatzteil (48) eine Dichte zwischen 1,02 g/cm³ und 1,07 g/cm³, bevorzugt zwischen 1,04g/cm³ und 1,05 g/cm³, aufweist.

67. Trennvorrichtung nach Anspruch 60, **dadurch gekennzeichnet, daß** der Einsatzteil (48) als Kegelstumpf ausgebildet ist.

68. Trennvorrichtung nach Anspruch 60, **dadurch gekennzeichnet, daß** der Einsatzteil (48) als Kugel ausgebildet ist.

69. Aufnahmeeinrichtung (1) mit einem Aufnahmebehälter (5), der einen Innenraum (10) mit einer Innenwandung (118) umgrenzt, sowie zwei in Richtung einer Längsachse (15) voneinander distanzierte Enden (6, 7) aufweist, von denen zumindest eines mit einer Öffnung ausgebildet ist, und im Bereich der Innenwandung (118) eine innere Abmessung (14) des Aufnahmebehälters (5) im Bereich des ersten offenen Endes (6) in einer senkrecht zur Längsachse (15) ausgerichteten Ebene (16) größer einer inneren Abmessung (18) im Bereich des weiteren Endes (7) in einer dazu parallelen Ebene (17) in der gleichen Raumrichtung ist, mit zumindest einer öffenbaren Verschlußvorrichtung (9) für das offene Ende (6) des Aufnahmebehälters (5), mit einer in den Innenraum (10) eingesetzten Trennvorrichtung (11), wobei diese von einer sich im Bereich der Verschlußvorrichtung (9) befindlichen Ausgangsstellung in eine davon in Richtung des weiteren Endes (7) distanzierte Arbeitsstellung verlagerbar ist, **dadurch gekennzeichnet, daß** die Trennvorrichtung (11) nach einem der Ansprüche 49 bis 68 ausgebildet ist und daß die innere Abmessung (14) bzw. ein innerer Umfang einer Hüll-Linie des Aufnahmebehälters (5) in der ersten Ebene (16; 50) gleich oder kleiner einer äußeren Abmessung (51) bzw. einem äußeren Umfang einer Hüll-Linie des Grundkörpers (41) in seinem unverformten Zustand in der gleichen Ebene (16; 50) und der gleichen Raumrichtung ist, und daß die äußere Abmessung (51) bzw. der äußere Umfang der Hüll-Linie des Grundkörpers (41) in seiner Arbeitsstellung größer als die minimale innere Abmessung (18) im Bereich des weiteren Endes (7) in der Ebene (17) und der gleichen Raumrichtung ist.

70. Aufnahmeeinrichtung nach Anspruch 69, **dadurch gekennzeichnet, daß** eine Verjüngung des Aufnahmebehälters (5) in seinem Innenraum (10) zwischen den beiden Ebenen (16, 17) zwischen 0,1° und 3,0°, bevorzugt zwischen 0,6° und 0,8°, beträgt.

71. Aufnahmeeinrichtung nach Anspruch 69 oder 70, **dadurch gekennzeichnet, daß** ein Querschnitt des Aufnahmebehälters (5) in den Ebenen (16, 17, 71) kreisförmig ausgebildet ist.

72. Aufnahmeeinrichtung nach einem der Ansprüche 69 bis 71, **dadurch gekennzeichnet, daß** die äußere Abmessung (51) des Grundkörpers (41) in der senkrecht zur Längsachse (15) ausgerichteten Ebene (50) in seinem ersten Endbereich (45) im unverformten Zustand gleich oder größer der inneren Abmessung (14) des Aufnahmebehälters (5) in seinem ersten offenen Ende (6) in der Ebene (16) sowie der gleichen Raumrichtung ist.

73. Aufnahmeeinrichtung nach Anspruch 69, **dadurch gekennzeichnet, daß** der Kegelwinkel (53) des Kegelstumpfes der Verjüngung des Innenraums (10) des Aufnahmebehälters (5) zwischen den beiden Ebenen (16, 17) entspricht.

74. Aufnahmeeinrichtung nach einem der Ansprüche 69 bis 73, **dadurch gekennzeichnet, daß** eine Bogenlänge (55) des Spaltes (54) im Bereich der Anlagefläche (42) des Grundkörpers (41) in seiner Ausgangsstellung im Aufnahmebehälter (5) gleich der Umfangsdifferenz zwischen dem inneren Umfang des Aufnahmebehälters (5) in der senkrecht zur Längsachse (15) ausgerichteten Ebene (16) im Bereich der Ausgangsstellung und dem inneren Umfang des Aufnahmebehälters (5) im Bereich der Arbeitsstellung in der Ebene (71) ist.

75. Aufnahmeeinrichtung nach einem der Ansprüche 69 bis 74, **dadurch gekennzeichnet, daß** im Bereich der Arbeitsstellung zwischen dem Aufnahmebehälter (5) und der Trennvorrichtung (11) eine Positioniervorrichtung (140) angeordnet ist.

76. Aufnahmeeinrichtung nach Anspruch 75, **dadurch gekennzeichnet, daß** die Positioniervorrichtung durch eine Verkleinerung der inneren Abmessung (124) des Innenraums (10) und unter Bildung einer in etwa in einer senkrecht zur Längsachse (15) ausgerichteten Anschlagfläche (141) gebildet ist.

77. Aufnahmeeinrichtung nach einem der Ansprüche 69 bis 76, **dadurch gekennzeichnet, daß** der Aufnahmebehälter (5) und/oder der Grundkörper (41) und/oder der Einsatzteil (48) aus einem flüssigkeitsdichten sowie gegebenenfalls gasdichten Kunststoff gebildet ist bzw. sind.

78. Aufnahmeeinrichtung nach Anspruch 77, **dadurch gekennzeichnet, daß** der Kunststoff aus der Gruppe von Polyethylenterephthalat (PET), Polypropylen (PP), Polyethylen (PE), Polystyrol (PS), High-Density-Polyethylen (PE-HD), Acrylnitril-Butadien-Styrol-Copolymere (ABS) bzw. einer Kombination daraus gewählt ist.

79. Aufnahmeeinrichtung nach einem der Ansprüche 69 bis 78, **dadurch gekennzeichnet, daß** der Grundkörper (41) und/oder der Einsatzteil (48) zumindest bereichsweise mit einer Beschichtung versehen ist.

80. Aufnahmeeinrichtung nach Anspruch 79, **dadurch gekennzeichnet, daß** die Beschichtung durch eine Silikonschicht gebildet ist.

81. Aufnahmeeinrichtung nach einem der Ansprüche 69 bis 80, **dadurch gekennzeichnet, daß** der Innenraum (10) des Aufnahmebehälters (5) auf einen gegenüber dem atmosphärischen Druck geringeren Druck evakuiert ist.

## Claims

1. Separating device (11) for inserting in an interior (10) of a container (5) of a holding device (1) for body fluids, tissue parts or tissue cultures, having at least two base bodies (121) with a sealing device (120) which can be directed towards an internal wall (118) of the container (5) and having ends regions (45, 46) spaced at a distance apart from one another in the direction of a longitudinal axis (15) between which a flow passage (119) extends, **characterised in that** the base bodies (121) are provided in the form of at least one pressing element (122), such as resilient webs, coil springs for example, which can be applied against certain regions of the internal wall (118) of the container (5) and, in an initial position, the flow passage (119) is established between the adjacently disposed base bodies (121) spaced at a distance apart from one another by the pressing element (122), and in an operating position, in which a dimension (124) of the interior (10) of the container (5) is smaller than the external circumference of an envelope line of the separating device (11) in the same position, the base bodies (121) close the flow passage (119).

2. Separating device as claimed in claim 1, **characterised in that** it has a density greater than 1.05 g/cm³.

3. Separating device as claimed in claim 2, **characterised in that** the density is between 1.5 g/cm³ and 3.5 g/cm³, preferably between 2.0 g/cm³ and 2.5 g/cm³.

4. Separating device as claimed in one or more of the preceding claims, **characterised in that** it comprises several, preferably two, base bodies (121), which are displaced at the same relative speed during their entire displacement motion relative to the container (5).

5. Separating device as claimed in one or more of the preceding claims, **characterised in that** the base bodies (121) can be displaced relative to one another in a plane perpendicular to the longitudinal axis (15).

6. Separating device as claimed in claim 1, **characterised in that** the pressing element (122) is disposed between regions of the base bodies (121) directed towards one another.

7. Separating device as claimed in one of the preceding claims, **characterised in that** the base bodies (121) are held in their position relative to one another by the pressing element (122).

8. Separating device as claimed in one of the preceding claims, **characterised in that** the base bodies (121) are joined to one another in displacement via the pressing element or elements (122).

9. Separating device as claimed in one of the preceding claims, **characterised in that** the pressing element or elements (122) are disposed symmetrically relative to the longitudinal axis (15).

10. Separating device as claimed in one of the preceding claims, **characterised in that** the pressing element (122) has resilient webs (126) which are V-shaped as viewed in the direction of the longitudinal axis (15) and converge in the direction towards the longitudinal axis (15).

11. Separating device as claimed in one of claims 1 to 9, **characterised in that** the pressing element (122) has resilient webs (126) which are V-shaped as viewed in the direction of the longitudinal axis (15) and converge in the direction remote from the longitudinal axis (15).

12. Separating device as claimed in claim 11, **characterised in that** the pressing element (122) has two resilient webs (126), each of which is V-shaped as viewed in the direction of the longitudinal axis (15), and which converge in the direction remote from the longitudinal axis (15), and the other mutually facing ends of the resilient webs (126) are joined to one another.

13. Separating device as claimed in one of claims 1 to 9, **characterised in that** the pressing element (122) is provided in the form of respective complementary curved resilient webs (126) as viewed in the direction of the longitudinal axis (15), disposed in a plane substantially perpendicular to the flow passage (119), which are joined to one another at mutually facing end regions which may or may not be provided with a circular connecting part (145).

14. Separating device as claimed in one of the preceding claims, **characterised in that** the base bodies (121) and the pressing element or elements (122) are made from the same type of material.

15. Separating device as claimed in one of the preceding claims, **characterised in that** an appropriate recess (127) is provided in order to accommodate the pressing element (122) in at least one of the mutually facing regions of the base bodies (121).

16. Separating device as claimed in one of the preceding claims, **characterised in that** a sealing system (129) for sealing off the flow passage or passages (119) is provided between the base bodies (121) in the area of the end region (45) directed towards a first end (6) of the container (5).

17. Separating device as claimed in one of the preceding claims, **characterised in that** the sealing device (120) which can be directed towards the internal wall (118) is disposed in the area of the end region (45) which can be directed towards the first end (6) of the container (5).

18. Separating device as claimed in one of the preceding claims, **characterised in that** the sealing device (120) is provided in the form of at least one sealing lip (130) extending continuously around the periphery of the base body (121).

19. Separating device as claimed in claim 18, **characterised in that** the sealing lip (130) projects radially outwards beyond the base body (121) in the direction remote from the longitudinal axis (15).

20. Separating device as claimed in claim 18 or 19, **characterised in that** the sections of the sealing lips (130) adjacent to the flow passage (119) locate with one another or overlap at least when they are in the operating position.

21. Separating device as claimed in one of the preceding claims, **characterised in that** the sealing device (120) and/or the sealing system (129) are fluid-tight.

22. Separating device as claimed in one of the preceding claims, **characterised in that** the base bodies (121) are provided in the form of a section of a hollow cylinder or hollow truncated cone in a region associated with the internal wall (118) of the container (5).

23. Separating device as claimed in one of the preceding claims, **characterised in that** several support elements (132) are provided on the base bodies (121) projecting out from the external periphery and an external surface (131) thereof in the direction remote from the longitudinal axis (15).

24. Separating device as claimed in claim 23, **characterised in that** the support elements (132) are provided in the form of webs aligned parallel with the longitudinal axis (15).

25. Separating device as claimed in one of the preceding claims, **characterised in that** the base bodies (121) have a baffle surface (134) disposed in the area of the first end region (45) directed towards the first end (6) of the container (5), preferably provided in the form of cone portions (133), tapering in the direction towards the longitudinal axis (15) and as far as the other end region (46).

26. Separating device as claimed in one of the preceding claims, **characterised in that** the base bodies (121) have an inflow surface (135) in the area of the second end region (46) directed towards the other end (7) of the container (5), which extend in the direction towards the longitudinal axis (15) and are inclined relative to the first end region (45).

27. Separating device as claimed in one of the preceding claims, **characterised in that** at least one guide part (146) projects out from one of the base bodies (121) between mutually facing regions of the base bodies (121) and a seating orifice (147) co-operating therewith is provided on the other base body (121) for the guide part (146).

28. Separating device as claimed in claim 27, **characterised in that** a retaining projection (148) co-operating with the seating orifice (147) is provided on the guide part (146).

29. Separating device as claimed in one of the preceding claims, **characterised in that** the base bodies (121) are pivotably linked to one another by a hinge joint (151) engaging around the peripheral region thereof and the flow passage (119).

30. Separating device as claimed in claim 29, **characterised in that** the hinge joint (151) forms the pressing element (122).

31. Separating device as claimed in claim 29 or 30, **characterised in that** the hinge joint (151) forms a support element (132).

32. Separating device as claimed in one of the preceding claims, **characterised in that** the base bodies (121) are respectively provided in the form of a support body (136) and the sealing device (120) and/or sealing system (129) disposed thereon.

33. Separating device as claimed in claim 32, **characterised in that** the support body (136) has a higher density and a higher modulus of elasticity than the sealing device (120) and the sealing system (129).

34. Separating device as claimed in one of the preceding claims, **characterised in that that** at least certain regions of the base bodies (121) are provided with a coating.

35. Separating device as claimed in claim 34, **characterised in that** the coating is a layer of silicone.

36. Holding device (1) with a container (5) which bounds an interior (10) with an internal wall (118) and having two ends (6, 7) spaced at a distance apart in the direction of the longitudinal axis (15), at least one of which has an orifice, and in the region of the internal wall (118), an internal dimension (14) of the container (5) in the region of the first open end (6) in a plane (16) perpendicular to the longitudinal axis (15) is bigger than an internal dimension (18) in the region of the other end (7) in a plane (17) parallel therewith in the same spatial direction, having at least one closing device (9) for the open end (6) of the container (5), with a separating device (11) inserted in the interior (10), this closing device (9) being displaceable from an initial position to an operating position spaced apart in the direction towards the other end (7), **characterised in that** the separating device (11) is designed as claimed in one of claims 1 to 35 and has at least two base bodies (121) which are applied by at least one pressing element (122) against certain regions of the internal wall (118) of the container (5), and the internal dimension (14) and an internal periphery of an envelope line of the interior (10) in the first plane (16) is bigger than an external dimension (123) and an external periphery of an envelope line of the separating device (11) in its operating position in the same spatial direction, and a flow passage (119) is established between the ends (6, 7) of the container (5) in the region of the separating device (11) in the initial position, and an internal dimension (124) and an internal periphery of an envelope line of the interior (10) in the region of the operating position of the separating device (11) is smaller than the external periphery of an envelope line of the separating device (11) in the same position, and the base bodies (121) of the separating device (11) automatically seal off the flow passage (119) in the operating position.

37. Holding device as claimed in claim 36, **characterised in that** a retaining mechanism (137) is provided for the separating device (11) inserted in the interior (10).

38. Holding device as claimed in claim 37, **characterised in that** the retaining mechanism (137) is provided in the form of a shoulder (138) projecting out from the periphery of the internal wall (118) in the direction towards the longitudinal axis (15).

39. Holding device as claimed in claim 37 or 38, **characterised in that** the retaining mechanism (137) is provided in the form of a web (139) projecting out from at least certain regions of the periphery of the internal wall (118) in the direction towards the longitudinal axis (15).

40. Holding device as claimed in claim 39, **characterised in that** the web (139) extends continuously around the periphery of the internal wall (118).

41. Holding device as claimed in one of claims 37 to 40, **characterised in that** the retaining mechanism (137) is provided in the form of a reduction in the internal dimension (14) of the interior (10).

42. Holding device as claimed in one of claims 37 to 41, **characterised in that** the retaining mechanism (137) is provided in the form of a groove-shaped recess extending continuously around the periphery of the internal wall (118).

43. Holding device as claimed in one of claims 36 to 42, **characterised in that** a positioning mechanism (140) is provided between the container (5) and the separating device (11) in the region of the operating position.

44. Holding device as claimed in claim 43, **characterised in that** the positioning device (140) is provided in the form of a reduction in the internal dimension (124) of the interior (10) and forms an abutment surface (141) essentially perpendicular to the longitudinal axis (15).

45. Holding device as claimed in one of claims 36 to 44, **characterised in that** a taper of the container (5) in its interior (10) between the two planes (16, 17) is between 0.1° and 3.0°, preferably between 0.6° and 0.8°.

46. Holding device as claimed in one of claims 36 to 45, **characterised in that** the container (5) and/or the container (149) and/or the base body (121) and/or the sealing device (120) or sealing arrangement (129) and/or the pressing element (122) is or are made from a fluid-tight and in particular a water-tight and optionally gas-tight plastic.

47. Holding device as claimed in claim 46, **characterised in that** the plastic is selected from the group consisting of polyethylene terephthalate (PET), polypropylene (PP), polyethylene (PE), polystyrene (PS), high-density polyethylene (HDPE), acrylonitrile butadiene styrene copolymers (ABS), thermoplastic elastomers (TPE), thermoplastic polyurethane (TPU), ultra-high molecular polyethylene with a very high molecular weight (PE-UHMW), polycarbonate (PC), polyamide (PA), polyoxymethylene (POM), silicone rubber, pharmaceutical rubber, bromobutyl rubber, rubber, a gel or a combination thereof.

48. Holding device as claimed in one of claims 36 to 47, **characterised in that** the interior (10) of the container (5) is evacuated to a pressure below atmospheric pressure.

49. Separating device (11) for inserting in an interior (10) of a container (5) of a holding device (1) for bodily fluids, pieces or tissue or tissue cultures, having an elastically deformable base body (41) with an abutment surface (42) which can be directed towards the container (5) and end regions (45, 46) spaced at a distance apart from one another in the direction of a longitudinal axis (15) between which the abutment surface (42) extends, and having at least one sealable flow passage (119), **characterised in that**, in an initial position, a gap (54) is provided in the base body (41) extending between the two end regions (45, 46) and widens starting from its centre towards the abutment surface (42), and in an operating position in which an internal dimensions of the interior (10) of the container (5) is smaller than the external periphery of an envelope line of the separating device (11) is the same position, the flow passage (119) can be closed off by means of at least one automatically acting valve system, and the mutually facing gap (54) surfaces of the gap are moved so that they lie in contact with one another.

50. Separating device as claimed in claim 49, **characterised in that** the widening gap (54) is conical in shape

51. Separating device as claimed in claim 49 or 50, **characterised in that** an external dimension (51) of the base body (41) in a plane (50) perpendicular to the longitudinal axis (15) is bigger in its first end region (45) than in its other end region (46).

52. Separating device as claimed in one of claims 49 to 51, **characterised in that** the base body (41) is designed in the form of a truncated cone in which the larger external dimension (51) is disposed in its first end region (45) in the plane (50) perpendicular to the longitudinal axis (15).

53. Separating device as claimed in claim 52, **characterised in that** the truncated cone has a cone angle (53) of between 0.1° and 3.0°, preferably between 0.6° and 0.8°.

54. Separating device as claimed in one of claims 49 to 53, **characterised in that** when the separating device (11) is in the operating position, mutually facing gap faces (56, 57) of the gap (54) sit in a abutment with one another forming a seal.

55. Separating device as claimed in one of claims 49 to 54, **characterised in that** the base body (41) has a concave recess (47) in its first end region (45), the shape of which essentially matches the part of the closing device (9) facing it.

56. Separating device as claimed in one of claims 49 to 55, **characterised in that** the base body (41) has a connecting orifice (44) disposed at its centre and extending in the direction of the longitudinal axis (15) between the end regions (45, 46).

57. Separating device as claimed in claim 56, **characterised in that** the connecting orifice (44) diverges starting from the first end region (45) in the direction of the other end region (46).

58. Separating device as claimed in claim 56, **characterised in that** the connecting orifice is frustoconical in shape.

59. Separating device as claimed in claim 57 or 58, **characterised in that** the diverging region of the connecting orifice (44) extends across only a part-region of a distance (49) between the two end regions (45, 46).

60. Separating device as claimed in one of claims 56 to 59, **characterised in that** an insert part (48) is introduced into the diverging portion of the connecting orifice (44).

61. Separating device as claimed in one of claims 56 to 60, **characterised in that** the connecting orifice (44) has a clearance width (58) in the first end region (45) in the plane (50) perpendicular to the longitudinal axis (15) in the initial position, which is the same as or smaller than an external dimension of the insert part (48).

62. Separating device as claimed in one of claims 56 to 61, **characterised in that** a projecting retaining mechanism (60) projecting into the connecting orifice (44) is provided for the insert part (48) on the base body (41) in the other end region (46).

63. Separating device as claimed in one of claims 56 to 62, **characterised in that** the two end regions (45, 46) of the base body (41) are in flow communication via the connecting orifice (44) when the insert part (48) is in abutment with the retaining mechanism (60).

64. Separating device as claimed in one of claims 56 to 63, **characterised in that** the insert part (48) sits in abutment with boundary walls of the diverging portion of the connecting orifice (44) forming a sealing.

65. Separating device as claimed in one or more of preceding claims 49 to 64, **characterised in that** the base body (41) has a density of between 1.02 g/cm³ and 1.07 g/cm³, preferably between 1.4g/cm³ ad 1.5 g/cm³.

66. Separating device as claimed in one of claims 60 to 64, **characterised in that** the insert part (48) has a density of between 1.02 g/cm³ and 1.07 g/cm³, preferably between 1.04g/cm³ and 1.05 g/cm³.

67. Separating device as claimed in claim 60, **characterised in that** the insert part (48) is provided in the form of a truncated cone.

68. Separating device as claimed in claim 60, **characterised in that** the insert part (48) is a ball.

69. Holding device (1) with a container (5) bounding an interior (10) with an internal wall (118), and two ends (6, 7) spaced at a distance apart in the direction of the longitudinal axis (15), at least one of which has an orifice, and an internal dimension (14) of the container (1) in the region of the first open end (6) in a plane (16) perpendicular to the longitudinal axis (15) is bigger than an internal dimension (18) in the region of the other end (7) in a plane (17) parallel therewith in the same spatial direction, having at least one closing device (9) for the open end (6) of the container (5), with a separating device (11) inserted in the interior (10), this closing device (9) being displaceable from an initial position to an operating position spaced apart in the direction towards the other end (7), **characterised in that** the separating device (11) is designed as claimed in one of claims 49 to 68 and the internal dimension (14) and an internal periphery of an envelope line of the container (5) in the first plane (16; 50) is the same as or smaller than an external dimension (51) and an external periphery of an envelope line of the base body (41) in its non-deformed state in the same plane (16; 50) and in the same spatial direction, and the external dimension (51) and the external periphery of the envelope line of the base body (41) in its operating position is bigger than the minimum internal dimension (18) in the region of the other end (7) in the plane (17) and in the same spatial direction.

70. Holding device as claimed in claim 69, **characterised in that** a taper of the container (5) in its interior (10) between the planes (16, 17) is between 0.1° and 3.0°, preferably between 0.6° and 0.8°.

71. Holding device as claimed in claim 69 or 70, **characterised in that** a cross section of the container (5) in planes (16, 17, 71) is circular.

72. Holding device as claimed in one of claims 69 to 71, **characterised in that** the external dimension (51) of the base body (41) in the plane (50) perpendicular to the longitudinal axis (15) at its first end region (45) is the same as or smaller in the non-deformed state than the internal dimension (14) of the container (5) at its first open end (6) in the plane (16) and in the same spatial direction.

73. Holding device as claimed in claim 69, **characterised in that** the cone angle (53) of the truncated cone matches the taper in the interior (10) of the container (5) between the two planes (16, 17).

74. Holding device as claimed in one of claims 69 to 73, **characterised in that** an arc length (55) of the gap (54) in the region of the abutment surface (42) of the base body (41) in its initial position in the container (5) is the same as the circumferential difference between the internal circumference of the container (5) in the plane (16) perpendicular to the longitudinal axis (15) in the region of the initial position and the internal circumference of the container (5) in the region of the operating position in the plane (71).

75. Holding device as claimed in one of claims 69 to 74, **characterised in that** a positioning device (140) is provided in the region of the operating position between the container (5) and the separating device (11).

76. Holding device as claimed in claim 75, **characterised in that** the positioning device is provided in the form of a reduction in the internal dimension (124) of the interior (10), forming an abutment surface (141) oriented substantially perpendicular to the longitudinal axis (15).

77. Holding device as claimed in one of claims 69 to 76, **characterised in that** the container (5) and/or the base body (41) and/or the insert part (48) is or are made from a fluid-tight and optionally also gas-tight plastic.

78. Holding device as claimed in claim 77, **characterised in that** the plastic is selected from the group consisting of polyethylene terephthalate (PET), polypropylene (PP), polyethylene (PE), polystyrene (PS), high-density polyethylene (HD PE), acrylonitrile butadiene styrene copolymers (ABS) or a combination thereof.

79. Holding device as claimed in one of claims 69 to 78, **characterised in that** at least certain regions of the base body (41) and/or the insert part (48) are provided with a coating.

80. Holding device as claimed in claim 79, **characterised in that that** the coating is a layer of silicone.

81. Holding device as claimed in one of claims 69 to 80, **characterised in that** the interior (10) of the container (5) is evacuated to a pressure below atmospheric pressure.

## Revendications

1. Dispositif de séparation (11) destiné à être placé dans un espace intérieur (10) d'un récipient (5) d'une installation de réception (1) pour des liquides corporels, des parties de tissu, respectivement de cultures de tissu, qui présente au moins deux corps de base (121) avec un dispositif d'étanchéité (120) pouvant être orienté vers une paroi intérieure (118) du récipient (5) ainsi que des zones d'extrémité (45, 46) espacées l'une de l'autre dans la direction d'un axe longitudinal (15), entre lesquelles s'étend au moins un canal d'écoulement (119) pouvant être fermé, **caractérisé en ce que** les corps de base (121) peuvent être appliqués par au moins un élément de pression (122), comme par exemple des barrettes à ressort, des ressorts spirals, par zones à la paroi intérieure (118) du récipient (5) et, dans une position de départ, le canal d'écoulement (119) est réalisé entre les corps de base avoisinants (121) et espacés l'un de l'autre par l'élément de pression (122) et, dans une position de travail, dans laquelle une dimension (124) de l'espace intérieur (10) du récipient (5) est plus petite que le pourtour extérieur d'une ligne enveloppante du dispositif de séparation (11) dans la même position, les corps de base (121) ferment le canal d'écoulement (119).

2. Dispositif de séparation selon la revendication 1, **caractérisé en ce que** celui-ci présente une densité supérieure à 1,05 g/cm³.

3. Dispositif de séparation selon la revendication 2, **caractérisé en ce que** la densité se situe entre 1,5 g/cm³ et 3,5 g/cm³, de préférence entre 2,0 g/cm³ et 2,5 g/cm³.

4. Dispositif de séparation selon l'une des revendications précédentes, **caractérisé en ce que** celui-ci comprend plusieurs, de préférence, deux corps de base (121) qui, pendant l'ensemble de leur mouvement de déplacement, ont par rapport au récipient (5) la même vitesse de déplacement relative.

5. Dispositif de séparation selon l'une des revendications précédentes, **caractérisé en ce que** les corps de base (121) sont ajustables dans un plan orienté perpendiculairement à l'axe longitudinal (15) l'un relativement à l'autre.

6. Dispositif de séparation selon la revendication 1, **caractérisé en ce que** l'élément de pression (122) est disposé entre les zones orientées l'une vers l'autre des corps de base (121).

7. Dispositif de séparation selon l'une des revendications précédentes, **caractérisé en ce que** les corps de base (121) sont maintenus par l'élément de pression (122) dans leur position relative.

8. Dispositif de séparation selon l'une des revendications précédentes, **caractérisé en ce que** les corps de base (121) sont reliés en mouvement par le ou les éléments de pression (122).

9. Dispositif de séparation selon l'une des revendications précédentes, **caractérisé en ce que** le ou les éléments de pression (122) sont disposés d'une manière symétrique à l'axe longitudinal (15).

10. Dispositif de séparation selon l'une des revendications précédentes, **caractérisé en ce que** l'élément de pression (122) est formé par des barrettes élastiques (126) en forme de V vues dans la direction de l'axe longitudinal (15) et se rejoignant en direction de l'axe longitudinal (15).

11. Dispositif de séparation selon l'une des revendications 1 à 9, **caractérisé en ce que** l'élément de pression (122) est formé par des barrettes élastiques (126) en forme V vues dans la direction de l'axe longitudinal (15) et se rejoignant dans la direction éloignée de l'axe longitudinal (15).

12. Dispositif de séparation selon la revendication 11, **caractérisé en ce que** l'élément de pression (122), vu en direction de l'axe longitudinal (15), est formé à chaque fois par deux barrettes élastiques (126) en forme de V, et se rejoignant dans la direction éloignée de l'axe longitudinal (15), et les autres extrémités orientées les unes vers les autres des barrettes élastiques (126) sont reliées les unes aux autres.

13. Dispositif de séparation selon l'une des revendications 1 à 9, **caractérisé en ce que** l'élément de pression (122), vu en direction de l'axe longitudinal (15) et dans un plan orienté à peu près perpendiculairement au canal d'écoulement (119), est formé par des barrettes élastiques (126) courbées à chaque fois d'une manière diamétralement opposée, qui sont reliées à leurs zones d'extrémité orientées les unes vers les autres et le cas échéant par l'agencement d'une partie de liaison circulaire (145).

14. Dispositif de séparation selon l'une des revendications précédentes, **caractérisé en ce que** les corps de base (121) et le ou les éléments de pression (122) sont réalisés en un matériau similaire.

15. Dispositif de séparation selon l'une des revendications précédentes, **caractérisé en ce que** pour la réception de l'élément de pression (122) dans au moins l'une des zones orientées l'une vers l'autre des corps de base (121), un évidement correspondant (127) est prévu.

16. Dispositif de séparation selon l'une des revendications précédentes, **caractérisé en ce que**, entre les corps de base (121) au voisinage de la zone d'extrémité (45) pouvant être orientée vers la première extrémité (6) du récipient (5), un agencement d'étanchéité (129) est prévu pour rendre étanche le ou les canaux d'écoulement (119).

17. Dispositif de séparation selon l'une des revendications précédentes, **caractérisé en ce que** le dispositif d'étanchéité (120) pouvant être orienté vers la paroi intérieure (118) est disposé au voisinage de la zone d'extrémité (45) pouvant être orientée vers la première extrémité (6) du récipient (5).

18. Dispositif de séparation selon l'une des revendications précédentes, **caractérisé en ce que** le dispositif d'étanchéité (120) est formé par une lèvre d'étanchéité (130) s'étendant au moins sur le pourtour du corps de base (121).

19. Dispositif de séparation selon la revendication 18, **caractérisé en ce que** la lèvre d'étanchéité (130) fait saillie sur le corps de base (121) radialement vers l'extérieur dans la direction éloignée de l'axe longitudinal (15).

20. Dispositif de séparation selon la revendication 18 ou 19, **caractérisé en ce que** les sections des lèvres d'étanchéité (130) avoisinant le canal d'écoulement (119), au moins dans la position de travail de celles-ci, se recouvrent ou se chevauchent.

21. Dispositif de séparation selon l'une des revendications précédentes, **caractérisé en ce que** le dispositif d'étanchéité (120) et/ou l'agencement d'étanchéité (129) est réalisé pour être étanche au liquide.

22. Dispositif de séparation selon l'une des revendications précédentes, **caractérisé en ce que** les corps de base (121) sont réalisés dans une zone pouvant être attribuée à la paroi intérieure (118) du récipient (5) par une section d'un cylindre creux respectivement d'un cône tronqué creux.

23. Dispositif de séparation selon l'une des revendications précédentes, **caractérisé en ce que** sont disposés au corps de base (121) plusieurs éléments d'appui (132) sur le pourtour extérieur de celui-ci et faisant saillie sur une surface extérieure (131) dans la direction éloignée de l'axe longitudinal (15).

24. Dispositif de séparation selon la revendication 23, **caractérisé en ce que** les éléments d'appui (132) sont formés par des barrettes orientées parallèlement à l'axe longitudinal (15).

25. Dispositif de séparation selon l'une des revendications précédentes, **caractérisé en ce que** les corps de base (121) présentent au voisinage de la première zone d'extrémité (45) pouvant être orientée vers la première extrémité (6) du récipient (5) une face de guidage (134) formée de préférence par des sections coniques (133) et diminuée en direction de l'axe longitudinal (15) et vers l'autre zone d'extrémité (46).

26. Dispositif de séparation selon l'une des revendications précédentes, **caractérisé en ce que** les corps de base (121) présentent au voisinage de la deuxième zone d'extrémité (46) pouvant être orientée vers l'autre extrémité (7) du récipient (5) une face d'afflux (135) inclinée en direction de l'axe longitudinal (15) et vers la première zone d'extrémité (45).

27. Dispositif de séparation selon l'une des revendications précédentes, **caractérisé en ce que**, entre les zones orientées les unes vers les autres des corps de base (121), au moins une partie de guidage saillante (146) est disposée à l'un des corps de base (121), et dans l'autre corps de base (121) une ouverture de réception (147) coopérant avec celle-ci pour la partie de guidage (146).

28. Dispositif de séparation selon la revendication 27, **caractérisé en ce qu'**un prolongement de retenue (148) coopérant avec l'ouverture de réception (147) est disposé à la partie de guidage (146).

29. Dispositif de séparation selon l'une des revendications précédentes, **caractérisé en ce que** les corps de base (121) sont reliés d'une manière pivotante par une articulation à charnière (151) disposée dans la zone de bord de ceux-ci et passant sur le canal d'écoulement (119).

30. Dispositif de séparation selon la revendication 29, **caractérisé en ce que** l'articulation à charnière (151) forme l'élément de pression (122).

31. Dispositif de séparation selon la revendication 29 ou 30, **caractérisé en ce que** l'articulation à charnière (151) forme un élément d'appui (132).

32. Dispositif de séparation selon l'une des revendications précédentes, **caractérisé en ce que** les corps de base (121) sont formés à chaque fois par un corps de support (136) et le dispositif d'étanchéité (120) et/ou l'agencement d'étanchéité (129) disposés à celui-ci.

33. Dispositif de séparation selon la revendication 32, **caractérisé en ce que** le corps de support (136) présente par rapport au dispositif d'étanchéité (120) respectivement à l'agencement d'étanchéité (129) une densité plus élevée ainsi qu'un module d'élasticité plus élevé.

34. Dispositif de séparation selon l'une des revendications précédentes, **caractérisé en ce que** le corps de base (121) est pourvu au moins par zones d'un revêtement.

35. Dispositif de séparation selon la revendication 34, **caractérisé en ce que** le revêtement est formé par une couche de silicone.

36. Dispositif de réception (1) avec un récipient (5) qui entoure un espace intérieur (10) avec une paroi intérieure (118) et présente deux extrémités (6, 7) espacées l'une de l'autre en direction d'un axe longitudinal (15), dont au moins l'une présente une ouverture, et dans la zone de la paroi intérieure (118), une dimension intérieure (14) du récipient (5), dans la zone de la première extrémité ouverte (6), dans un plan (16) orienté perpendiculairement à l'axe longitudinal (15), est plus grande qu'une dimension intérieure (18) dans la zone de l'autre extrémité (7) dans un plan (17) parallèle à celle-ci dans la même direction spatiale, avec au moins un dispositif de fermeture (9) pouvant être ouvert pour l'extrémité ouverte (6) du récipient (5), avec un dispositif de séparation (11) placé dans l'espace intérieur (10), où celui-ci peut être déplacé à partir d'une position de départ au voisinage du dispositif de fermeture (9) en une position de travail espacée en direction de l'autre extrémité (7), **caractérisé en ce que** le dispositif de séparation (11) est réalisé selon l'une des revendications 1 à 35 et comprend au moins deux corps de base (121) qui sont appliqués par au moins un élément de pression (122) par zones à la paroi intérieure (118) du récipient (5), et **en ce que** la dimension intérieure (14) respectivement un pourtour intérieur d'une ligne enveloppante de l'espace intérieur (10) dans le premier plan (16) est plus grande qu'une dimension extérieure (123) respectivement un pourtour extérieur d'une ligne enveloppante du dispositif de séparation (11) dans sa position de travail et dans la même direction spatiale, et dans la position de départ, un canal d'écoulement (119) est réalisé entre les extrémités (6, 7) du récipient (5) dans la zone du dispositif de séparation (11), et une dimension intérieure (124) respectivement un pourtour intérieur d'une ligne enveloppante de l'espace intérieur (10), au voisinage de la position de travail du dispositif de séparation (11), est plus petite que le pourtour extérieur d'une ligne enveloppante du dispositif de séparation (121) dans la même position, et les corps de base (121) du dispositif de séparation (11) ferment en position de travail le canal d'écoulement (119).

37. Dispositif de réception selon la revendication 36, **caractérisé en ce qu'**au voisinage de la position de départ, un dispositif de retenue (137) est disposé pour le dispositif de séparation (11) placé dans l'espace intérieur (10).

38. Dispositif de réception selon la revendication 37, **caractérisé en ce que** le dispositif de retenue (137) est formé par au moins un bout rapporté (138) faisant saillie sur le pourtour de la paroi intérieure (118) en direction de l'axe longitudinal (15).

39. Dispositif de réception selon la revendication 37 ou 38, **caractérisé en ce que** le dispositif de retenue (137) est formé par une baguette (139) faisant saillie au moins par zones sur le pourtour de la paroi intérieure (118) en direction de l'axe longitudinal (15).

40. Dispositif de réception selon la revendication 39, **caractérisé en ce que** la baguette (139) est disposée pour s'étendre en continu sur le pourtour de la paroi intérieure (118).

41. Dispositif de réception selon l'une des revendications 37 à 40, **caractérisé en ce que** le dispositif de retenue (137) est formé par une diminution de la dimension intérieure (14) de l'espace intérieur (10).

42. Dispositif de réception selon l'une des revendications 37 à 41, **caractérisé en ce que** le dispositif de retenue (137) est formé par un creux en forme de rainure s'étendant en continu sur le pourtour de la paroi intérieure (118).

43. Dispositif de réception selon l'une des revendications 36 à 42, **caractérisé en ce qu'**un dispositif de positionnement (140) est disposé dans la zone de la position de travail entre le récipient (5) et le dispositif de séparation (11).

44. Dispositif de réception selon la revendication 43, **caractérisé en ce que** le dispositif de positionnement (140) est formé par une diminution de la dimension intérieure (124) de l'espace intérieur (10) et en formant une face de butée (141) orientée à peu près perpendiculairement à l'axe longitudinal (15).

45. Dispositif de réception selon l'une des revendications 36 à 44, **caractérisé en ce qu'**une diminution du récipient (5) dans son espace intérieur (10) entre les deux plans (16, 17) représente entre 0,1° et 3,0°, de préférence entre 0,6° et 0,8°.

46. Dispositif de réception selon l'une des revendications 36 à 45, **caractérisé en ce que** le récipient (5) et/ou le récipient (149) et/ou le corps de base (121) et/ou le dispositif d'étanchéité (120) respectivement l'agencement d'étanchéité (129) et/ou l'élément de pression (122) est respectivement sont formés en un matériau synthétique étanche au liquide ainsi que le cas échéant étanche aux gaz.

47. Dispositif de réception selon la revendication 46, **caractérisé en ce que** le matériau est sélectionné dans le groupe constitué de polyéthylène-téréphtalate (PET), propylène (PP), polyéthylène (PE), polystyrène (PS), polyéthylène haute densité (PE-HD), des copolymères d'acrynitrile-butadiène-styrène (ABS), d'élastomères thermoplastiques (TPE), de polyuréthane thermoplastique (TPU), de polyéthylène ultra-haut-moléculaire avec une masse molaire très élevée (PE-UHMW), polycarbonate (PC), polyamide (PA), polyoxyméthylène (POM), caoutchouc silicone, pharma-caoutchouc, caoutchouc de brome butyle, caoutchouc, un gel respectivement une combinaison de ceux-ci.

48. Dispositif de réception selon l'une des revendications 36 à 47, **caractérisé en ce que** l'espace intérieur (10) du récipient (5) est évacué à une pression inférieure à la pression atmosphérique.

49. Dispositif de séparation (11) destiné à être placé dans un espace intérieur (10) d'un récipient (5) d'un dispositif de réception (1) pour des liquides corporels, des parties de tissu respectivement des cultures de tissu, qui présente un corps de base élastiquement déformable (41) avec une face d'application (42) pouvant être orientée vers une paroi intérieure (118) du récipient (5) ainsi que des zones d'extrémité (45, 46) espacées l'une de l'autre en direction d'un axe longitudinal (15) entre lesquelles s'étendent au moins un canal d'écoulement (119) pouvant être fermé ainsi qu'une face d'application (42), **caractérisé en ce que** dans une position de départ, dans le corps de base (41), une fente (54) s'étendant entre les deux zones d'extrémité (45, 46) et s'élargissant, en partant de son centre vers la face d'application (42) est ménagée et, dans une position de travail, dans laquelle une dimension intérieure de l'espace intérieur (10) du récipient (5) est plus petite que le pourtour extérieur d'une ligne enveloppante du dispositif de séparation (11) dans la même position, le canal d'écoulement (119) peut être fermé par au moins un agencement de vanne à auto-action, et les deux faces orientées l'une vers l'autre de la fente (54) sont amenées à s'appliquer l'une à l'autre.

50. Dispositif de séparation selon la revendication 49, **caractérisé en ce que** la fente qui s'élargit (54) est réalisée en forme de coin.

51. Dispositif de séparation selon la revendication 49 ou 50, **caractérisé en ce que** le corps de base (41), dans un plan (50) orienté perpendiculairement à l'axe longitudinal (15) présente dans sa première zone d'extrémité (45) une plus grande dimension extérieure (51) que dans son autre zone d'extrémité (46).

52. Dispositif de séparation selon l'une des revendications 49 à 51, **caractérisé en ce que** le corps de base (41) est réalisé comme cône tronqué qui présente dans sa première zone d'extrémité (45) dans le plan (50) orienté perpendiculairement à l'axe longitudinal (15) la plus grande dimension extérieure (51).

53. Dispositif de séparation selon la revendication 52, **caractérisé en ce que** le cône tronqué présente un angle conique (53) entre 0,1° et 3,0°, de préférence entre 0,6° et 0,8°.

54. Dispositif de séparation selon l'une des revendications 49 à 53, **caractérisé en ce que** dans la position de travail du dispositif de séparation (11), les faces de fente (56, 57) orientées l'une vers l'autre de la fente (54) s'appliquent d'une manière étanche l'une à l'autre.

55. Dispositif de séparation selon l'une des revendications 49 à 54, **caractérisé en ce que** le corps de base (41) présente dans sa première zone d'extrémité (45) un évidement concave (47) qui, dans sa forme, est sensiblement adapté à une partie pouvant être orientée vers celui-ci d'un dispositif de fermeture (9).

56. Dispositif de séparation selon l'une des revendications 49 à 55, **caractérisé en ce que** le corps de base (41) présente une ouverture de liaison (44) disposée dans la zone du centre et s'étendant dans la direction de l'axe longitudinal (15) et entre les zones d'extrémité (45, 46).

57. Dispositif de séparation selon la revendication 56, **caractérisé en ce que** l'ouverture de liaison (44), en partant de la première zone d'extrémité (45) en direction de l'autre zone d'extrémité (46), est réalisée en s'élargissant.

58. Dispositif de séparation selon la revendication 57, **caractérisé en ce que** l'ouverture de liaison est réalisée en une forme tronconique.

59. Dispositif de séparation selon la revendication 57 ou 58, **caractérisé en ce que** la zone qui s'élargit de l'ouverture de liaison (44) s'étend seulement sur une zone partielle d'une distance (49) entre les deux zones d'extrémité (45, 46).

60. Dispositif de séparation selon l'une des revendications 56 à 59, **caractérisé en ce qu'**une pièce d'insertion (48) est placée dans la section qui s'élargit de l'ouverture de liaison (44).

61. Dispositif de séparation selon l'une des revendications 56 à 60, **caractérisé en ce que** l'ouverture de liaison (44) présente dans la première zone d'extrémité (45) dans le plan (50) orienté perpendiculairement à l'axe longitudinal (15), dans la position de départ, une largeur intérieure (58) qui est égale ou inférieure à une dimension extérieure de la pièce d'insertion (48).

62. Dispositif de séparation selon l'une des revendications 56 à 61, **caractérisé en ce qu'**est disposé au corps de base (41), dans l'autre zone d'extrémité (46), un dispositif de retenue (60) faisant saillie dans l'ouverture de liaison (44) pour la pièce d'insertion (48).

63. Dispositif de séparation selon l'une des revendications 56 à 62, **caractérisé en ce que** les deux zones d'extrémité (45, 46) du corps de base (41), lors de l'application de la pièce d'insertion (48) au dispositif de retenue (60), sont en liaison d'écoulement par l'ouverture de liaison (44).

64. Dispositif de séparation selon l'une des revendications 56 à 63, **caractérisé en ce que** la pièce d'insertion (48), en position de travail, s'applique d'une manière étanche à des parois de délimitation de la section qui s'élargit de l'ouverture de liaison (44).

65. Dispositif de séparation selon l'une des revendications 49 à 64, **caractérisé en ce que** le corps de base (41) présente une densité entre 1,02 g/cm³ et 1,07 g/cm³, de préférence entre 1,04 g/cm³ et 1,05 g/cm³.

66. Dispositif de séparation selon l'une des revendications 60 à 64, **caractérisé en ce que** la pièce d'insertion (48) présente une densité entre 1,02 g/cm³ et 1,07 g/cm³, de préférence entre 1,04 g/cm³ et 1,05 g/cm³.

67. Dispositif de séparation selon la revendication 60, **caractérisé en ce que** la pièce d'insertion (48) est réalisée comme cône tronqué.

68. Dispositif de séparation selon la revendication 60, **caractérisé en ce que** la pièce d'insertion (48) est réalisée comme bille.

69. Dispositif de réception (1) avec un récipient (5) qui délimite un espace intérieur (10) avec une paroi intérieure (118) et présente deux extrémités (6, 7) espacées l'une de l'autre en direction d'un axe longitudinal (15) dont au moins une présente une ouverture, et dans la zone de la paroi intérieure (118), une dimension intérieure (14) du récipient (5) au voisinage de la première extrémité ouverte (6) dans un plan (16) orienté perpendiculairement à l'axe longitudinal (15) est plus grande qu'une dimension intérieure (18) au voisinage de l'autre extrémité (7) dans un plan (17) parallèle à celui-ci dans la même direction spatiale, avec au moins un dispositif de fermeture (9) pouvant être ouvert pour l'extrémité ouverte (6) du récipient (5), avec un dispositif de séparation (11) placé dans l'espace intérieur (10), où celui-ci peut être déplacé à partir d'une position de départ se trouvant au voisinage du dispositif de fermeture (9) dans une position de travail espacée de celui-ci en direction de l'autre extrémité (7), **caractérisé en ce que** le dispositif de séparation (11) est réalisé selon l'une des revendications 49 à 68, et **en ce que** la dimension intérieure (14) respectivement un pourtour intérieur d'une ligne enveloppante du récipient (5) dans le premier plan (16; 50) est égale ou inférieure à une dimension extérieure (51) respectivement un pourtour extérieur d'une ligne enveloppante du corps de base (41) dans son état non déformé dans le même plan (16; 50) et la même direction spatiale, et **en ce que** la dimension extérieure (51) respectivement le pourtour extérieur de la ligne enveloppante du corps de base (41) dans sa position de travail est plus grande que la dimension minimale intérieure (18) dans la zone de l'autre extrémité (7) dans le plan (17) et dans la même direction spatiale.

70. Dispositif de réception selon la revendication 69, **caractérisé en ce qu'**une diminution du récipient (5) dans son espace intérieur (10) entre les deux plans (16, 17) représente entre 0,1° et 3,0°, de préférence entre 0,6° et 0,8°.

71. Dispositif de réception selon la revendication 69 ou 70, **caractérisé en ce qu'**une section transversale du récipient (5) dans les plans (16, 17, 71) est réalisée en une forme circulaire.

72. Dispositif de réception selon l'une des revendications 69 à 71, **caractérisé en ce que** la dimension extérieure (51) du corps de base (41) dans le plan (50) orienté perpendiculairement à l'axe longitudinal (15), dans sa première zone d'extrémité (45), à l'état non déformé, est égale ou supérieure à la dimension intérieure (14) du récipient (5) dans sa première extrémité ouverte (6) dans le plan (16) et dans la même direction spatiale.

73. Dispositif de réception selon la revendication 69, **caractérisé en ce que** l'angle conique (53) du cône tronqué correspond à la diminution de l'espace intérieur (10) du récipient (5) entre les deux plans (16, 17).

74. Dispositif de réception selon l'une des revendications 69 à 73, **caractérisé en ce qu'**une longueur d'arc (55) de la fente (54) au voisinage de la face d'application (42) du corps de base (41) dans sa position de départ dans le récipient (5) est égale à la différence du pourtour entre le pourtour intérieur du récipient (5) dans le plan (16) orienté perpendiculairement à l'axe longitudinal (15) au voisinage de la position de départ et le pourtour intérieur du récipient (5) au voisinage de la position de travail dans le plan (71).

75. Dispositif de réception selon l'une des revendications 69 à 74, **caractérisé en ce qu'**un dispositif de positionnement (140) est disposé dans la zone de la position de travail entre le récipient (5) et le dispositif de séparation (11).

76. Dispositif de réception selon la revendication 75, **caractérisé en ce que** le dispositif de positionnement est formé par une diminution de la dimension intérieure (124) de l'espace intérieur (10) et en formant une face de butée (141) orientée à peu près perpendiculairement à l'axe longitudinal (15).

77. Dispositif de réception selon l'une des revendications 69 à 76, **caractérisé en ce que** le récipient (5) et/ou le corps de base (41) et/ou la pièce d'insertion (48) est respectivement sont formés en un matériau synthétique étanche au liquide ainsi que le cas échéant étanche aux gaz.

78. Dispositif de réception selon la revendication 77, **caractérisé en ce que** le matériau est sélectionné dans le groupe constitué de polyéthylène-téréphtalate (PET), polypropylène (PP), polyéthylène (PE), polystyrène (PS), polyéthylène haute densité (PE-HD), des copolymères d'acrylnitrile-butadiène-styrène (ABS) respectivement d'une combinaison de ceux-ci.

79. Dispositif de réception selon l'une des revendications 69 à 78, **caractérisé en ce que** le corps de base (41) et/ou la pièce d'insertion (48) sont pourvus au moins par zones d'un revêtement.

80. Dispositif de réception selon la revendication 79, **caractérisé en ce que** le revêtement est formé par une couche de silicone.

81. Dispositif de réception selon l'une des revendications 69 à 80, **caractérisé en ce que** l'espace intérieur (10) du récipient (5) est évacué à une pression inférieure à la pression atmosphérique.
